# EUROPEAN PATENT APPLICATION

(11) **EP 2 385 370 A1**
(43) Date of publication of application: **09.11.2011**
(21) Application number: 11158438.9
(22) Date of filing: 10.04.2009
(51) Int. Cl.: G01N 33/50, G01N 33/574, G01N 33/53, C12Q 1/68

(54) **Methods for identification and use of agents targeting cancer stem cells**

(30) Priority: 10.04.2008 US 43948 P; 15.05.2008 US 53563 P
(62) Divisional of application: 09729661.0
(71) Applicant: Massachusetts Institute of Technology (MIT), Cambridge, MA 02139 (US); Whitehead Institute For Biomedical Research, Cambridge, MA 02142-1479 (US)
(72) Inventor: Gupta, Piyush, Boston, MA 02114 (US); Onder, Tamer T., Cambridge, MA 02142 (US); Lander, Eric S., Cambridge, MA 02138 (US); Weinberg, Robert, Brookline, MA 02446 (US); Mani, Sendurai, Houston, TX 77025 (US); Liao, Mai-jing, Winchester, MA 01890 (US)
(74) Representative: HOFFMANN EITLE

(57) **Abstract**

The invention relates to methods for identifying compounds and compositions that target cancer stem cells. In some aspects, the invention relates to treatment methods that use compounds and compositions that specifically target cancer stem cells for inhibiting the growth and/or survival of cancer stem cells in a subject in need thereof. Other aspects of the invention relate to the use of cancer stem cell biomarkers in the selection of a treatment for inhibiting the growth and/or survival of cancer stem cells in a subject in need thereof.

## Description

### RELATED APPLICATIONS

This application claims priority under 35 U.S.C. § 119(e) from U.S. provisional application serial number 61/043,948, filed April 10, 2008, and application serial number 61/053,563, filed May 15, 2008, the entire content of which is incorporated by reference herein.

### GOVERNMENT FUNDING

This invention was made with Government support from the National Cancer Institute's Initiative for Chemical Genetics of the National Institutes of Health under Grant No. N01-CO-12400 and the U.S. Army Medical Research and Materiel Command under Grant No. W81XWH-05-1-0268. The Government has certain rights in the invention.

### FIELD OF THE INVENTION

The invention relates to methods for identifying compounds and compositions that target cancer stem cells. In some aspects, the invention relates to treatment methods that use compounds and compositions that specifically target cancer stem cells for inhibiting the growth and/or survival of cancer stem cells in a subject in need thereof. Other aspects of the invention relate to the use of cancer stem cell biomarkers in the selection of a treatment for inhibiting the growth and/or survival of cancer stem cells in a subject in need thereof.

### BACKGROUND OF INVENTION

Cancer can arise from a number of genetic and epigenetic alterations that cause defects in mechanisms controlling cell migration, proliferation, differentiation, and growth. Recent findings have demonstrated that tumor formation and growth are driven by a minor subpopulation of cancer cells, termed cancer stem cells, within tumors. Cancer stem cells (CSCs) are cells within a tumor mass that have the capacity to seed and generate secondary tumors, and are responsible for the primary cause of cancer mortality that is metastatic dissemination. This concept has significant implications for the development and preclinical assessment of potential cancer therapies.

### SUMMARY OF INVENTION

Cancer stems cells (CSCs) drive the long term survival of a tumor. Conventional cancer therapies, while successful in eradicating the bulk of tumors, are typically less effective on the insidious CSCs. The selective drug resistance exhibited by these malignant cells contributes to significant morbidity and mortality in cancer. Moreover, CSCs possess the ability to seed tumors at limiting dilutions in animal models. Thus, there is a need for drugs that specifically and selectively target CSCs. However, several factors limit the ability to effectively identify such drugs. A key constraint is a difficulty in obtaining and maintaining cultures of the CSCs, which when isolated differentiate and cease to propagate. Thus, discovering drug candidates targeting this malignant subset remains an ongoing challenge of cancer biologists.

Aspects of the present invention are based on the discovery and development of methods for identifying novel therapeutics that target cancer stem cells. In certain aspects of the present invention the discovery of cancer stem cell-targeted therapies is achieved by high-throughput screening methods. In other aspects of the invention compounds that specifically target cancer stem cells are disclosed. Thus, the present invention discloses cancer stem cell targeting therapies which are identified by high-throughput screening methods and their uses.

According to one aspect of the invention, methods for testing the ability of a compound to inhibit the growth and/or survival of a cancer stem cell are provided. The methods include (a) contacting one or more test cells with a sample of the compound, wherein the one or more test cells has undergone an epithelial to mesenchymal transition, and (b) detecting the level of inhibition of the growth and/or survival of the one or more test cells by the compound.

In some embodiments, the epithelial to mesenchymal transition results from inhibiting the activity of E-Cadherin in the one or more test cells. In some embodiments, the inhibiting the activity of E-Cadherin in the one or more test cells includes contacting the one or more test cells with a blocking antibody to E-Cadherin, inducing the expression of dysadherin in the one or more test cells, or interfering with cell-polarity genes in the one or more test cells. In some embodiments, the inhibiting the activity of E-Cadherin in the one or more test cells comprises contacting the one or more test cells with a small-interfering nucleic acid complementary to E-Cadherin mRNA.

In some embodiments, the epithelial to mesenchymal transition results from inducing the activity of a transcription factor in the one or more test cells, wherein the transcription factor is selected from: Snail1, Snail2, Goosecoid, FoxC2, TWIST, E2A, SIP-1/Zeb-2, dEF1/ZEb1, LEF1, Myc, HMGA2, TAZ, Klf8, HIF-1, HOXB7, SIM2s, and Fos.

In some embodiments, the epithelial to mesenchymal transition results from inducing the activity of TWIST. In some embodiments, the inducing the activity of TWIST in the one or more test cells comprises contacting the one or more test cells with an expression vector encoding TWIST.

In some embodiments, the epithelial to mesenchymal transition results from contacting the one or more test cells with a growth factor selected from: a TGF-β/BMP superfamily member, a Wnt-family member, an FGF family member, a Notch Ligand, an EGF family member, an IGF family member, PDGF, and HGF.

In some embodiments, the epithelial to mesenchymal transition results from modulating the activity of a signaling pathway in the one or more test cells, wherein the signaling pathway is selected from TGF-β, Wnt, BMP, Notch, HGF-Met, EGF, IGF, PDGF, FGF, P38-mapk, Ras, PI3Kinase-Akt, Src, and NF-kB.

In some embodiments, the epithelial to mesenchymal transition results from subjecting the one or more test cells to a stress selected from: hypoxia, irradiation, and chronic chemotherapy treatment. In some embodiments, the epithelial to mesenchymal transition results from subjecting the one or more test cells to a treatment with nicotine or a reactive-oxygen species producer such as hydrogen peroxide (H₂0₂). (See, e.g., Dasgupta et al. Nicotine induces cell proliferation, invasion and epithelial-mesenchymal transition in a variety of human cancer cell lines. Int J Cancer. 2009 Jan 1;124(1):36-45 and Lim et al. Epigenetic changes induced by reactive oxygen species in hepatocellular carcinoma: methylation of the E-cadherin promoter. Gastroenterology. 2008 Dec;135(6):2128-40, 2140-el-8. Epub 2008 Jul 31.) In some embodiments, the epithelial to mesenchymal transition results from subjecting the one or more test cells to a treatment with nAChR agonists, C3a or MFG-E8. (See, e.g., Tang Z, et al., C3a mediates epithelial-to-mesenchymal transition in proteinuric nephropathy, J Am Soc Nephrol. 2009 Mar;20(3):459-60; Jinushi M, et al., Milk fat globule EGF-8 promotes melanoma progression through coordinated Akt and twist signaling in the tumor microenvironment, Cancer Res. 2008 Nov 1;68(21):8889-98.)

In some embodiments, the one or more test cells comprise non-tumorigenic cells.

In some embodiments, the one or more test cells has been rendered tumorigenic after undergoing an EMT. In other embodiments, the one or more test cells has been rendered tumorigenic prior to undergoing an EMT.

In some embodiments, the methods include (a) contacting one or more test cells and one or more control cells with a sample of the compound, wherein the one or more test cells has undergone an epithelial to mesenchymal transition and the one or more control cells has not undergone an EMT, (b) detecting the level of inhibition of the growth and/or survival of the one or more test cells and control cells by the compound; and (c) identifying the compound as a candidate CSC-selective chemotherapeutic agent if the compound has a greater inhibitory effect on the growth and/or survival of the test cells than the control cells. In certain embodiments, the test cells and the control cells are genetically matched. In some embodiments, the test cells express or contain a small interfering RNA that inhibits expression of E-cadherin and the control cells do not express such an RNA.

In some embodiments, the methods further include contacting one or more control cells with a sample of the compound and detecting the level of inhibition of the growth and/or survival of the one or more control cells by the compound. In some embodiments, the one or more control cells is an epithelial cell that has not undergone an epithelial to mesenchymal transition. In certain embodiments, the one or more control cells is contacted with a control expression construct or a control small-interfering nucleic acid. In some embodiments, the control small-interfering nucleic acid does not target an endogenous gene of the one or more control cells, optionally wherein the small-interfering nucleic acid targets GFP mRNA. In some embodiments, the control expression construct encodes a GFP protein or a reporter protein. In some embodiments, the one or more control cells comprise non-tumorigenic cells. In other embodiments, the one or more control cells comprise tumorigenic cells.

In some embodiments of the methods, each of the one or more test cells is contacted with a different dose of, and/or for a different duration with, the compound than at least one other test cell; and/or wherein each of the one or more control cells is contacted with a different dose of, and/or for a different duration with, the compound than at least one other control cell. In certain embodiments, the methods further include analyzing a test and/or control dose response curve, wherein the test dose response curve indicates the level of inhibition of the one or more test cells by the compound at a plurality of doses; and wherein the control dose response curve indicates the level of inhibition on the one or more control cells by the compound at a plurality of doses. In some embodiments, the analyzing comprises determining an EC50 value for compound on the one or more test cells and/or the one or more control cells. In certain embodiments, the EC50 value for the compound on the one or more control cells is statistically significantly less than the EC50 value for the compound on the one or more test cells. In other embodiments, the EC50 value for the compound on the one or more control cells is statistically significantly greater than the EC50 value for the compound on the one or more test cells.

In some embodiments, the compound is a control compound, optionally which is selected from doxorubicin, paclitaxel, actinomycin D, camptothecin, and staurosporine.

In some embodiments, the one or more control cells and the one or more test cells are in a co-culture. In certain embodiments, the one or more test cells have an identifying characteristic that is detectable and distinct from an identifying characteristic of the one or more control cells, optionally wherein the identifying characteristic comprises a level of expression of GFP protein and/or a cancer stem cell biomarker. In some embodiments, the level of expression of GFP protein in the one or more test cells is compared with the level of expression of GFP protein in the one or more control cells. In some embodiments, the methods further include monitoring the ratio of the one or more test cells to one or more control cells by detecting the identifying characteristic of each cell in a sample of the co-culture, optionally wherein the detecting comprises fluorescence-activated cell sorting.

In some embodiments, the method further includes testing a plurality of test samples, wherein each of the test samples comprises at least one of the one or more test cells; and/or further comprising testing a plurality of control samples, wherein each of the control samples comprises at least one of the one or more control cells. In some embodiments, each test sample and/or each control sample is in a separate culture chamber, optionally wherein each culture chamber is a well of a multi-well plate. In some embodiments, the multi-well plate has a number of wells selected from 6, 12, 24, 96, 384, or 1536.

In some embodiments, the compound is obtained from a library consisting of a plurality of compounds, optionally wherein the library is selected from a natural products library, a diversity library, a kinase-inhibitor library, a HDAC-inhibitor library, a library of known bioactive compounds, a peptide library, an antibody library, or an RNAi library. In certain embodiments, the contacting the one or more test cells comprises transferring a sample of the compound from the library to a culture chamber containing the test sample; and/or wherein the contacting the one or more control cells comprises transferring a sample of the compound from the library to a culture chamber containing the control sample. In some embodiments, the methods further include identifying a lead compound that is substantially more cytotoxic to the one or more test cells than the one or more control cells, by comparing the level of inhibition of the growth and/or survival of the one or more test cells by the compound to the level of inhibition of the growth and/or survival of the one or more control cells by the compound. In certain embodiments, the methods further include contacting one or more cancer cells with a sample of the lead compound; and detecting the level of inhibition of the growth and/or survival of the one or more cancer cells by the lead compound. In some embodiments, the one or more cancer cells are obtained from a colon carcinoma, a pancreatic cancer, a breast cancer, an ovarian cancer, a prostate cancer, a squamous cell carcinoma, a cervical cancer, a lung carcinoma, a small cell lung carcinoma, a bladder carcinoma, a squamous cell carcinoma, a basal cell carcinoma, an adenocarcinoma, a sweat gland carcinoma, a sebaceous gland carcinoma, a papillary carcinoma, a papillary adenocarcinoma, a cystadenocarcinoma, a medullary carcinoma, a bronchogenic carcinoma, a renal cell carcinoma, a hepatocellular carcinoma, a bile duct carcinoma, a choriocarcinoma, a seminoma, a embryonal carcinoma, a Wilms' tumor, or a testicular tumor. In certain embodiments, at least one of the one or more cancer cells is a cancer stem cell, which has a cancer stem cell biomarker.

In some embodiments, the cancer stem cell biomarker is the cell surface marker profile of CD44⁺ and CD24⁻. In some embodiments, the cancer stem cell is a MDA-MB-231, SUM 159, or T47D breast cancer cell.

In some embodiments, the detecting the level of inhibition of the growth and/or survival of the one or more cancer cells comprises determining the fraction of the one or more cancer cells that is the at least one cancer stem cell.

In some embodiments, the detecting the level of inhibition of the growth and/or survival of the one or more cancer cells comprises determine the capacity of the one or more cancer cells to form colonies in suspension culture.

In some embodiments, the detecting the level of inhibition of the growth and/or survival of the one or more cancer cells comprises determine the capacity of the one or more cancer cells to form tumors *in vivo.*

In some embodiments, the methods further include producing a refined lead compound by modifying the lead compound to achieve (i) improved potency, (ii) decreased toxicity (improved therapeutic index); (iii) decreased side effects; (iv) modified onset of therapeutic action and/or duration of effect; and/or (v) modified pharmacokinetic parameters (absorption, distribution, metabolism and/or excretion). In certain embodiments, the methods further include determining the *in vivo* toxicology profile of the lead or refined lead compound by a performing a quantitative structure activity relationship analysis of the lead or refined lead compound. In some embodiments, the methods further include producing a Pharmaceutical composition by formulating the lead or refined lead compound with a pharmaceutically acceptable carrier. In some embodiments, the methods further include testing the lead or refined lead compound *in vivo*, by administering the pharmaceutical composition to a subject having a cancer cell, and evaluating the toxicity of the compound to the subject and/or the effect of the compound on the growth and/or survival of the cancer cell in the subject. In certain embodiments, the subject is selected from a mouse, a rat, a rabbit, a dog, a cat, a sheep, a pig, a non-human primate, and a human.

In some embodiments, the methods further include determining the expression of one or more cancer stem cell markers in the test cell and/or the control cell. In certain embodiments, the one or more cancer stem cell markers are selected from: CD20, CD24, CD34, CD38, CD44, CD45, Cod105, CD133, CD166, EpCAM, ESA, SCA1, Pecam, and Stro1. In some embodiments, the one or more cancer stem cell markers are CD24 and CD44.

In some embodiments, the detecting the level of inhibition of the growth and/or survival of the test cell and/or the control cell by the compound comprises performing an assay selected from: a cell counting assay, a replication labeling assay, a cell membrane integrity assay, a cellular ATP-based viability assay, a mitochondrial reductase activity assay, a caspase activity assay, an Annexin V staining assay, a DNA content assay, a DNA degradation assay, and a nuclear fragmentation assay.

According to another aspect of the invention, methods for characterizing one or more cells are provided. The methods include (a) contacting the one or more cells with a compound selected from doxorubicin, paclitaxel, actinomycin D, camptothecin, and staurosporine, (b) detecting a level of inhibition of the growth and/or survival of the one or more cells by the compound, and (c) comparing the results of (b) to a control level, wherein if the level of inhibition of the growth and/or survival of the one or more cells by the compound is statistically significantly less than the control level then the one or more cells have a cancer stem cell characteristic.

In some embodiments, the methods further include evaluating a cancer stem cell biomarker in the one or more cells. In certain embodiments, the cancer stem cell biomarker is selected from E-cadherin expression, TWIST expression, and a CD44/CD24 cell surface marker profile. In some embodiments, the functional assay is a colony formation assay or an in vivo tumor seeding assay.

In some embodiments, the control level is a level of inhibition of the growth and/or survival of one or more cancer cells that are not cancer stem cells by the compound.

According to another aspect of the invention, methods for treating a subject having, or suspected of having, cancer are provided. The methods include administering to the subject an effective amount of paclitaxel in combination with an effective amount of a pharmaceutical composition comprising etoposide, salinomycin, abamectin, or nigericin.

According to another aspect of the invention, methods for treating a subject having, or suspected of having, cancer are provided. The methods include administering to the subject an effective amount of a pharmaceutical composition comprising etoposide, salinomycin, abamectin, nigericin, or a derivative of any of the foregoing.

According to another aspect of the invention, methods for selecting a treatment for a subject having cancer are provided. The methods include evaluating a cancer stem cell biomarker in the cancer and, if the cancer stem cell biomarker is detected, treating the subject by administering to the subject an effective amount of a pharmaceutical composition comprising salinomycin, abamectin, etoposide or nigericin or a derivative thereof, optionally in combination with paclitaxel or a derivative thereof.

In some embodiments, evaluating the cancer stem cell biomarker comprises obtaining a sample of the cancer from the subject.

In some embodiments, the cancer stem cell biomarker is selected from: E-cadherin expression; TWIST expression, and a CD44/CD24 cell surface marker profile. In certain embodiments, the E-cadherin and/or TWIST expression in the cancer is determining by measuring a level of E-cadherin and/or TWIST protein and/or RNA expression in the cancer, and optionally comparing the level to a reference standard. In some embodiments, the reference standard is the level of E-cadherin and/or TWIST protein and/or RNA expression in a cancer stem cell. In other embodiments, the reference standard is the level of E-cadherin and/or TWIST protein and/or RNA expression in a cancer cell that is not a cancer stem cell.

In some embodiments, the cancer is a colon carcinoma, a pancreatic cancer, a breast cancer, an ovarian cancer, a prostate cancer, a squamous cell carcinoma, a cervical cancer, a lung carcinoma, a small cell lung carcinoma, a bladder carcinoma, a squamous cell carcinoma, a basal cell carcinoma, an adenocarcinoma, a sweat gland carcinoma, a sebaceous gland carcinoma, a papillary carcinoma, a papillary adenocarcinoma, a cystadenocarcinoma, a medullary carcinoma, a bronchogenic carcinoma, a renal cell carcinoma, a hepatocellular carcinoma, a bile duct carcinoma, a choriocarcinoma, a seminoma, a embryonal carcinoma, a Wilms' tumor, or a testicular tumor. In certain embodiments, the cancer is a breast carcinoma or a lung carcinoma.

In some embodiments, the subject is mammal, which is optionally a human.

In some embodiments, the pharmaceutical composition is administered intravenously, intramuscularly, subcutaneously, intraperitoneally, orally or as an aerosol.

According to another aspect of the invention, methods for treating a subject having, or suspected of having, cancer are provided. The methods include administering to the subject an effective amount of a pharmaceutical composition comprising a compound that selectively inhibits growth and/or proliferation of test cells that have undergone an EMT relative to inhibition of growth and/or proliferation of control cells that have not undergone an EMT.

In some embodiments, the test and control cells are genetically matched.

In some embodiments, the test cells are non-tumorigenic.

According to another aspect of the invention, methods for treating a subject having, or suspected of having, cancer are provided. The methods include administering to the subject an effective amount of a compound that selectively inhibits proliferation of, or kills, cancer stem cells relative to its effect on non-CSC cancer cells.

According to another aspect of the invention, methods for treating a subject having, or suspected of having, cancer are provided. The methods include administering to the subject an effective amount of a compound that selectively inhibits proliferation of, or kills, cancer stem cells relative to its effect on noncancerous cells.

According to another aspect of the invention, methods of identifying a target for drug discovery are provided. The methods include steps of: providing a compound that selectively inhibits growth and/or proliferation of the test cells, wherein the test cells are cells that have undergone EMT; and identifying a biological target of the compound.

In some embodiments, the biological target is a protein or RNA expressed by the test cells.

In some embodiments, the compound is a compound listed in Table 1 or a derivative therof.

In some embodiments, the methods further include performing a screen to identify a second compound that interacts with or acts on the biological target.

According to another aspect of the invention, methods of identifying a target for drug discovery are provided. The methods include steps of: contacting test cells or test cell lysate with a compound that selectively inhibits growth and/or proliferation of the test cells, wherein the contacting is performed under conditions in which the compound can physically interact with cellular biomolecules, and wherein the test cells are cells that have undergone EMT; isolating a cellular biomolecule that physically interacts with the compound; and identifying the biomolecule.

In some embodiments, the methods further include performing a screen to identify a second compound that interacts with or acts on the biomolecule.

In some embodiments, the compound is a compound listed in Table 1 or a derivative thereof.

In some embodiments, the compound is attached to a support thereby forming an affinity matrix.

According to another aspect of the invention, methods of generating a cancer stem cell are provided. The methods include inducing a cancer cell to undergo an EMT.

In some embodiments, the cancer cell is an experimentally produced cancer cell. In some embodiments, the experimentally produced cancer cell comprises an expression vector encoding an oncogene. In certain embodiments, the oncogene is V12H-RAS.

In some embodiments, the cancer cell is derived from a naturally occurring cancer.

In some embodiments, the epithelial to mesenchymal transition results from inhibiting the activity of E-Cadherin in the cancer cell. In certain embodiments, the inhibiting the activity of E-Cadherin in the cancer cell comprises: contacting the cancer cell with a blocking antibody to E-Cadherin, inducing the expression of dysadherin in the cancer cell, or interfering with cell-polarity genes in the cancer cell. In certain other embodiments, the inhibiting the activity of E-Cadherin in the cancer cell comprises contacting the cancer cell with a small-interfering nucleic acid complementary to E-Cadherin mRNA.

In some embodiments, the cancer cell is induced to undergo EMT by expressing a transcription factor in the one or more test cells, wherein the transcription factor is selected from: Snail1, Snail2, Goosecoid, FoxC2, TWIST, E2A, SIP-1/Zeb-2, dEF1/ZEb1, LEF1, Myc, HMGA2, TAZ, Klf8, HIF-1, HOXB7, SIM2s, and Fos. In certain embodiments, the cancer cell is induced to undergo EMT by constitutively inducing the activity of the transcription factor.

In some embodiments, the cancer stem cell has an increased likelihood of (i) initiating a tumor; (ii) forming a colony in soft agar suspension culture; and/or (iii) forming a tumor sphere, relative to the likelihood had the cancer cell not been induced to undergo EMT.

In some embodiments, the methods further comprise assessing the ability of a test agent to inhibit proliferation, colony formation in soft agar suspension culture, tumor sphere formation, and/or tumor initiating ability of the cancer stem cell. In certain embodiments, the test agent is identified as a candidate agent for cancer therapy if the proliferation, colony formation in soft agar suspension culture, tumor sphere formation, and/or tumor initiating ability of the cancer stem cell is inhibited.

In some embodiments, the methods further comprise introducing the cancer cell having properties of a cancer stem cell into an animal host. In certain embodiments, the methods further comprise assessing the ability of a test agent to inhibit proliferation or tumor initiating ability of the cancer stem cell in the animal host. In specific embodiments, the test agent is identified as a candidate agent for cancer therapy if the proliferation or tumor initiating ability of the cancer stem cell is inhibited.

According to some aspects of the invention, a cancer stem cell generated by any of the foregoing methods is provided.

According to other aspects of the invention, an animal host comprising the foregoing cancer stem cell is provided.

According to other aspects of the invention; kits are provided that comprise a container having a cancer cell that has been induced to undergo an EMT to generate a cancer stem cell, using any of the methods disclosed herein. In some embodiments, the kits further comprise a second container having a cancer cell that has not been induced to undergo an EMT and that is genetically matched with the cancer cell that has been induced to undergo an EMT to generate the cancer stem cell.

According to other aspects of the invention, kits are provided that comprise a container having a non-cancer cell that has been induced to undergo an EMT to generate a non-cancer stem cell, using any of the methods disclosed herein. In some embodiments, the kits further comprise a second container having a non-cancer cell that has not been induced to undergo an EMT and that is genetically matched with the non-cancer cell that has been induced to undergo an EMT to generate the non-cancer stem cell.

In certain embodiments, the container, or containers, provided with the kits further comprise a cryopreservation agent. It is to be appreciate that the kits are not limited to any one particular cryopreservation agent and that the skilled artisan will be capable of selecting an appropriate cryopreservation agent (e.g., DMSO). Exemplary methods and reagents for cryopreservation of cells are disclosed in Freshney R.I., Culture of Animal Cells, A Manual of Basic Technique, 4th Ed., Chapter 19, Wiley-Liss, Inc. (2000).

### BRIEF DESCRIPTION OF DRAWINGS

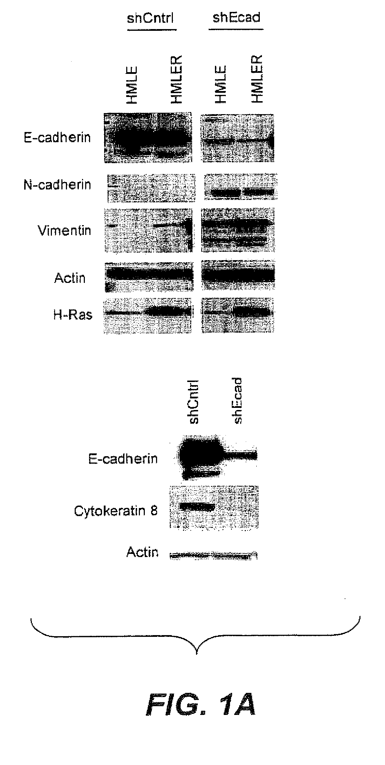

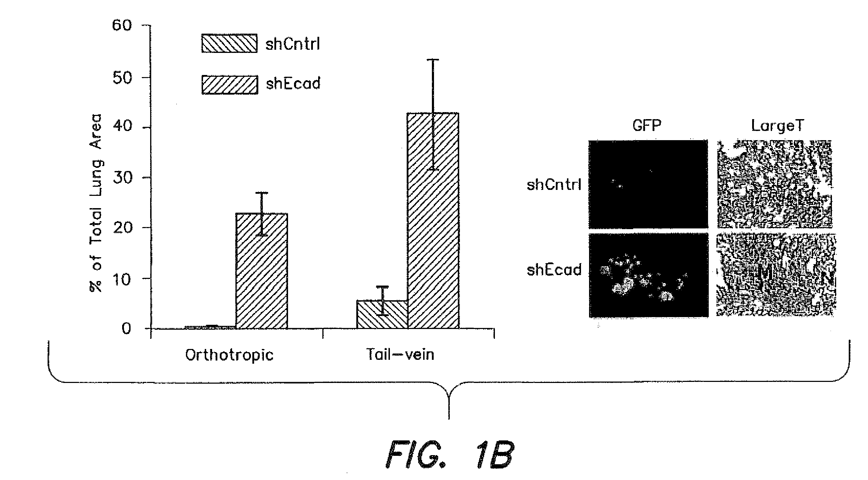

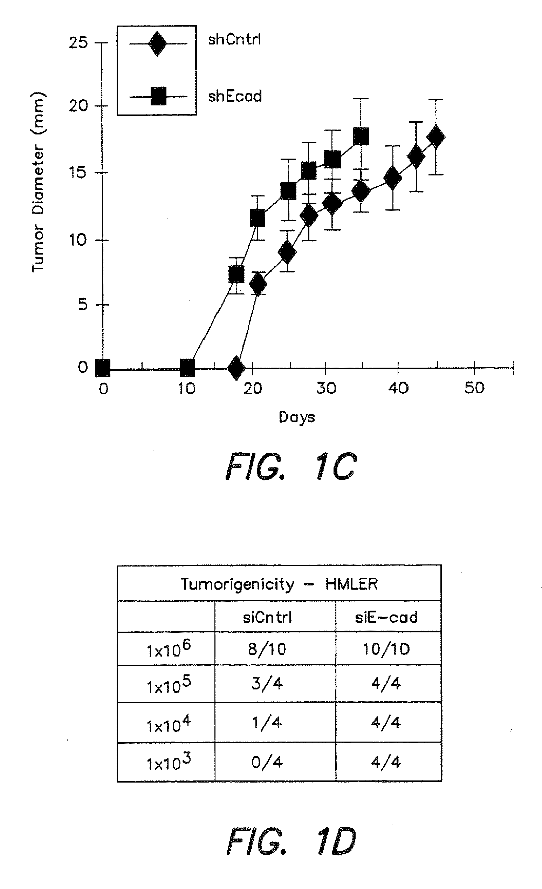

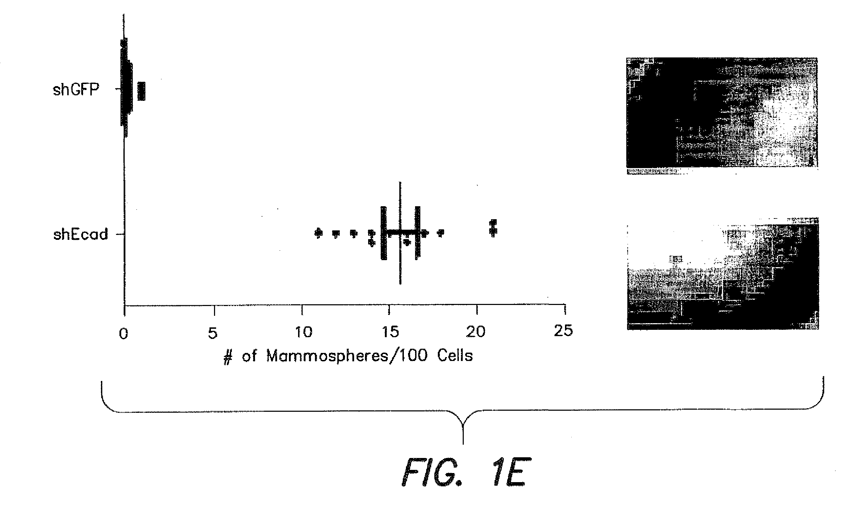

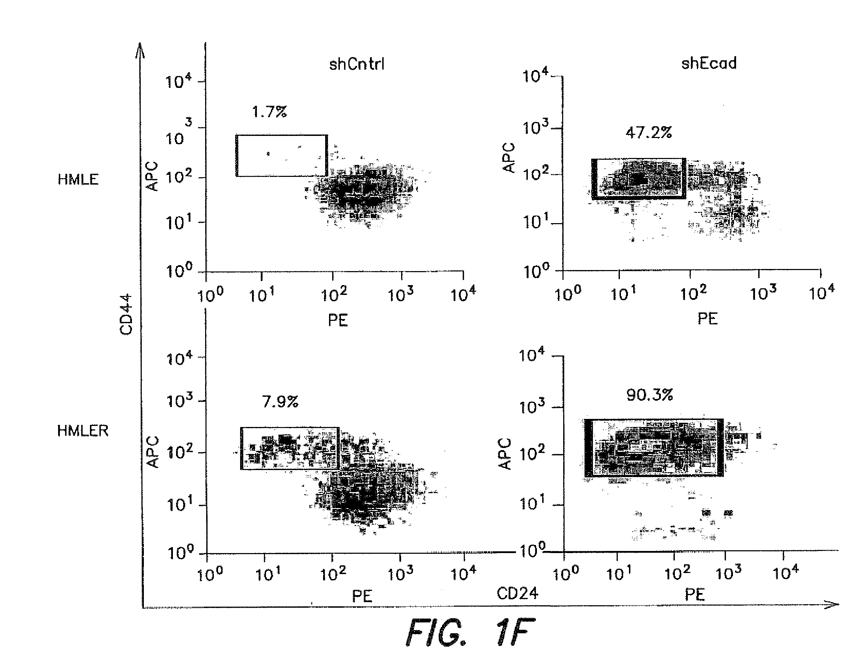

Fig. 1 depicts the effects of E-Cadherin inhibition and EMT induction on metastasis, primary tumor incidence, mammosphere forming ability and Antigenic marker expression. Fig. 1A depicts expression levels of E-Cadherin and mesenchymal proteins N-cadherin and Vimentin in immortalized HMLE and transformed HMLER cells. β-actin is used as a loading control. Bottom panel shows the expression of Cytokeratin 8 in shCntrl and shEcad HMLER cells. Figure 1B depicts (left) quantification of total lung metastasis burden in mice bearing orthotopic primary tumors of HMLER-shCntrl and shEcad cells or 8-weeks after tail-vein injection of these lines. Each bar represents the mean ± s.d. of 5 mice analyzed per group (* p<0.001). Right: Representative fluorescence images of mouse lung lobes bearing orthotopic primary tumors of HMLER-shCntrl and shEcadcells. GFP signal denotes the presence of tumor cells. Also shown are representative immunohistochemical staining of sections with anti-Large T antibody from the same set of lungs. Brown nuclear staining denotes the tumor cells. N, lung tissue; M, metastatic nodule (Right Panels). Fig. 1C depicts growth patterns of primary subcutaneous tumors formed by the HMLER-shCntrl and HMLER-shEcad cells. Each data point represents the mean ± s.d. (standard deviation) of 8 primary tumors. Figure 1D depicts the incidence of subcutaneous tumors in NOD/Scid mice inoculated with either HMLER-shCtnrl or HMLER-shEcad cells at the indicated number of cells injected after 10 weeks of incubation. Note that while HMLER-shCntrl cells cannot initiate tumors at lower dilutions HMLER-shEcad retain the ability to do. (E) Flow cytomery analysis of HMLE and HMLER derived cell lines with respect to CD24 and CD44 expression. Red circles denote the CD24⁻CD44⁺ CSC-like fraction. Note that both immortalized (HMLE) and transformed (HMLER) cells expressing the shE-Cad contain significantly greater numbers of cells displaying the CSC antigenic profile

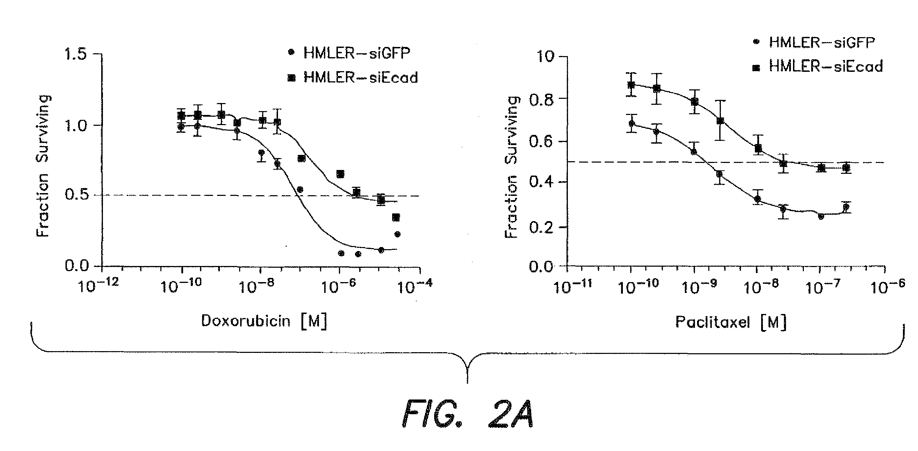

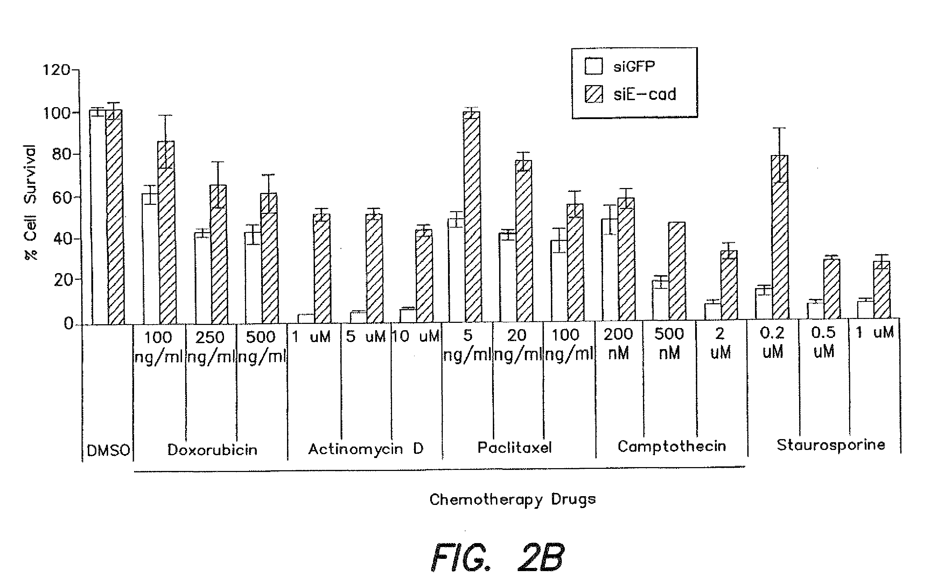

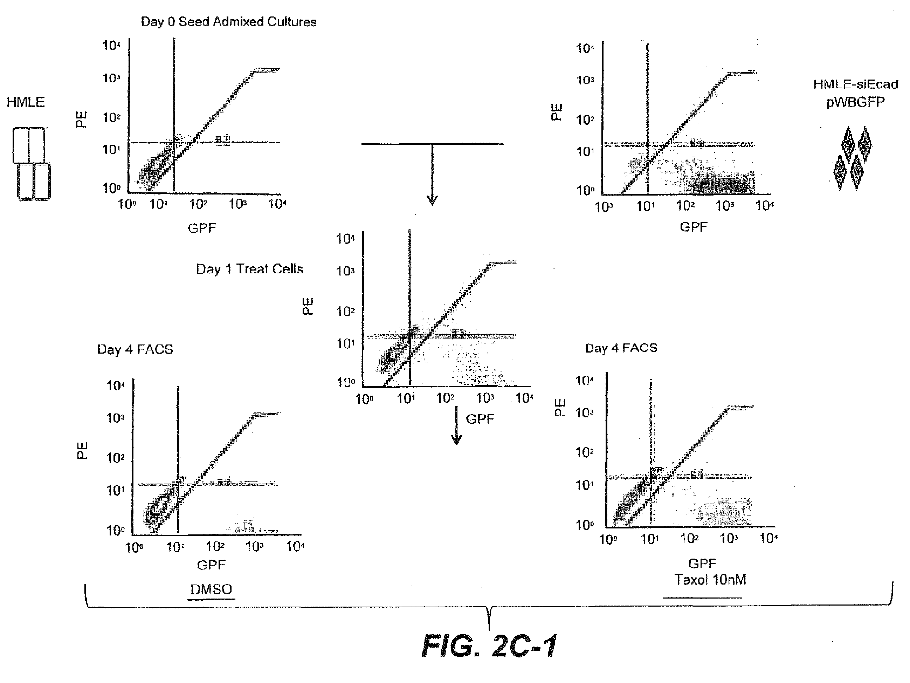

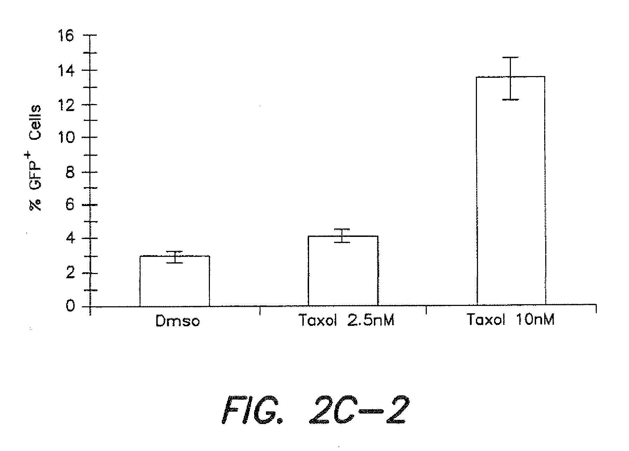

Fig. 2. depicts that passage through the EMT leads to resistance to conventional chemotherapy drugs. Fig. 2A depicts dose response curves of transformed HMLERshCntrl (light gray line) and HMLERshEcad (dark gray line) cells treated with Paclitaxel and Doxorubicin. Dotted line denotes the 50% fraction surviving. Note that for both drugs and any given concentration the HMLERshEcad cells are more resistant compared to the HMLERshCntrl cells. Fig. 2B depicts the response of immortalized cells to chemotherapy drugs. HMLEshCntrl and HMLEshEcad cells were treated with the indicated drugs at three concentrations for 3 days and assayed for viability using the MTS assay. While Doxorubucin, Actinomycin D, Paclitaxel and Campthotecin are chemotherapeutic drugs, Staurosporine is a general kinase inhibitor that potently induce apoptosis. Fig. 2C depicts paclitaxel treatment leads to selective expansion of a minority cell population that has undergone an EMT. Control HMLE cells and GFP expressing HMLEshEcad cells were mixed at a ratio of 20:1 and seeded onto 6-well plates in triplicate. DMSO or Paclitaxel at 2.5nm or 10nm concentration was added onto the mixed cell populations 1 day after seeding followed by a 3 day incubation. The number of GFP-positive cells at the end of treatment were analyzed by flow cytometry. The graph on the right shows the quantification of GFP-positivity. Note the increase in GFP positivity from 5% to 14% upon 10mM Paclitaxel treatment

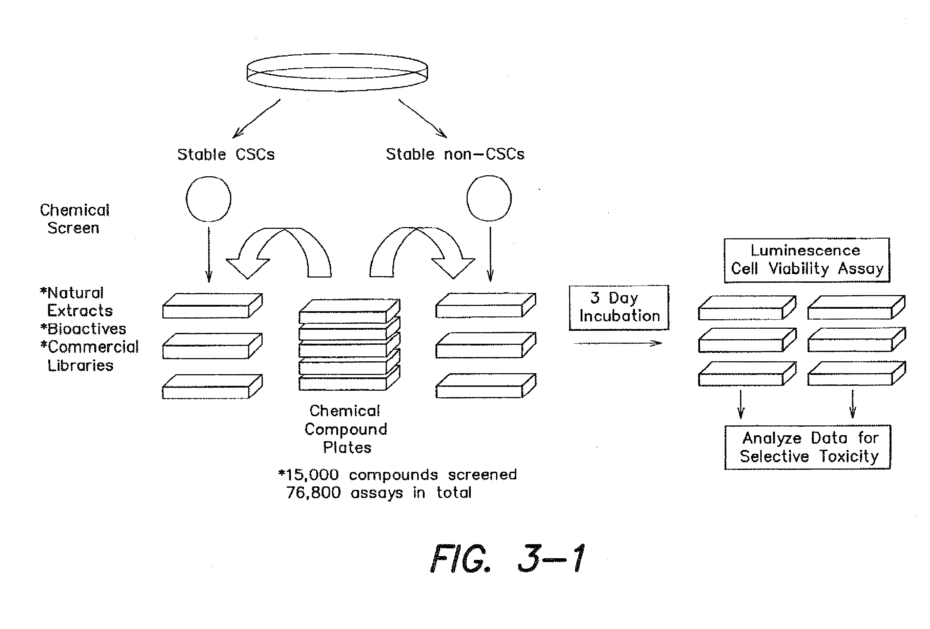

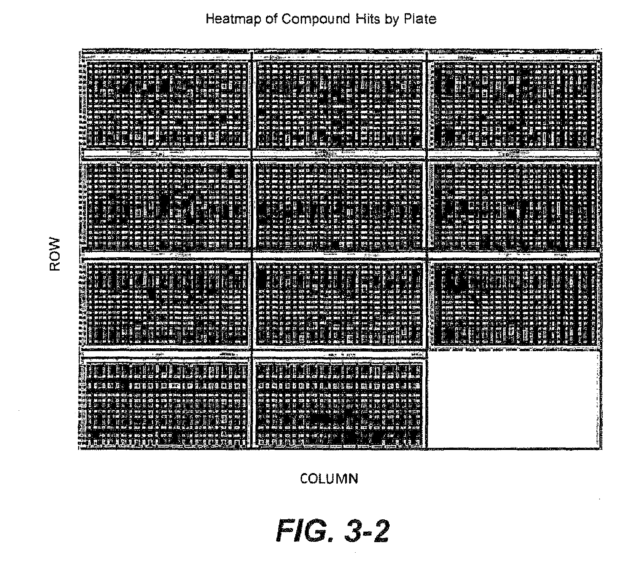

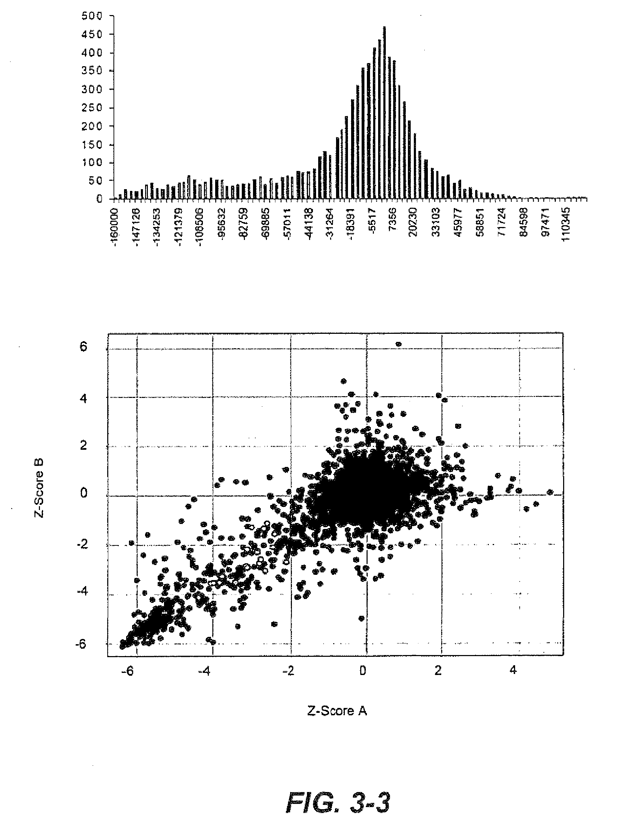

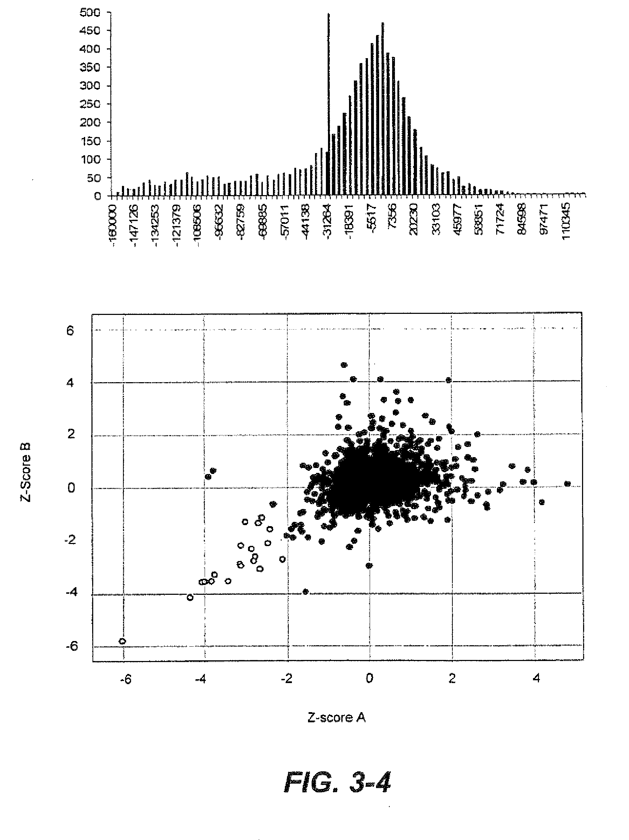

Fig. 3 depicts high throughput screening to identify compounds with Cancer stem cell specific toxicity and provides (A) schematic depicting the screen design; (B) evidence that the location of hits do not display any significant positional bias, and (C) the distribution of z-scores from the initial screen

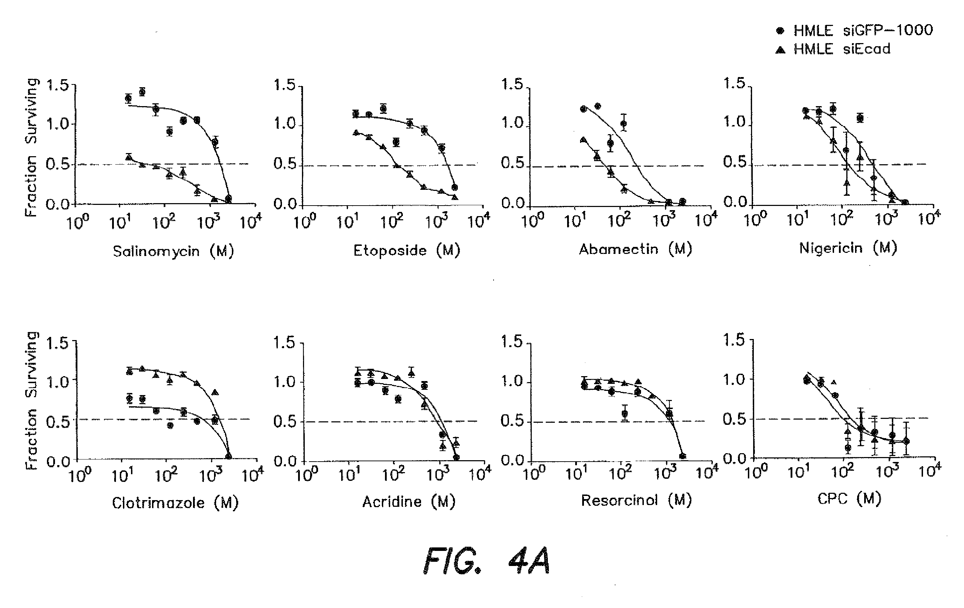

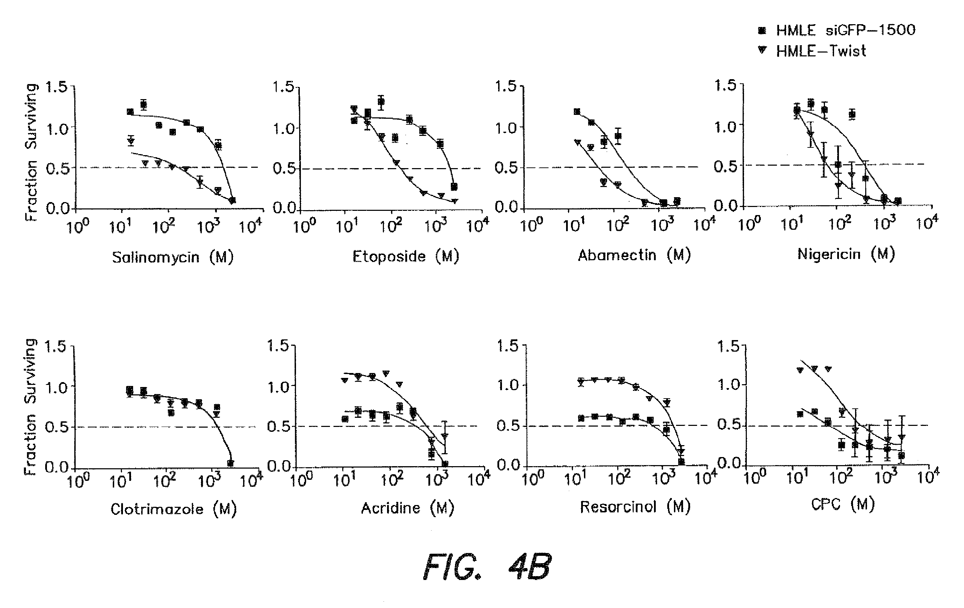

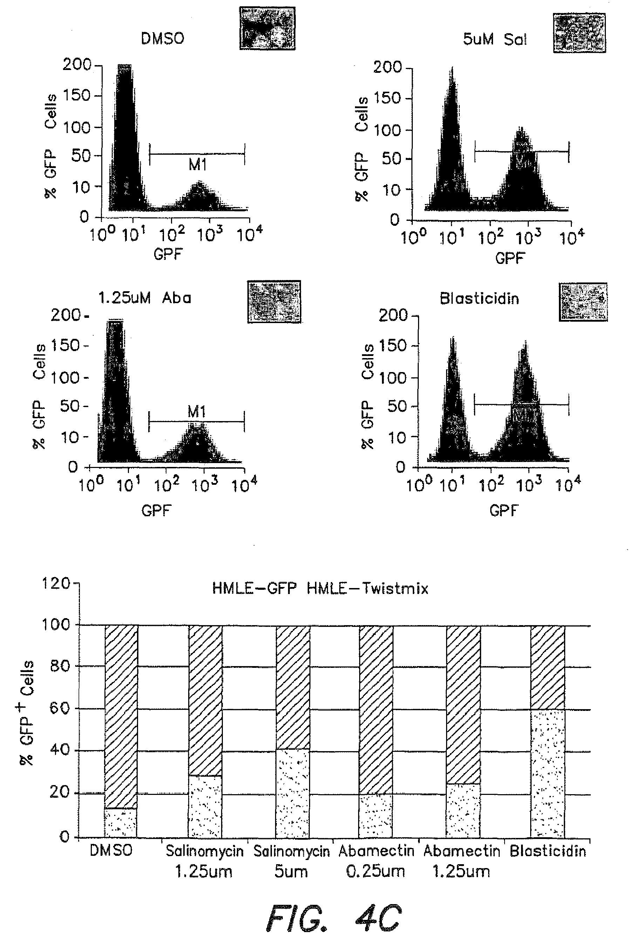

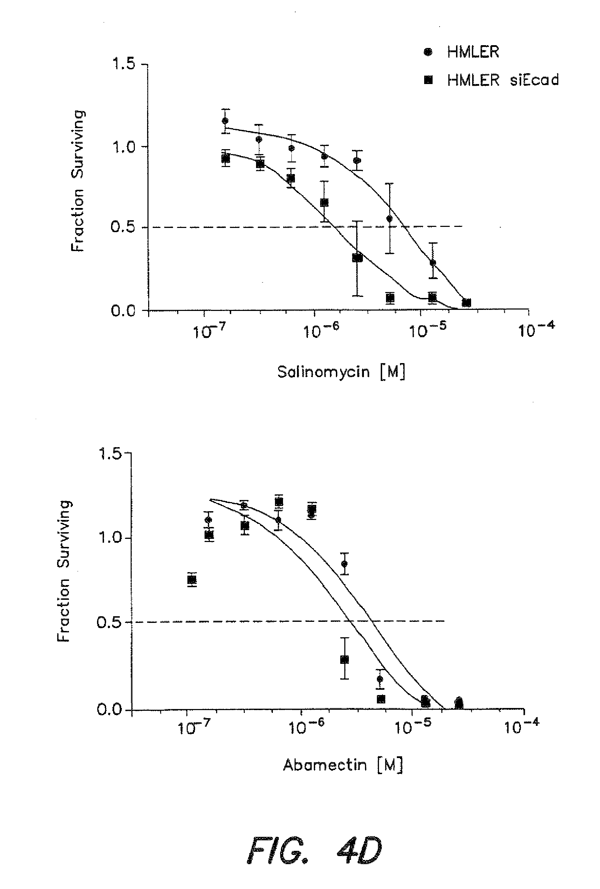

Fig. 4A depicts dose response curves of HMLEshCntrl (A, light gray curve) and HMLEshEcad (A, dark gray curve) cells treated with the 8 compounds identified during the initial screen. 1000 cells of each line were seeded onto 384 well plates and treated with 8 different concentrations of compounds within a 2.5 log range. The cell viability after 3 day treatment was measured by Cell-Titer-Glo. Note that while the top four compounds (Left-to-Right: Salinomycin, Abamectin, Etoposide and Nigericin) exhibit selective toxicity towards the HMLEshEcad cells the bottom four compounds (Left-to-Right: Clotrimazole, Acridine, Resorcinol, and CetylPyridinium Chloride, CPC) do not display such a behaviour. Fig. 4B depicts dose response curves of HMLEshCntrl (curve with square data points) and HMLETwist (curve with triangular data points) cells treated with the same compounds as in (A). Note that selectivity of Salinomycin, Abamectin, Etoposide and Nigericin (Top, Left-to-Right) is retained even though a different method of EMT induction is used. (Bottom panel left-to-right: Clotrimazole, Acridine, Resorcinol, and CetylPyridinium Chloride, CPC) Fig. 4C depicts control GFP-expressing HMLE cells and unlabelled HMLE-twist cells were mixed and plated onto 10-cm dishes. 1 Day after seeding DMSO, Salinomycin (1.25 or 5 µM) or Abamectin (0.25 or 1.25 µM) was added at the indicated concentration. As the GFP vector contains a Blasticidin resistance gene, we used Blasticidin treatment as a positive control for the expansion of GFP-expressing control HMLE cells. After 3 day incubation the mix population of cells were analyzed by flow cytometry and the percentage of GFP-positive cells were determined. Note that both salinomycin and abamectin treatment leads to a selection against the GFP-negative HMLE-twist cells and a relative expansion of the GFP-positive control cells. Fig. 4D depicts dose response curves of transformed HMLERshCntrl (light gray line) and HMLERshEcad (dark gray lines) cells treated with Salinomycin and Abamectin. Dotted line denotes the 50% fraction surviving. Note that the selective toxicity of Salinomycin against cells that have undergone an EMT remains even when the cells are transformed.

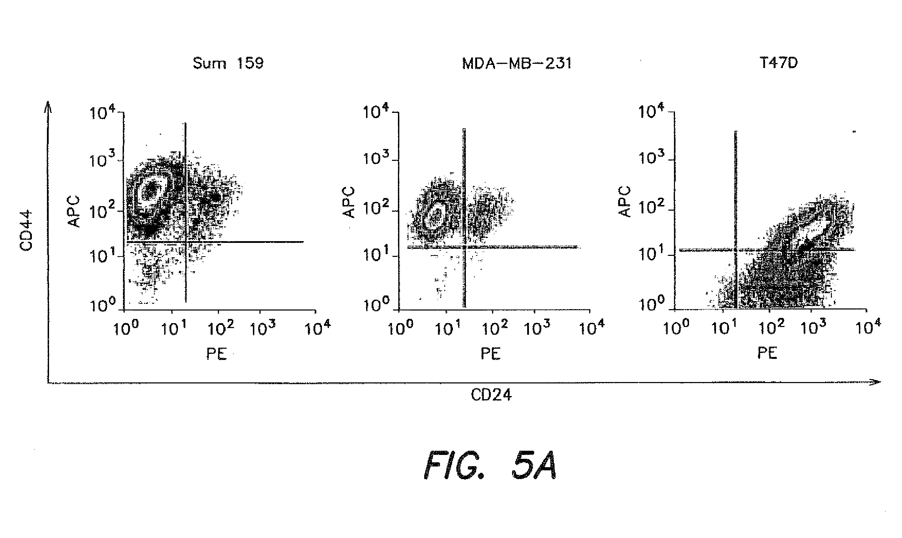

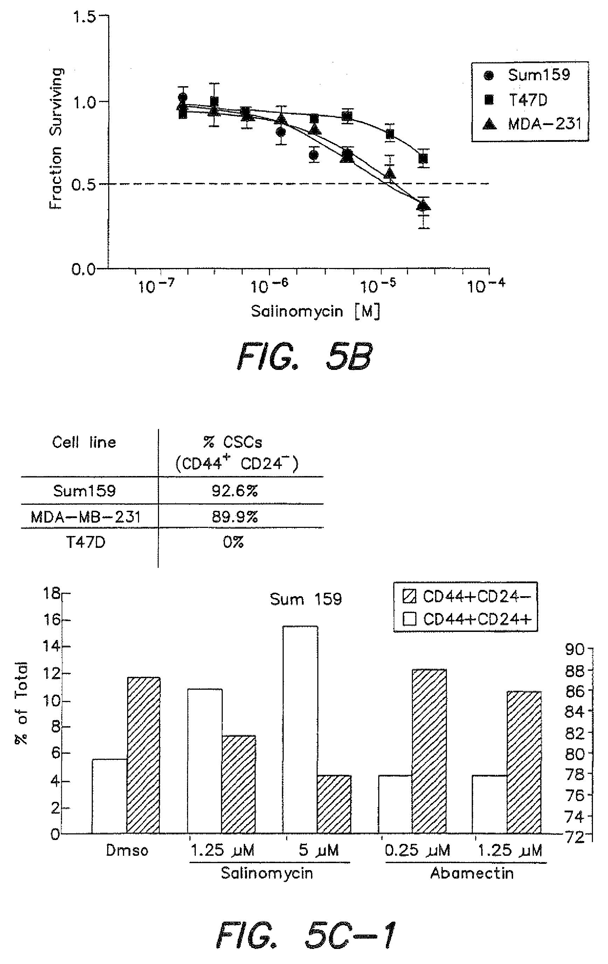

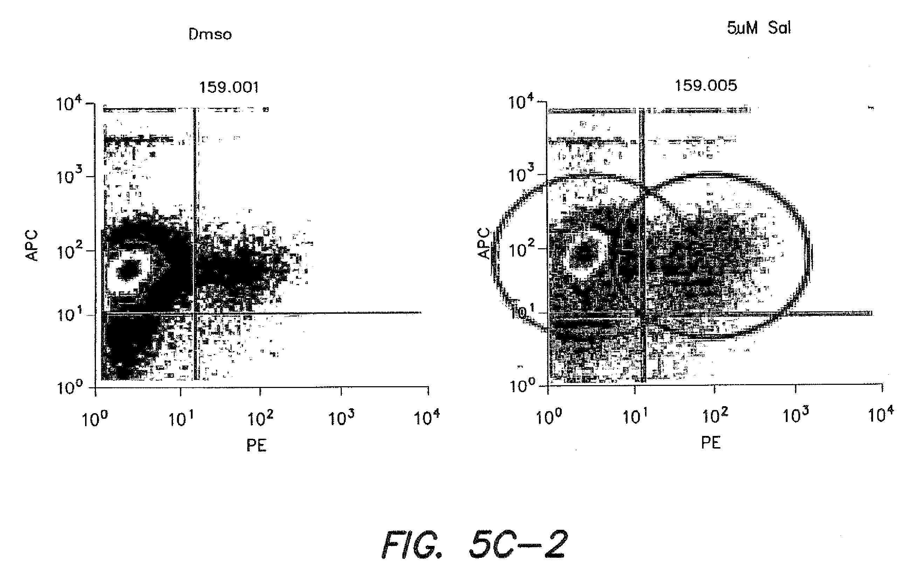

Fig. 5A depicts flow cytometry analysis of Sum159, MDA-MB-231 and T47D cell lines with respect to cell surface expression of CD44 and CD24. Note that while the majority of cells in Sum159 and MDA-MB-231 line contain large amounts of CSCs, T47D cells have very few such cells. Fig 5B depict dose response curves of Sum159, MDA-MB-231 and T47D cell treated with Salinomycin. Fig. 5C depicts a decrease in the CSC population (light gray bars) within Sum159 cell line and the concomitant increase in the non-CSC population (dark gray bars) in response to treatment with Salinomycin.

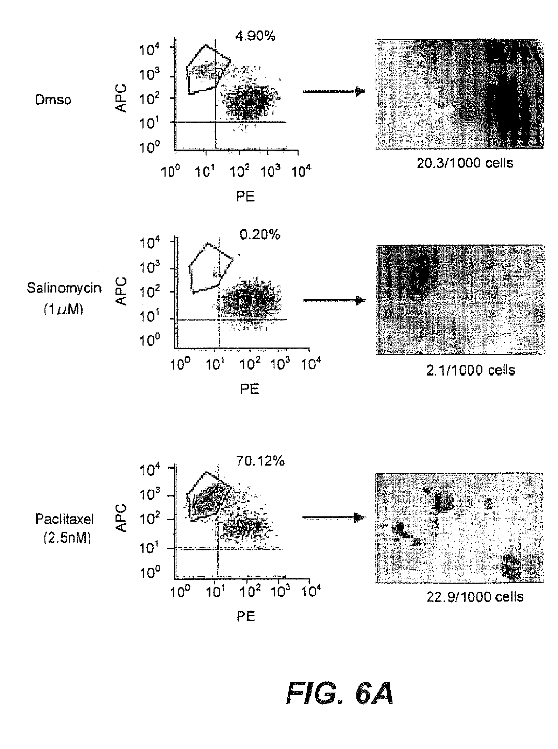

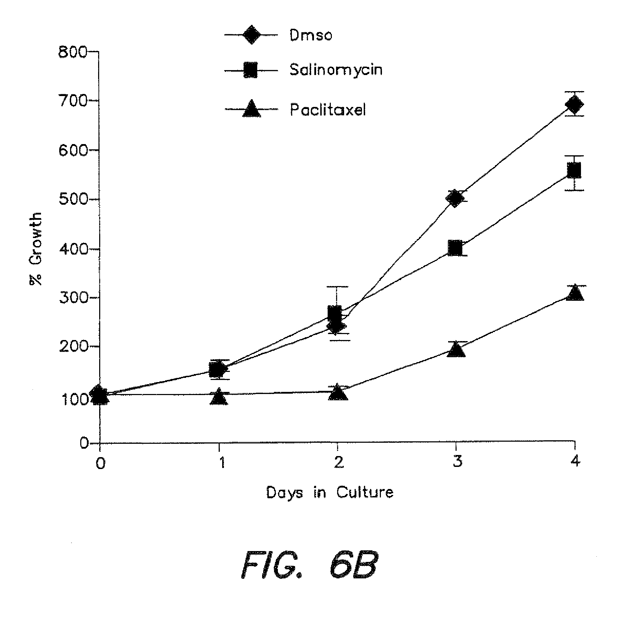

Fig 6 depicts effects of salinomycin on the CSC population in HMLER cell line. (A) HMLER cells were treated with vehicle control (dmso), salinomycin or paclitaxel at the indicated concentrations for 15 days. FACS analysis were performed at the end of treatment to gauge the CD44+/CD24- CSC population (right panels). Note that salinomycin decreases the CD44+/CD24- population from a basal of 4.9% to 0.2%. Treated cultures were also subjected to the mammosphere formation assay (left panels). Number of mammopsheres formed per 1000 cells from each culture are indicated below the representative photographs of assay wells. (B) Growth rates of HMLER cultures after treatment with vehicle control (dmso), salinomycin or paclitaxel at the indicated concentrations in (A) for 15 days.

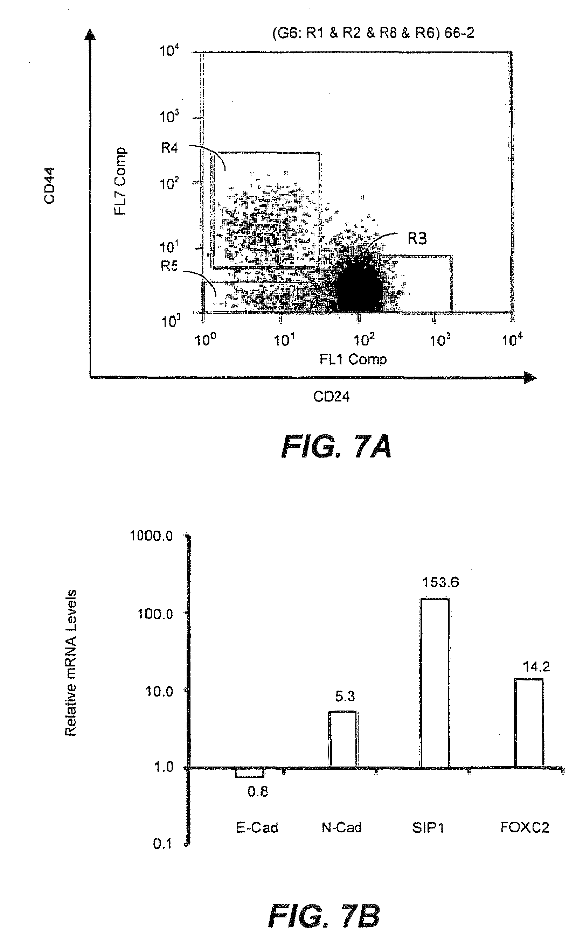

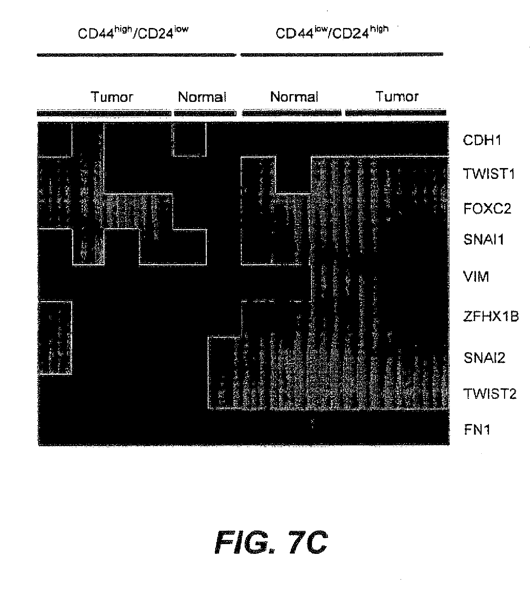

Figure 7 depicts that primary mouse mammary stem cells, normal human breast stem-like cells, and neoplastic human breast stem-like cells express markers associated with EMT. (A) CD44high/CD24low cells (R4) and CD44low/CD24high cells (R3) were isolated from human reduction mammoplasty tissues using FACS. (B) The expression levels of the mRNAs encoding E-cadherin, N-cadherin, SIP-1 and FOXC2 in CD44high/CD24low cells (R4) relative to CD44low/CD24high cells (R3), as determined by Real-time RT-PCR. GAPDH mRNA was used to normalize the variability in template loading. The data are reported as mean +/- SEM. (C) Heat map depicting the expression levels of mRNAs encoding EMT markers in CD44high/CD24low cells compared to CD44low/CD24high cells, as determined by SAGE analysis. Dark gray and light gray squares correspond to high and low mRNA levels, respectively.

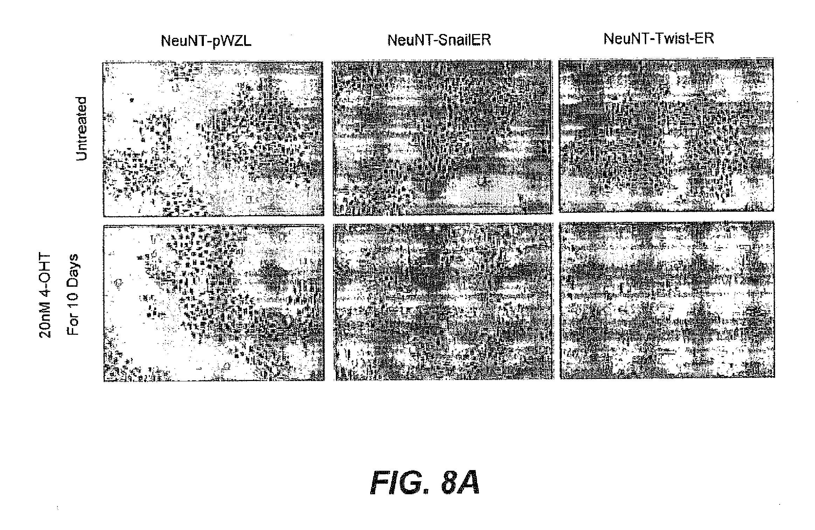

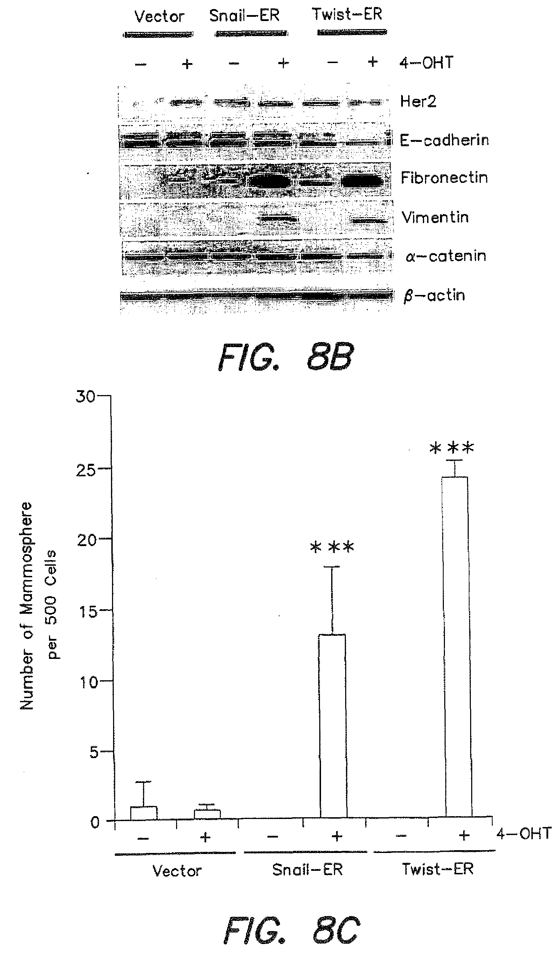

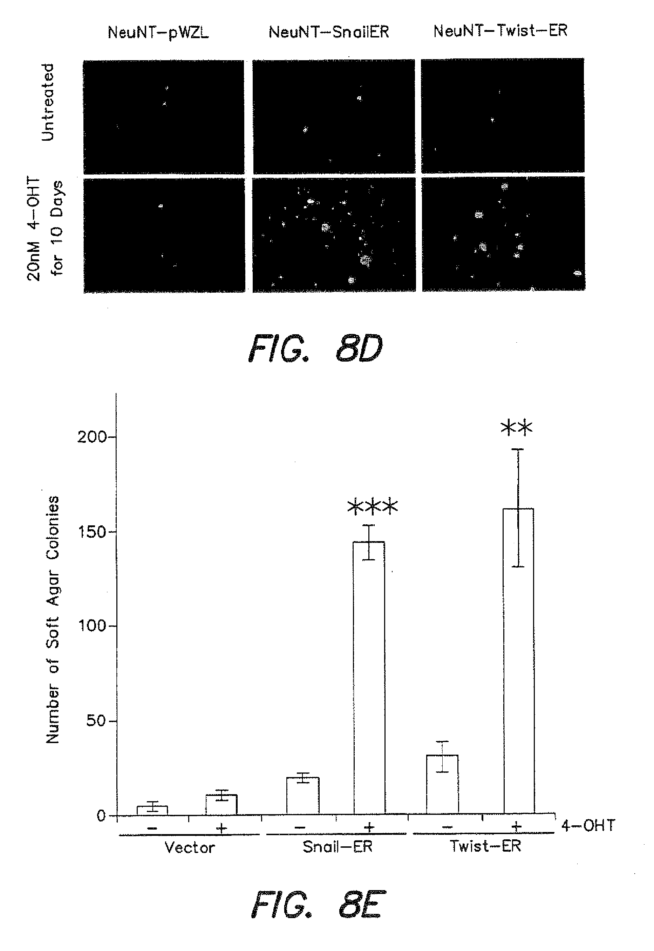

Figure 8 depicts that EMT induces phenotypes associated with cancer stem cells. (A) Phase-contrast images ofNeuNT-Snail-ER, NeuNT-Twist-ER and NeuNTcontrol vector cells treated with tamoxifen for a period of 10 days as well as images of untreated cells. (B) Western blot analysis of expression of HER2/neu, E-cadherin, fibronectin, and vimentin proteins in the cells shown in panel A. β-actin was used as a loading control. (C) Quantification of the mammospheres seeded by NeuNT-Snail-ER, NeuNT-Twist-ER or NeuNTcontrol vector cells treated or not treated with tamoxifen for 10 days. The data are reported as mean +/- SD. (D) Images of the colonies formed during soft agar culture of NeuNT-Snail-ER, NeuNT-Twist-ER and NeuNT-control vector cells after being treated with tamoxifen for 10 days. The soft agar assays were performed in the absence of tamoxifen. (E) Quantification of the soft agar colonies shown in panel D. The data are reported as mean +/-SD (** - P<0.01; *** - P<0.001 compared to the control).

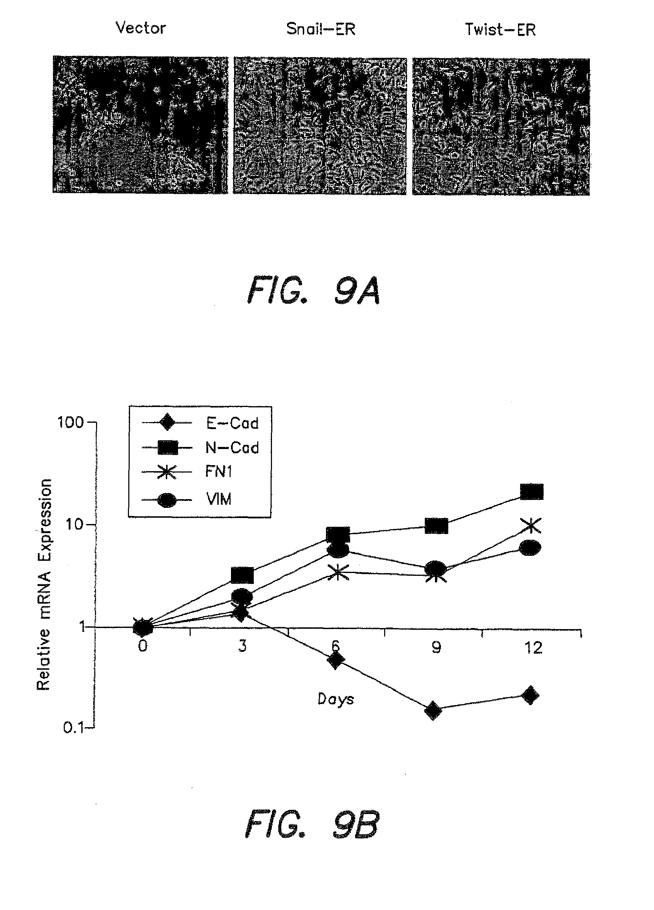

Figure 9 depicts that induction of EMT by either Snail-ER or Twist-ER tamoxifeninducible vectors generates cells with stem-cell properties (A) Phase-contrast images of Snail-ER, Twist-ER or control vector cells treated with tamoxifen for 12 days. (B). Expression levels of mRNAs encoding proteins associated with an EMT, as observed in HMLE cells induced to undergo EMT by ectopic expression of Snail-ER (tamoxifen-induced). The expression levels are reported relative to Day 0 of the 12-day tamoxifen treatment. GAPDH mRNA was used to normalize variability in template loading. The data are reported as mean +/- SEM.

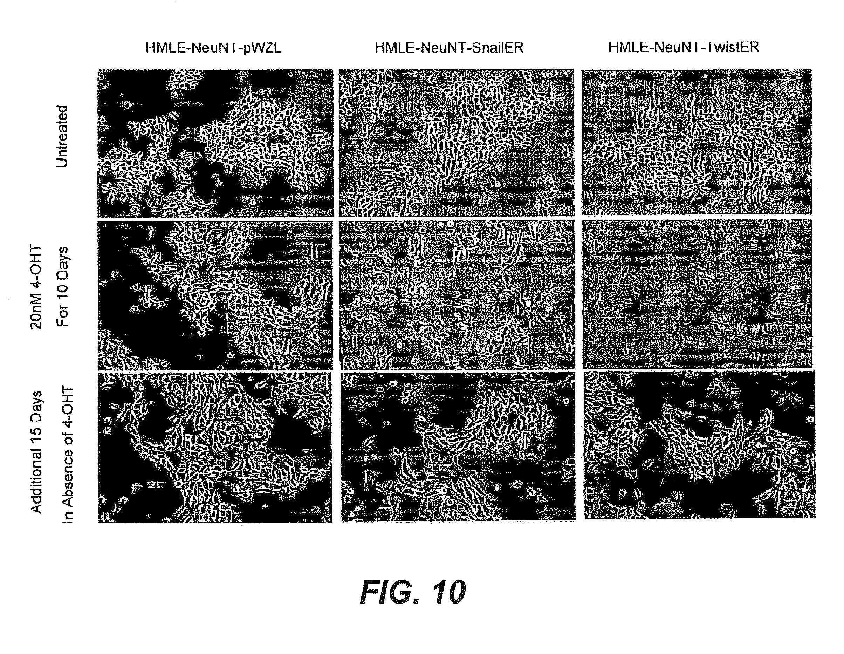

Figure 10 depicts phase contrast images of HMLEN-Snail-ER or HMLEN-Twist-ER cells that underwent EMT after ten days of tamoxifen (4-OHT) treatment (middle row) or were left untreated (top row). Following this 4-OHT treatment, 4-OHT was withdrawn and cells were observed 15 days later. Uninfected cells or cells infected with the indicated viral vectors are shown in the columns.

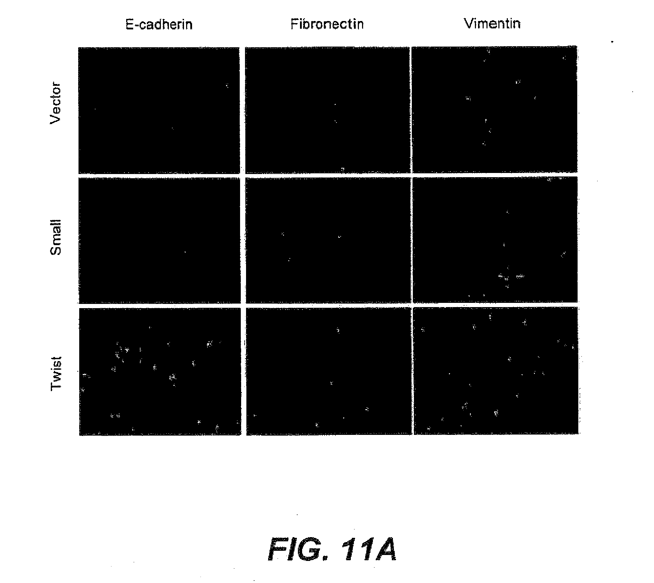

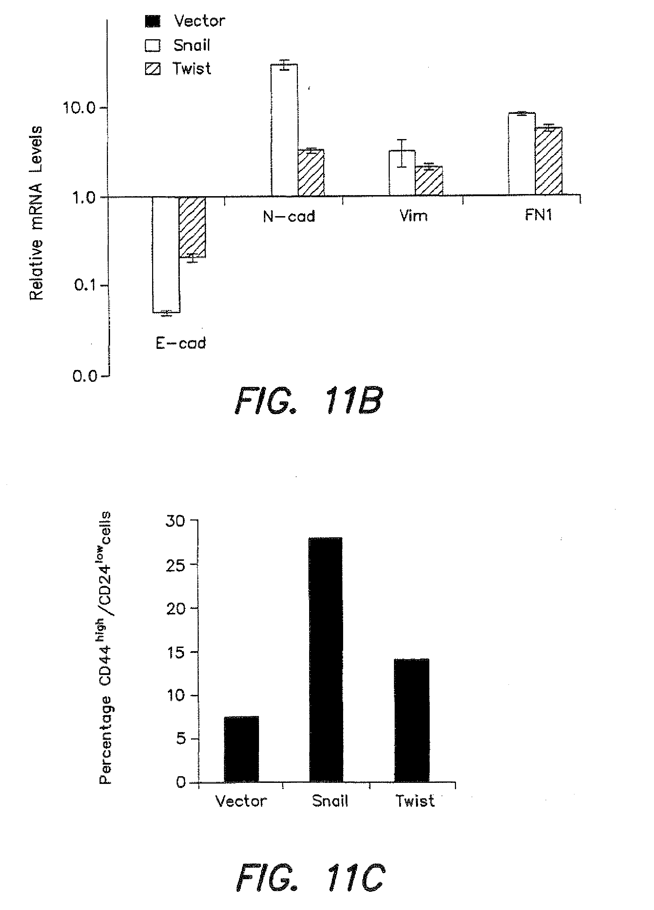

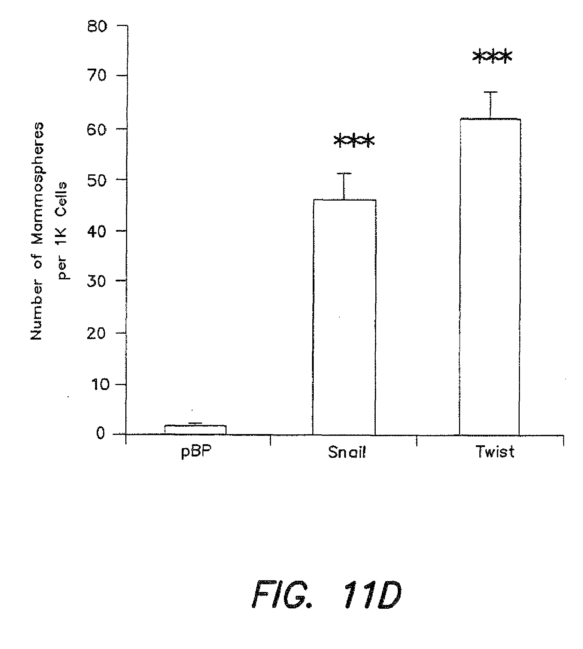

Figure 11 depicts induction of an EMT in HMLER cells using Snail, Twist, or a control vector (A). Immunostaining of HMLER cells induced to undergo EMT by ectopic expression of Snail or Twist, using antibodies against E-Cadherin, N-cadherin, fibronectin and vimentin. Immunostaining of control cells is shown for comparison (B). Expression of mRNAs associated with EMT in HMLERs that were induced to undergo EMT by the ectopic expression of Snail or Twist relative to control vector-infected cells, as determined by Real-time RT-PCR. GAPDH mRNA was used to normalize variability in template loading. The data are reported as mean +/- SEM. (C). The percentage of CD44high/CD24low in cells in HMLER cells that underwent an EMT induced by ectopic expression of Snail, Twist or control vector. (D). Number of mammosphere/1000 HMLER cells expressing Snail, Twist or control vector. The data are reported as mean +/- SEM.

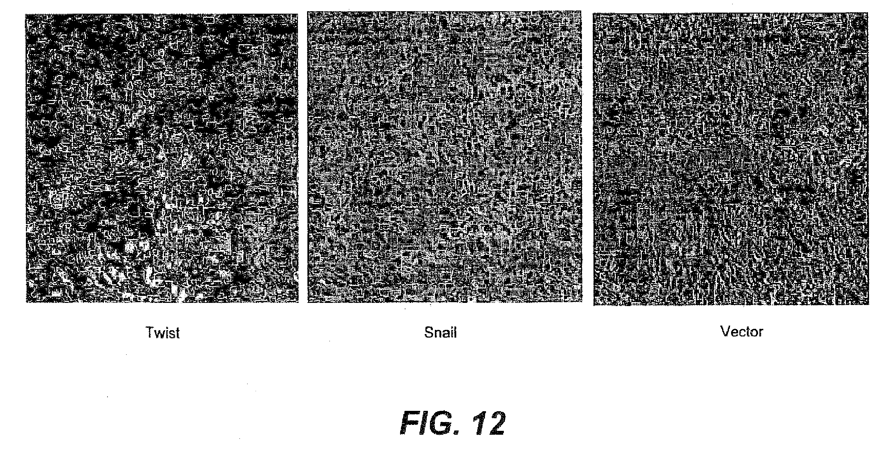

Figure 12 depicts H&E staining of tumors derived from HMLER cells expressing Snail, Twist, or the control vector. Light microscope images (10X) of the tumors derived from (A). Vector (B). Snail (C). Twist expressing HMLER cells.

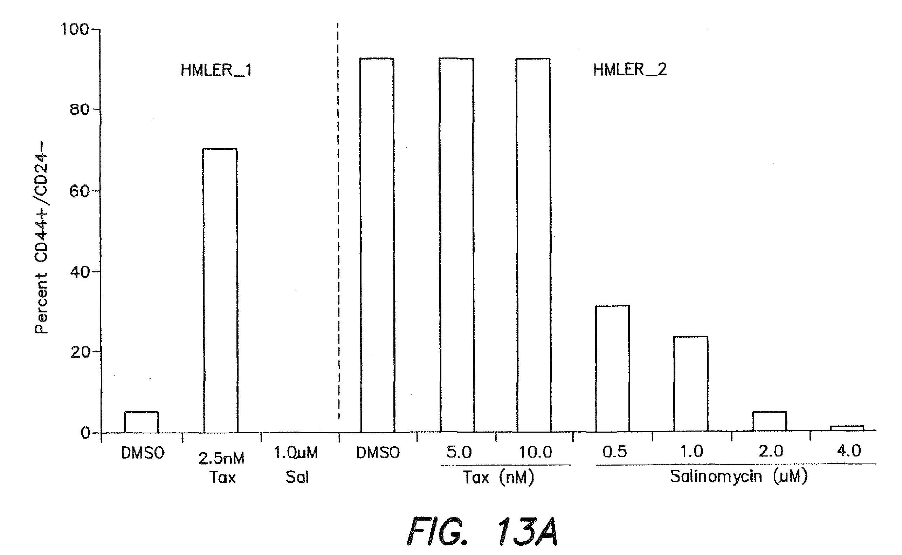

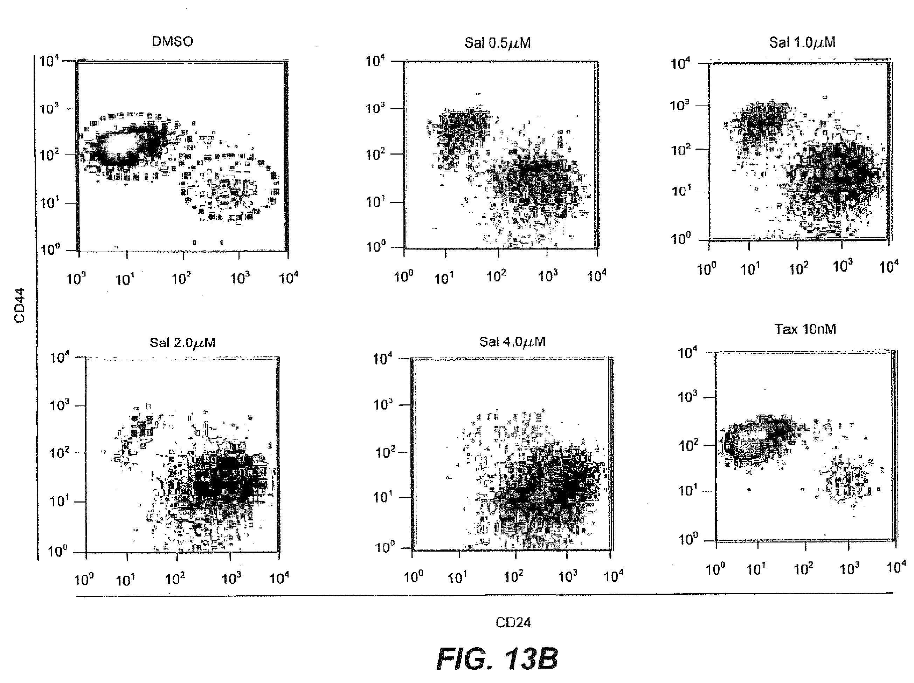

Figure 13A depicts HMLER cells that were treated with DMSO, paclitaxel or salinomycin for 4 days, at the specified doses. The percent of CD44^{high}/CD24^{low} cells following compound treatment is shown and was quantified by fluorescence-activated cell sorting. The bar charts depict the results of two independent experiments with two different HMLER cell populations (HMLER)_1, HMLER_2). Figure 13B depicts fluorescence-activated cell sorting profiles of HMLER_2 cancer cell populations treated with chemical compounds shown (CD44 vs. CD24). The upper left ellipse (----) denotes the CSC-enriched fraction and the lower left ellipse (----) the CSC-depleted fraction.

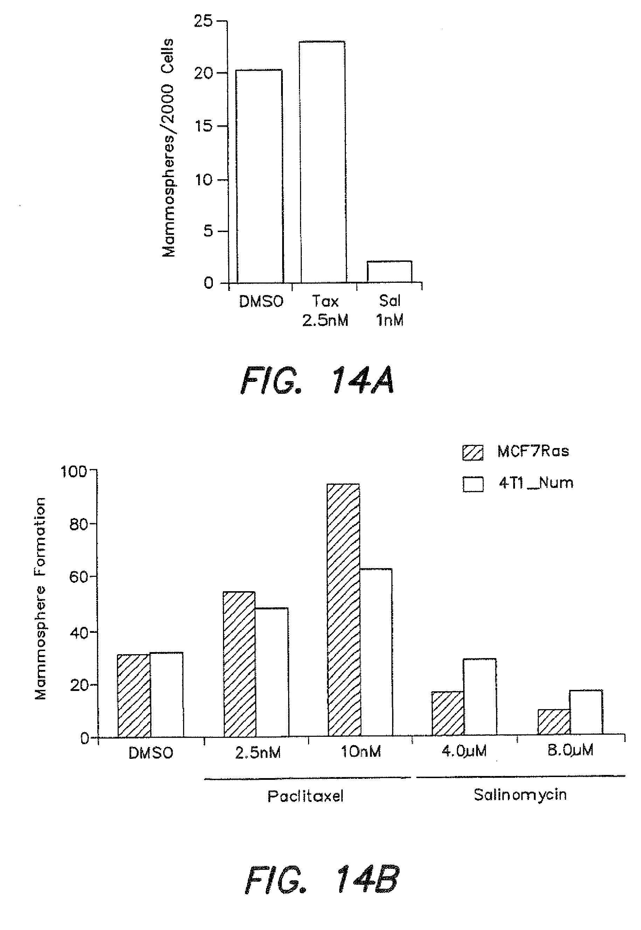

Figure 14A depicts the mammosphere forming potential of parental HMLER cells treated with either DMSO, paclitaxel (taxol) or salinomycin at the specified doses. Figure 14B depicts MCF7Ras or 4T1 cells that were treated with DMSO, paclitaxel or salinomycin. Mammosphere-forming potential is shown below.

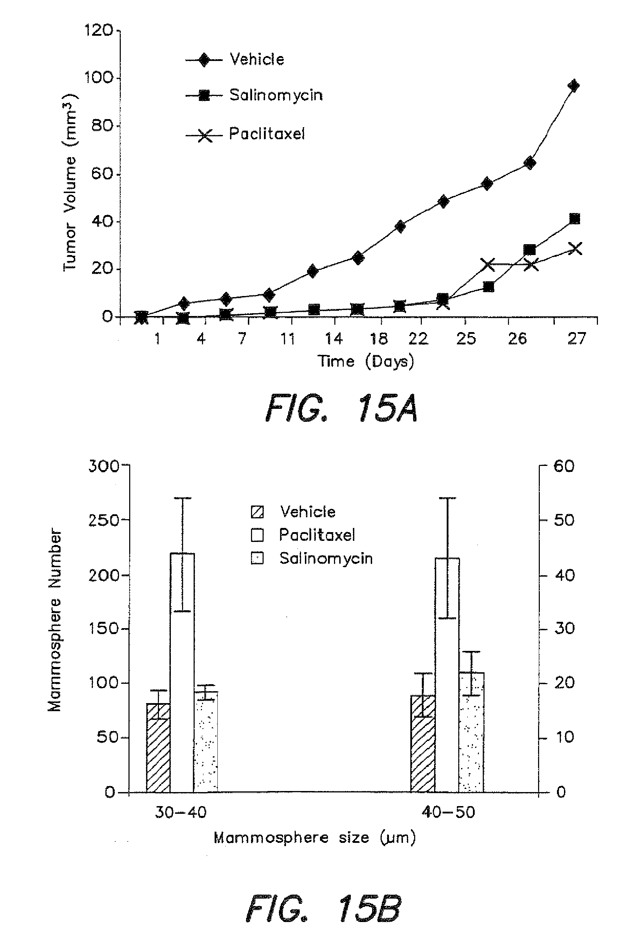

Figure 15A depicts *in vivo* tumor formation by SUM159 breast cancer cells in mice that were treated with salinomycin, paclitaxel or vehicle. Salinomycin treatment leads to an inhibition of tumor growth. Figure 15B depicts the mammosphere-forming potential of cancer cells obtained from SUM159 tumors from salinomycin, paclitaxel or DMSO-treated mice.

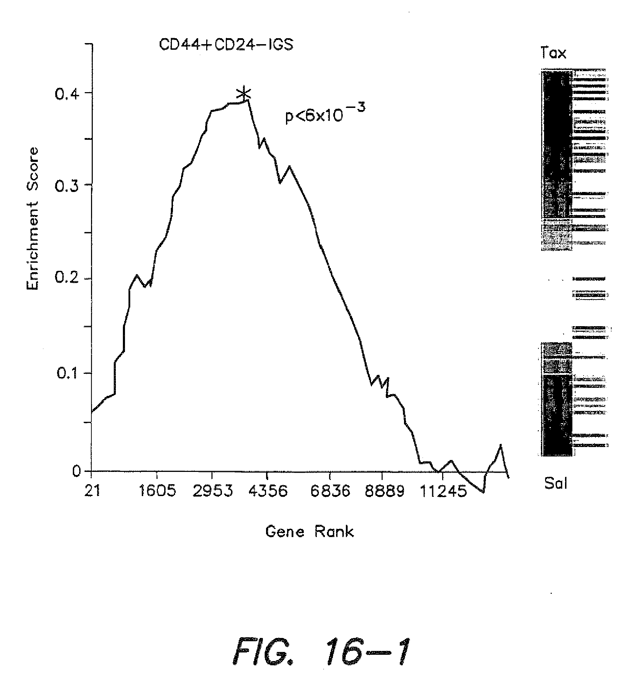

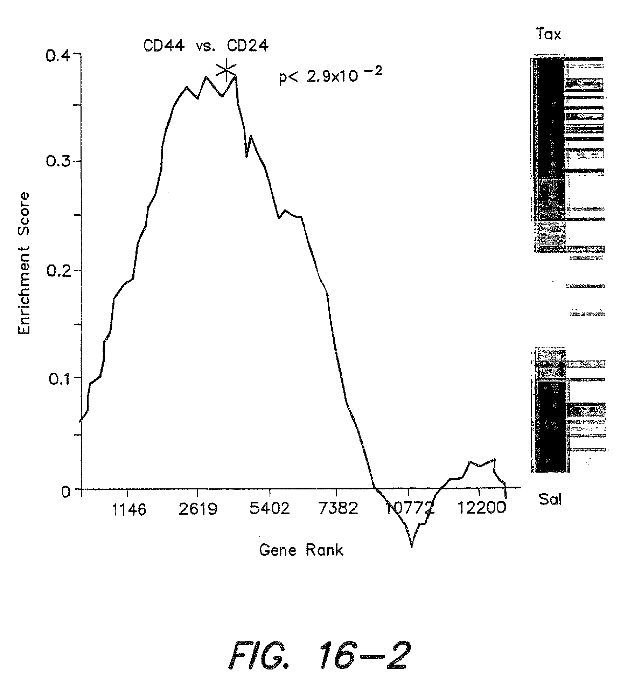

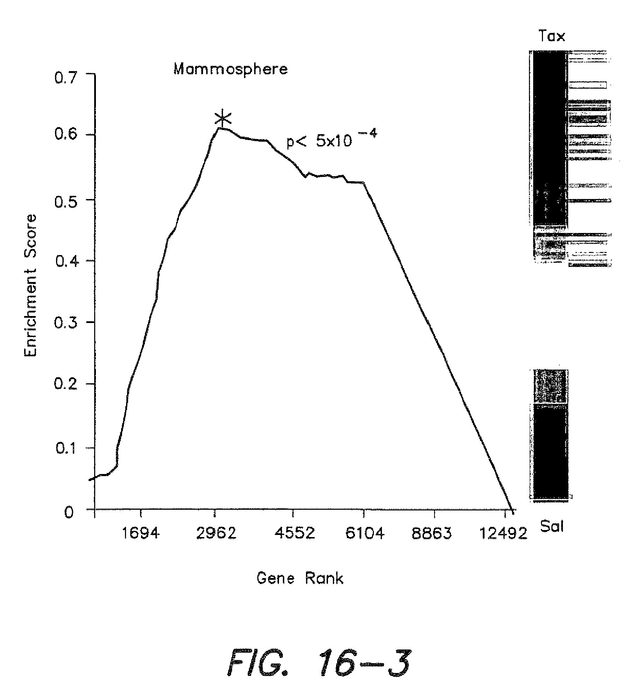

Figure 16 depicts that salinomycin treatment reduces the expression of clinically relevant breast CSC and progenitor genes. Gene set enrichment analysis was used to determine whether three previously reported sets of genes associated with stem-like cells were repressed in response to salinomycin in comparison with paclitaxel treatment. Graphed are the Kolmogorov-Smirnov enrichment scores versus Gene ranks based on differential expression. P-values reflecting statistical significance for each analysis are shown.

### DETAILED DESCRIPTION

Tumorigenesis involves a number of genetic and epigenetic alterations that cause defects in cell proliferation, differentiation, growth, and survival. These cellular defects give rise to tumors that can be either benign or malignant. Whereas benign tumors often remain localized in a primary tumor that remains localized at the site of origin and that is often self limiting in terms of tumor growth, malignant tumors have a tendency for sustained growth and an ability to spread or metastasize to distant locations. Malignant tumors develop through a series of stepwise, progressive changes that lead to uncontrolled cell proliferation and an ability to invade surrounding tissues and metastasize to different organ sites.

Recent findings have demonstrated that tumor formation and growth are driven by a minor subpopulation of cancer cells within tumors, cancer stem cells (Al-Hajj M, Wicha MS, Benito-Hernandez A, Morrison SJ, Clarke MF., Proc Natl Acad Sci U S A 2003;100(7):3983-8) (Li C, Heidt DG, Dalerba P, et al., Cancer Res 2007;67(3):1030-7) (O'Brien CA, Pollett A, Gallinger S, Dick JE., Nature 2007;445(7123):106-10) (Ricci-Vitiani L, Lombardi DG, Pilozzi E, et al., Nature 2007;445(7123):111-5) (Singh SK, Hawkins C, Clarke ID, et al., Nature 2004;432(7015):396-401). Cancer stem cells (CSCs) are defined functionally as those cells within a tumor mass that have the capacity to seed and generate secondary tumors. In some aspects, CSCs have been operationally defined by their ability to seed tumors at limiting dilutions in animal models. Implicit in this functional categorization is the notion that cancer stem cells are the cells within tumors responsible for the primary cause of cancer mortality--metastatic dissemination. This concept has significant implications for the development and preclinical assessment of potential cancer therapies. The present invention describes a new method that enables the discovery of novel therapeutics that target cancer stem cells. The discovery of cancer stem cell-targeted therapies was not possible in a high-throughput screening setting prior to the invention of the method described herein. We establish a proof-of-principle for our invention by discovering novel compounds that specifically target cancer stem cells through reducing the described method to practice. Thus, the present invention makes possible for the first time the identification of therapies that target cancer stem cells using high-throughput screening methods.

### Epithelial to Mesenchymal Transition:

Normal and tumor cells exist in various states of differentiation in vitro and in vivo. These differentiation states are regulated through the integration of complex signals arising at least in part from the tissue microenvironment in which a cell resides. Cells (e.g., cancer cells) can be induced to undergo an epithelial-to-mesenchymal transition (EMT) through a number of genetic perturbations, e.g. via the overexpression of particular factors (e.g., Twist, Snail, TGF-beta, or MMPs), or by the inhibition of adherens junction proteins such as E-Cadherin. Cells that have been induced into EMT, irrespective of the inducing method used, express similar phenotypic traits and protein markers (e.g.,, biomarkers), indicating that the EMT is a core differentiation program. The present invention relates to the discovery that cells that have been induced into EMT share many of the properties of cancer stem cells, including the expression of cell surface markers associated with cancer stem cells, growth in suspension culture, tumor formation at low cell numbers in vivo, and resistance to certain standard chemotherapy drugs. Thus, the state of differentiation exhibited by cells that have undergone an epithelial-to-mesenchymal transition, also referred to as mesenchymal transdifferentiation, or epithelial-to-mesenchymal transdifferentiation, can be exploited to identify therapies that specifically target cancer stem cells. In some embodiments, methods are provided to induce an epithelial-mesenchymal transition (e.g., to produce test cells).

As used herein, an "epithelial to mesenchymal transition" (EMT) refers to a transformation, or partial transformation, of an epithelial cell into a cell having one or more mesenchymal charateristics that also has one or more properties of a cancer or non-cancer stern cell. The one or more cancer or non-cancer stem cell properties may include the presence or absence (high expression levels or low expression levels) of one or more proteins (e.g., cell surface markers) and/or an increase in one or more (at least 2, at least 3, at least 4, at least 5, at least 6) functional properties including the ability to grow (proliferate) in suspension cultures, ability to form tumors *in vivo* at low cell seeding numbers, resistance to certain chemotherapies (e.g., resistance to paclitaxel), metastatic ability, migration ability, resistance to apoptosis or anoikis, scattering, and elongation of cell shape. It is to be understood that the extent to which a cell that has undergone an EMT exhibits an increase or decrease in the expression of one or more proteins or an increase in one or more functional properties of a cancer stem cell may be assessed by performing a comparison with a control cell, e.g., a cell that has not undergone an EMT (a negative control cell) or a cell that has undergone an EMT (a positive control cell), e.g., a cancer stem cell.

Examples of proteins for which increased expression in a cell that has undergone an EMT, compared with a cell that has not undergone an EMT, is indicative of the EMT includes N-cadherin, Vimentin, Fibronectin, Snail1 (Snail), Snail2 (Slug), Twist, Goosecoid, FOXC2, Sox10, MMP-2, MMF-3, MMP-9, Integrin vß6, CD44, and ESA. Examples of proteins for which decreased expression in a cell that has undergone an EMT, compared with a cell that has not undergone an EMT, is indicative of the EMT includes E-cadherin, Desmoplakin, Cytokeratin, Occludin, and CD24. The extent to which a epithelial cell has undergone an EMT may be assessed by determining the expression of at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, or more proteins. The expression of such proteins may be determined using a variety of assay methods including those disclosed herein and others which are known in the art. It is to be understood that some proteins whose expression levels are indicative of an EMT in a cell are also proteins that may cause the EMT to occur. For example, overexpression of Twist in an epithelial cell causes the cell to undergo an EMT. Similarly, a reduction in expression of E-cadherin in an epithelial cell causes the cell to undergo an EMT. Thus, when an EMT is brought about by increasing (e.g., by cDNA mediated overexpression) the expression of a protein such as Twist or decreasing (e.g., by RNAi mediated inhibition) the expression of a protein such as E-cadherin alterations in the expression of other proteins (e.g., CD44, Desmoplakin) may serve as suitable indicators of the EMT.

It is to be understood that functional properties of cancer or non-cancer stem cells may be assessed using a variety of methods known in the art. Growth in suspension cultures, for example, may be assessed by growing in a spinner flask cells that have undergone an EMT and measuring the change in cell number in the spinner flask over time. This change in cell number may be compared with the change in cell number of control cells that have not undergone an EMT and which have been grown under same or similar conditions. Up to a 14%, 20%, 30%, 40%, 50%, 60%, 70%, 80%, 90%, 100%, or more increase in doubling time of cells grown in suspension culture that have undergone an EMT compared with control cells grown under the same or similar conditions, but that have not undergone an EMT, may be indicative of an increased ability to grow in suspension.

The ability of cells that have undergone an EMT to form tumors *in vivo* at low cell seeding numbers may be assessed by a comparison with control cells that have not undergone an EMT. The ability to form tumors *in vivo* at low cell seeding numbers depends on a variety of factors which will be apparent to the skilled artisan, including for example the type of animal (e.g., a mouse) in which the cells are injected, the location where the cells are seeded (e.g., injected), and the ability of the animal to mount an immune response against the cells. As used herein, "low cell seeding numbers" means seeding of up to 10⁰, up to 10¹, up to 10², up to 10³, up to 10⁴, up to 10⁵, up to 10⁶ or more cells. The number of tumors formed *in vivo* following seeding of cells (e.g., a one or more locations or in one or more animals) is an exemplary parameter by which this comparison with a control may be made. The size (e.g., volume) of tumors formed *in vivo* following seeding of cells is another exemplary parameter by which this comparison with a control may be made. Up to a 14%, 20%, 30%, 40%, 50%, 60%, 70%, 80%, 90%, 100%, or more increase in tumor incidence or size, for example, may be indicative of an increased ability to form tumors *in vivo* at low cell seeding numbers.

Other methods for assessing functional properties of cancer or non-cancer stem cells are disclosed herein and elsewhere, and will be apparent to the skilled artisan. (See, e.g., SM Frisch and H Francis, Disruption of epithelial cell-matrix interactions induces apoptosis, J Cell Biol. 1994 Feb;124(4):619-26; Mushinski JF, et al., Inhibition of tumor cell motility by the interferon-inducible GTPase MxA. J Biol Chem. 2009 Mar 18; Liu YN, et al., Activated androgen receptor downregulates E-cadherin gene expression and promotes tumor metastasis. Mol Cell Biol. 2008 Dec;28(23):7096-108; Carpenter PM, et al., Motility Induction in Breast Carcinoma by Mammary Epithelial Laminin 332 (Laminin 5), Mol Cancer Res. 2009 Apr 7; Nakamura M, et al., Polarized hydroxyapatite promotes spread and motility of osteoblastic cells. Biomed Mater Res A. 2009 Mar 9; Gu W, et al., Measuring cell motility using quantum dot probes. Methods Mol Biol. 2007;374:125-31; and Sakai K, et al., Inducible expression of p57KIP2 inhibits glioma cell motility and invasion. J Neurooncol. 2004 Jul;68(3):217-23.)

The present invention discloses methods for inducing EMT in cells. In certain embodiments, the epithelial to mesenchymal transition is brought about by inhibiting the expression or activity of E-Cadherin in the cell. The expression or activity of E-Cadherin can be inhibited by using methods known to one of ordinary skill in the art. Exemplary methods for inhibiting E-cadherin expression or activity include contacting a cell with a small interfering nucleic acid complementary to E-Cadherin mRNA, contacting a cell with a blocking antibody to E-cadherin; inducing the expression of dysadherin, for example by cDNA-based overexpression of dysadherin, in a cell; and interfering with cell-polarity genes in the cell. For example, depletion of Scribble disrupts E-cadherin-mediated cell-cell adhesion and induces EMT (Qin Y, et al., J Cell Biol 2005;171:1061-71). Thus, in some embodiments, inhibition of Scribble, such as by RNA interference, can induce an EMT. Also, genetic screens in *Drosophila* identified a number of genes affecting cell polarity whose inactivation results in loss of E-cadherin expression (Pagliarini RA, et al., Science 2003;302:1227-31). Other exemplary methods for interfering with cell polarity genes to induce EMT are known in the art.

In certain embodiments, the activity of E-cadherin is inhibited by RNA interference. Methods for inhibiting gene expression, such as E-cadherin expression, by RNA interference are disclosed herein and known in the art. In some embodiments, a cell is transfected with a small interfering nucleic acid complementary to E-Cadherin mRNA in the cell to inhibit E-cadherin activity in the cell. Exemplary small interfering nucleic acids are disclosed herein and are known to persons skilled in the art. Methods for transfection of small interfering nucleic acids (e.g., siRNA) are well known in the art and examples are disclosed herein. In some embodiments, the cell has a stably integrated transgene that expresses a small interfering nucleic acid (e.g., shRNA, miRNA) that is complementary to E-Cadherin mRNA and that causes the downregulation of E-Cadherin mRNA through the RNA interference pathway.

Various strategies for gene knockdown known in the art can be used to inhibit the expression of a gene, for example E-cadherin and others disclosed herein that are useful for inducing EMT. For example, gene knockdown strategies may be used that make use of RNA interference (RNAi) and/or microRNA (miRNA) pathways including small interfering RNA (siRNA), short hairpin RNA (shRNA), double-stranded RNA (dsRNA), miRNAs, and other nucleotide-based molecules known in the art. In one embodiment, vector-based RNAi modalities (e.g., shRNA or shRNA-mir expression constructs) are used to reduce expression of a gene (e.g., E-cadherin) in a cell.

A broad range of RNAi-based modalities could be also employed to inhibit expression of a gene in a cell, such as siRNA-based oligonucleotides and/or altered siRNA-based oligonucleotides. Altered siRNA based oligonucleotides are those modified to alter potency, target affinity, safety profile and/or stability, for example, to render them resistant or partially resistant to intracellular degradation. Modifications, such as phosphorothioates, for example, can be made to oligonucleotides to increase resistance to nuclease degradation, binding affinity and/or uptake. In addition, hydrophobization and bioconjugation enhances siRNA delivery and targeting (De Paula et al., RNA. 13(4):431-56, 2007) and siRNAs with ribodifluorotoluyl nucleotides maintain gene silencing activity (Xia et al., ASC Chem. Biol. 1(3):176-83, (2006)). siRNAs with amide-linked oligoribonucleosides have been generated that are more resistant to S1 nuclease degradation than unmodified siRNAs (Iwase R et al. 2006 Nucleic Acids Symp Ser 50: 175-176). In addition, modification of siRNAs at the 2'-sugar position and phosphodiester linkage confers improved serum stability without loss of efficacy (Choung et al., Biochem. Biophys. Res. Commun. 342(3):919-26, 2006). Other molecules that can be used to inhibit expression of a gene (e.g., a gene, such as E-Cadherin, that negatively regulates EMT) include sense and antisense nucleic acids (single or double stranded), ribozymes, peptides, DNAzymes, peptide nucleic acids (PNAs), triple helix forming oligonucleotides, antibodies, and aptamers and modified form(s) thereof directed to sequences in gene(s), RNA transcripts, or proteins. Antisense and ribozyme suppression strategies have led to the reversal of a tumor phenotype by reducing expression of a gene product or by cleaving a mutant transcript at the site of the mutation (Carter and Lemoine Br. J. Cancer. 67(5):869-76, 1993; Lange et al., Leukemia. 6(11):1786-94, 1993; Valera et al., J. Biol. Chem. 269(46):28543-6, 1994; Dosaka-Akita et al., Am. J. Clin. Pathol. 102(5):660-4, 1994; Feng et al., Cancer Res. 55(10):2024-8, 1995; Quattrone et al., Cancer Res. 55(1):90-5, 1995; Lewin et al., Nat Med. 4(8):967-71, 1998). Ribozymes have also been proposed as a means of both inhibiting gene expression of a mutant gene and of correcting the mutant by targeted trans-splicing (Sullenger and Cech Nature 371(6498):619-22, 1994; Jones et al., Nat. Med. 2(6):643-8, 1996). Ribozyme activity may be augmented by the use of, for example, non-specific nucleic acid binding proteins or facilitator oligonucleotides (Herschlag et al., Embo J. 13(12):2913-24, 1994; Jankowsky and Schwenzer Nucleic Acids Res. 24(3):423-9,1996). Multitarget ribozymes (connected or shotgun) have been suggested as a means of improving efficiency of ribozymes for gene suppression (Ohkawa et al., Nucleic Acids Symp Ser. (29):121-2, 1993).

Triple helix approaches have also been investigated for sequence-specific gene suppression. Triple helix forming oligonucleotides have been found in some cases to bind in a sequence-specific manner (Postel et al., Proc. Natl. Acad. Sci. U.S.A. 88(18):8227-31, 1991; Duval-Valentin et al., Proc. Natl. Acad. Sci. U.S.A. 89(2):504-8,1992; Hardenbol and Van Dyke Proc. Natl. Acad. Sci. U.S.A. 93(7):2811-6, 1996; Porumb et al., Cancer Res. 56(3):515-22, 1936). Similarly, peptide nucleic acids have been shown to inhibit gene expression (Hanvey et al., Antisense Res. Dev. 1(4):307-17, 1991; Knudsen and Nielson Nucleic Acids Res. 24(3):494-500, 1996; Taylor et al., Arch. Surg. 132(11):1177-83, 1997). Minor-groove binding polyamides can bind in a sequence-specific manner to DNA targets and hence may represent useful small molecules for suppression at the DNA level (Trauger et al., Chem. Biol. 3(5):369-77, 1996). In addition, suppression has been obtained by interference at the protein level using dominant negative mutant peptides and antibodies (Herskowitz Nature 329(6136):219-22, 1987; Rimsky et al., Nature 341(6241):453-6, 1989; Wright et al., Proc. Natl. Acad. Sci. U.S.A. 86(9):3199-203, 1989). In some cases suppression strategies have led to a reduction in RNA levels without a concomitant reduction in proteins, whereas in others, reductions in RNA have been mirrored by reductions in protein.

The diverse array of suppression strategies that can be employed includes the use of DNA and/or RNA aptamers that can be selected to target a protein of interest (e.g, E-cadherin). For example, in the case of age related macular degeneration (AMD), anti-VEGF aptamers have been generated and have been shown to provide clinical benefit in some AMD patients (Ulrich H, et al. Comb. Chem. High Throughput Screen 9: 619-632, 2006).

In some embodiments, the activity of a protein that negatively regulates EMT (e.g., E-cadherin) is inhibited by contacting a cell with one or more binding agents (e.g., blocking antibodies to E-cadherin). The invention, therefore, embraces binding agents which, for example, can be antibodies or fragments of antibodies having the ability to selectively bind to proteins, such as E-cadherin, and induce EMT. Antibodies include polyclonal and monoclonal antibodies, which may be prepared according to conventional methodology. As used herein, E-cadherin blocking antibodies are antibodies that specifically bind to E-cadherin and induce EMT.

Significantly, as is well known in the art, only a small portion of an antibody molecule, the paratope, is involved in the binding of the antibody to its epitope (see, in general, Clark, W.R. (1986) The Experimental Foundations of Modem Immunology Wiley & Sons, Inc., New York; Roitt, I. (1991) Essential Immunology, 7th Ed., Blackwell Scientific Publications, Oxford). The pFc' and Fc regions, for example, are effectors of the complement cascade but are not involved in antigen binding. An antibody from which the pFc' region has been enzymatically cleaved, or which has been produced without the pFc' region, designated an F(ab')2 fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

Within the antigen-binding portion of an antibody, as is well-known in the art, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FRI through FR4) separated respectively by three complementarity determining regions (CDR1 through CDR3). The CDRs, and in particular the CDR3 regions, and more particularly the heavy chain CDR3, are largely responsible for antibody specificity.

It is now well established in the art that the non-CDR regions of a mammalian antibody may be replaced with similar regions of conspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody. See, e.g., U.S. patents 4,816,567, 5,225,539, 5,585,089, 5,693,762 and 5,859,205.

Fully human monoclonal antibodies also can be prepared by immunizing mice transgenic for large portions of human immunoglobulin heavy and light chain loci. Following immunization of these mice (e.g., XenoMouse (Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (HAMA) responses when administered to humans.

Thus, as will be apparent to one of ordinary skill in the art, the present invention also provides for F(ab')2, Fab, Fv and Fd fragments; chimeric antibodies in which the Fc and/or FR and/or CDR1 and/or CDR2 and/or light chain CDR3 regions have been replaced by homologous human or non-human sequences; chimeric F(ab')2 fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present invention also includes so-called single chain antibodies.

In some embodiments, E-cadherin is inhibited by contacting cells with a small molecule or peptide antagonist of E-cadherin. In some embodiments a cyclic peptide containing a cell adhesion recognition (CAR) sequence, HAV (His-Ala-Val), optionally along with flanking sequences on either side of the CAR sequence, is used. See, e.g., U.S. Pat. Pub. No. 20060183884. Peptides that specifically bind to E-cadherin may be identified by one of skill in the art using, e.g., techniques such as phage display.

In some embodiments, EMT is brought about by modulating the activity of a transcription factor (e.g., a transcription factor that modulates E-cadherin activity). The directionality of the modulation (e.g., inhibiting the activity or inducing the activity of the transcription factor) to induce EMT can be determined or confirmed by a skilled artisan using routine experimentation. In cases where it is desirable to inhibit the activity of a transcription factor, RNA interference is useful. For example, a small interfering nucleic acid, as disclosed herein, complementary to mRNA of a transcription factor can be used to inhibit the activity of the transcription factor. In some embodiments, EMT is brought about by inducing the activity of a transcription factor selected from: Snail1, Snail2, Goosecoid, FoxC2, TWIST, E2A, SIP-1/Zeb-2, dEF1/ZEb1, LEF1, Myc, HMGA2, TAZ, Klf8, HIF-1, HOXB7, SIM2s, and Fos. Exemplary methods for inducing the activity of transcription factors are known in the art. In some cases where it is desirable to induce the activity of a transcription factor exogenous expression of the transcription factor is useful. For example, induction of TWIST expression in a cell is known in the art to inhibit the activity of E-cadherin in the cell and induce EMT. In one embodiment, TWIST is induced in a cell by transfecting the cell with an expression vector encoding TWIST, thereby exogenously expressing TWIST in the cell. In some embodiments, a cell has a stably integrated transgene that expresses a transcription factor, such as TWIST, that causes an EMT in the cell.

In some embodiments, EMT is brought about by modulating the activity of a signaling pathway in a cell, wherein the signaling pathway is selected from TGF-β, Wnt, BMP, Notch, HGF-Met, EGF, IGF, PDGF, FGF, P38-mapk, Ras, PI3Kinase-Akt, Src, and NF-kB. In some embodiments, the signaling pathway that induces EMT is modulated by contacting a cell with a growth factor selected from: a TGF-β/BMP superfamily member, a Wnt-family member, an FGF family member, a Notch Ligand, an EGF family member, an IGF family member, PDGF, and HGF. In some embodiments, the signaling pathway that induces EMT is modulated by contacting a cell with TGF-β₁. Exemplary TGF-β/BMP superfamily members include TGF-β1, TGF-β2, TGF-β3, BMP2, BMP3, BMP4, BMP5, BMP6, BMP7, BMP8a, BMP8b, BMP10, BMP15, GDF1, GDF2, GDF3, GDF5, GDF6, GDF7, Myostatin/GDF8, GDF9, GDF10, GDF11, GDF15, Activin A and B/Inhibin A and B, Anti-müllerian hormone, and Nodal. Exemplary FGF family members include FGF1, FGF2, FGF4, FGF8, FGF10. Exemplary Wnt-family members include WNT1, WNT2, WNT2B/13, WNT3, WNT3A, WNT4, WNT5A, WNT5B, WNT6, WNT7A, WNT7B, WNT8A, WNT8B, WNT9A, WNT9B, WNT10A, WNT10B, WNT11, and WNT16. Exemplary EGF family members include Heparin-binding EGF-like growth factor (HB-EGF), transforming growth factor-α (TGF-α), Amphiregulin (AR), Epiregulin (EPR), Epigen, Betacellulin (BTC), neuregulin-1 (NRG1), neuregulin-2 (NRG2), neuregulin-3 (NRG3), and neureguline-4 (NRG4). Exemplary IGF family members include ICF1 and IGF2.

In some embodiments, the EMT is brought about by subjecting a cell to a stress selected from: hypoxia, irradiation, and chronic chemotherapy treatment. Methods for inducing stress in the cell are known in the art. Exemplary methods are disclosed in Docherty, NG, et al., Am J Physiol Renal Physiol 290: F1202-F1212, 2006 and Manotham K, et al., Kidney Int 65: 871-880, 2004, the contents of which are incorporated in their entirety by reference herein.

Exemplary methods for inducing EMT in a cell are disclosed in Weinberg RA, et al., WO2007/005611; Zavadil J et al., Oncogene 24: 5764-5774, 2005; Sato M, J Clin Invest 112: 1486-1494, 2003; Gregory PA, et al., Nat Cell Biol. Mar 30, 2008; Zeng R, et. al., J Am Soc Nephrol. 2008 Feb;19(2):380-7. Epub 2008 Jan 9; Krawetz R, et al., Cell Signal. 2008 Mar;20(3):506-17; Jiang YG, et al., Int J Urol. 2007 Nov 14(11):1034-9; Lo HW, et al., Cancer Res. 2007 Oct 1, 67(19):9066-76; Lester RD, et al., J Cell Biol. 2007 Jul 30 178(3):425-36; Moustakas A, et al., Cancer Sci. 2007 Oct 98(10):1512-20; and Wahab NA, et al., Nephron Exp Nephrol. 2006, 104(4):e129-34, the contents of which are incorporated herein by reference. However, these methods are not meant to be limiting and other appropriate methods will be apparent to one of ordinary skill in the art.

As used herein, a "vector" may be any of a number of nucleic acid molecules into which a desired sequence may be inserted by restriction and ligation for transport between different genetic environments or for expression in a host cell. Vectors are typically composed of DNA although RNA vectors are also available. Vectors include, but are not limited to, plasmids, phagemids and virus genomes or portions thereof.

An expression vector is one into which a desired sequence may be inserted, e.g., by restriction and ligation such that it is operably joined to regulator sequences and may be expressed as an RNA transcript. Vectors may further contain one or more marker sequences suitable for use in the identification of cells that have or have not been transformed or transfected with the vector. Markers include, for example, genes encoding proteins that increase or decrease either resistance or sensitivity to antibiotics or other compounds, genes that encode enzymes whose activities are detectable by standard assays known in the art (e.g., β-galactosidase or alkaline phosphatase), and genes that visibly affect the phenotype of transformed or transfected cells, hosts, colonies or plaques (e.g., green fluorescent protein).

As used herein, a coding sequence and regulatory sequences are said to be "operably" joined when they are covalently linked in such a way as to place the expression or transcription of the coding sequence under the influence or control of the regulatory sequences. If it is desired that the coding sequences be translated into a functional protein, two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region would be operably joined to a coding sequence if the promoter region were capable of effecting transcription of that DNA sequence such that the resulting transcript might be translated into the desired protein or polypeptide. It will be appreciated that a coding sequence need not encode a protein but may instead, for example, encode a functional RNA such as an shRNA.

The precise nature of the regulatory sequences needed for gene expression may vary between species or cell types, but shall in general include, as necessary, 5' non-transcribed and 5' non-translated sequences involved with the initiation of transcription and translation respectively, such as a TATA box, capping sequence, CAAT sequence, and the like. Such 5' non-transcribed regulatory sequences will include a promoter region that includes a promoter sequence for transcriptional control of the operably joined gene. Regulatory sequences may also include enhancer sequences or upstream activator sequences as desired. The vectors of the invention may optionally include 5' leader or signal sequences. The choice and design of an appropriate vector is within the ability and discretion of one of ordinary skill in the art. One of skill in the art will be aware of appropriate regulatory sequences for expression of interfering RNA, e.g., shRNA, miRNA, etc.

In some embodiments, a virus vector for delivering a nucleic acid molecule is selected from the group consisting of adenoviruses, adeno-associated viruses, poxviruses including vaccinia viruses and attenuated poxviruses, Semliki Forest virus, Venezuelan equine encephalitis virus, retroviruses, Sindbis virus, and Ty virus-like particle. Examples of viruses and virus-like particles which have been used to deliver exogenous nucleic acids include: replication-defective adenoviruses (e.g., Xiang et al., Virology 219:220-227, 1996; Eloit et al., J. Virol. 7:5375-5381, 1997; Chengalvala et al., Vaccine 15:335-339, 1997), a modified retrovirus (Townsend et al., J Virol. 71:3365-3374, 1997), a nonreplicating retrovirus (Irwin et al., J. Virol. 68:5036-5044, 1994), a replication defective Semliki Forest virus (Zhao et al., Proc. Natl. Acad. Sci. USA 92:3009-3013, 1995), canarypox virus and highly attenuated vaccinia virus derivative (Paoletti, Proc. Natl. Acad. Sci. USA 93:11349-11353, 1996), nonreplicative vaccinia virus (Moss, Proc. Natl. Acad. Sci. USA 93:11341-11348, 1996), replicative vaccinia virus (Moss, Dev. Biol. Stand. 82:55-63, 1994), Venzuelan equine encephalitis virus (Davis et al., J Virol. 70:3781-3787, 1996), Sindbis virus (Pugachev et al., Virology 212:587-594, 1995), lentiviral vectors (Naldini L, et al., Proc Natl Acad Sci U S A. 1996 Oct 15;93(21):11382-8) and Ty virus-like particle (Allsopp et al., Eur. J. Immunol 26:1951-1959, 1996).

Another virus useful for certain applications is the adeno-associated virus, a double-stranded DNA virus. The adeno-associated virus is capable of infecting a wide range of cell types and species and can be engineered to be replication-deficient. It further has advantages, such as heat and lipid solvent stability, high transduction frequencies in cells of diverse lineages, including hematopoietic cells, and lack of superinfection inhibition thus allowing multiple series of transductions. The adeno-associated virus can integrate into human cellular DNA in a site-specific manner, thereby minimizing the possibility of insertional mutagenesis and variability of inserted gene expression. In addition, wild-type adeno-associated virus infections have been followed in tissue culture for greater than 100 passages in the absence of selective pressure, implying that the adeno-associated virus genomic integration is a relatively stable event. The adeno-associated virus can also function in an extrachromosomal fashion.

In general, other useful viral vectors are based on non-cytopathic eukaryotic viruses in which non-essential genes have been replaced with the gene of interest. Non-cytopathic viruses include certain retroviruses, the life cycle of which involves reverse transcription of genomic viral RNA into DNA with subsequent proviral integration into host cellular DNA. In general, the retroviruses are replication-deficient (i.e., capable of directing synthesis of the desired transcripts, but incapable of manufacturing an infectious particle). Such genetically altered retroviral expression vectors have general utility for the high-efficiency transduction of genes *in vivo.* Standard protocols for producing replication-deficient retroviruses (including the steps of incorporation of exogenous genetic material into a plasmid, transfection of a packaging cell lined with plasmid, production of recombinant retroviruses by the packaging cell line, collection of viral particles from tissue culture media, and infection of the target cells with viral particles) are provided in Kriegler, M., "Gene Transfer and Expression, A Laboratory Manual," W.H. Freeman Co., New York (1990) and Murry, E.J. Ed. "Methods in Molecular Biology," vol. 7, Humana Press, Inc., Clifton, New Jersey (1991).

Various techniques may be employed for introducing nucleic acid molecules of the invention into cells, depending on whether the nucleic acid molecules are introduced *in vitro* or *in vivo* in a host. Such techniques include transfection of nucleic acid molecule-calcium phosphate precipitates, transfection of nucleic acid molecules associated with DEAE, transfection or infection with the foregoing viruses including the nucleic acid molecule of interest, liposome-mediated transfection, and the like. Other examples include: N-TER™ Nanoparticle Transfection System by Sigma-Aldrich, FectoFly™ transfection reagents for insect cells by Polyplus Transfection, Polyethylenimine "Max" by Polysciences, Inc., Unique, Non-Viral Transfection Tool by Cosmo Bio Co., Ltd., Lipofectamine™ LTX Transfection Reagent by Invitrogen, SatisFection™ Transfection Reagent by Stratagene, Lipofectamine™ Transfection Reagent by Invitrogen, FuGENE® HD Transfection Reagent by Roche Applied Science, GMP compliant in vivo-jetPEI™ transfection reagent by Polyplus Transfection, and Insect GeneJuice® Transfection Reagent by Novagen.

Some aspects of the invention provide methods for testing a cell that has undergone EMT to determine if the cell exhibits characteristics of a cancer stem cell. For example, cells that have undergone an EMT are tested using methods disclosed herein to determine the level of expression of cell surface markers associated with cancer stem cells, growth in suspension culture, tumor formation at low cell numbers *in vivo,* and resistance certain standard chemotherapy drugs.

Aspects of the invention provide test cells and control cells, for example test and control cells that are useful for identifying compounds that specifically target cancer stem cells. As described herein, test or control cells can be primary cells, non-immortalized cell lines, immortalized cell lines, transformed immortalized cell lines, benign tumor derived cells or cell lines, malignant tumor derived cells or cell lines, transgenic cell lines, etc. In some embodiments the tumor is a metastatic tumor, in which case the cells may be derived from the primary tumor or a metastasis. In preferred embodiments, test cells are cells that have undergone an epithelial to mesenchymal transition. Control cells can include both positive and negative controls cells. In one embodiment, a positive control cell is a cancer stem cell, optionally which expresses one or more cancer stem cell biomarker(s). In certain embodiments, a cancer stem cell biomarker is selected from E-Cadherin, TWIST, and a CD44⁺ CD24⁻ marker profile. Non limiting cancer stem cell biomarkers include : CD20, CD24, CD34, CD38, CD44, CD45, CD105, CD133, CD166, EpCAM, ESA, SCA1, Pecam, Stro1, FOXC2^{pos}, N-cadherin^{high}, E-cadherin^{low/neg}, alpha-catenin^{low/neg}, gamma-catenin^{low/neg}, vimentin^{pos}, and fibronectin^{pos}. Other exemplary cancer stem cell markers will be apparent to one of ordinary skill in the art. In some embodiments, a positive control cell is a cell that has undergone an EMT, for example a cell that has reduced E-Cadherin expression.

In some embodiments, a negative control cell is a cancer cell that is not a cancer stem cell, optionally which does not exhibit detectable expression of one or more cancer stem cell biomarker(s). More than one set of control cells may be provided, such as cancer cells that are not cancer stem cells and non-cancer cells. Cells (test or control) may be subjected to one or more genetic or chemical perturbations (e.g., siRNA treatment or Compound treatment) and then incubated for a predetermined time. The predetermined time is a time sufficient to produce a desired effect in a control cell (e.g., inhibit the growth and/or survival thereof).

In some embodiments the cells are mammalian cells, e.g., human cells or non-human animal cells, e.g., cells of non-human primate, rodent (e.g., mouse, rat, guinea pig, rabbit), origin, or interspecies hybrids. In certain embodiments the test and control cells are obtained from a biopsy (e.g., tissue biopsy, fine needle biopsy, etc.) or at surgery for a cancerous or noncancerous condition.

In some embodiments, cells (e.g., test cells, controls cells) of the invention may be derived from a cancer (e.g., naturally occurring cancer). In some embodiments, the cancer from which cells are derived is a colon carcinoma, a pancreatic cancer, a breast cancer, an ovarian cancer, a prostate cancer, a squamous cell carcinoma, a cervical cancer, a lung carcinoma, a small cell lung carcinoma, a bladder carcinoma, a squamous cell carcinoma, a basal cell carcinoma, an adenocarcinoma, a sweat gland carcinoma, a sebaceous gland carcinoma, a papillary carcinoma, a papillary adenocarcinoma, a cystadenocarcinoma, a medullary carcinoma, a bronchogenic carcinoma, a renal cell carcinoma, a hepatocellular carcinoma, a bile duct carcinoma, a choriocarcinoma, a seminoma, a embryonal carcinoma, a Wilms' tumor, melanoma, or a testicular tumor. In one embodiment, cancer is a lung carcinoma. In one embodiment, cancer is a breast carcinoma. Other cancers will be known to one of ordinary skill in the art. In some embodiments the cancer is a spontaneously arising cancer. In some embodiments the cancer is a cancer associated with a known or characteristic genetic mutation or polymorphism. In some embodiments the cancer is an experimentally produced cancer. In some embodiments the cancer is a hormone-responsive cancer. In some embodiments the cells are derived from an early stage cancer or precancerous lesion, e.g., a papilloma, adenoma, dysplastic lesion, etc., or a carcinoma in situ. In some embodiments the cancer is one that is responsive to a chemotherapeutic agent or combination thereof (e.g., any one or more of the chemotherapeutic agents discussed below). In some embodiments the cancer is one that is resistant to a chemotherapeutic agent or combination thereof.

In some embodiments, cancer cells are experimentally produced. Cancer cells can be experimentally produced by a number of methods known in the art that result in transformation of a non-cancer cell (non-transformed cell) to a cancer cell (transformed cell). Such experimentally produced cancer cells may be metastatic or non-metastatic.

In some cases, cancer cells are produced from non-cancer cells by transfecting the non-cancer cells (transiently or stably) with one or more expression vector(s) encoding an oncogene. Such oncogenes, when expressed, lead to neoplastic or hyperplastic transformation of a cell. The oncogene may be a complete sequence of the oncogene, preferably an oncogenic form of the oncogene, or it may be a fragment of the oncogene that maintains the oncogenic potential of the oncogene. Exemplary oncogenes include MYC, SRC, FOS, JUN, MYB, RAS, ABL, BCL2, HOXII, HOXI 1L2, TAL1/SCL, LMOI, LMO2, EGFR, MYCN, MDM2, CDK4, GLII, IGF2, activated EGFR, mutated genes, such as FLT3-ITD, mutated of TP53, PAX3, PAX7, BCR/ABL, HER2/NEU, FLT3R, FLT3-ITD,SRC, ABL, TANI, PTC, B-RAF, PML-RAR-alpha, E2A-PBX1, and NPM-ALK, as well as fusion of members of the PAX and FKHR gene families. Other exemplary oncogenes are well known in the art and several such examples are described in, for example, The Genetic Basis of Human Cancer (Vogelstein, B. and Kinzler, K. W. eds. McGraw-Hill, New York, N. Y., 1998). Homologues of such genes can also be used.

In some cases, cancer cells can be produced from non-cancer cells by transfecting the non-cancer cells (transiently or stably) with one or more expression vector(s) encoding an inhibitory molecule (e.g., shRNA, mirRNA) capable of inhibiting the expression of a tumor suppressor gene. Such inhibitory molecules, when expressed, lead to neoplastic or hyperplastic transformation of a cell. Exemplary tumor suppressor genes include RB, TP53, APC, NF-1, BRCA-1, BRCA-2 and WT-1. Other exemplary tumor suppressor genes are well known in the art.

In some cases, cancer cells can be produced from non-cancer cells by transfecting the non-cancer cells (transiently or stably) with one or more expression vector(s) encoding an inhibitory molecule (e.g., shRNA) capable of inhibiting the expression of a tumor suppressor gene and one or more expression vector(s) encoding an oncogene.

In some embodiments, cells (e.g., test cells, control cells) of the invention are derived from noncancerous tissue. For example, the cells may be derived from any epithelial tissue. One of skill in the art will appreciate that "epithelium" refers to layers of cells that line the cavities and surfaces of structures throughout the body and is also the type of tissue of which many glands are at least in part formed. Such tissues include, for example, tissues found in the breast, gastrointestinal tract (stomach, small intestine, colon), liver, biliary tract, bronchi, lungs, pancreas, kidneys, ovaries, prostate, skin, cervix, uterus, bladder, ureter, testes, exocrine glands, endocrine glands, blood vessels, etc. In some embodiments the epithelium is endothelium or mesothelium. In certain embodiments the cells are human breast epithelial cells. In certain embodiments the cells are noncancerous human breast cells obtained from a reduction mammoplasty. In certain embodiments, the test and control cells are derived from any cell type that normally expresses E-cadherin. In certain embodiments, the test and control cells are of a cell type that does not normally express N-cadherin. In certain embodiments, the test and control cells are of a cell type that normally expresses E-cadherin at levels at least 5, 10, 20, 50, or 100-fold higher levels, on average, than those at which it expresses N-cadherin.

In some embodiments the cells (test and/or control) have been modified, e.g., genetically modified, so as to express, inhibit, or delete one or more oncogenes or tumor suppressor genes. In some embodiments such modification immortalizes the cells. In some embodiments such modification transforms the cells to tumorigenic cells. For example, in certain embodiments test and/or control cells are immortalized by expressing telomerase catalytic subunit (e.g., human telomerase catalytic subunit; hTERT) therein. In certain embodiments test and/or control cells are transformed by expressing SV40 (e.g., early region) or Ras, optionally activated Ras such as H-rasV12, therein. In some embodiments cells are modified or treated so as to have reduced or essentially absent expression and/or functional activity of cell cycle checkpoint or DNA damage sensing proteins, e.g., p16, e.g., p16^{INK4a}, p53 and/or retinoblastoma (Rb) proteins. For example, cells can be modified to express a shRNA targeted to one or more of these genes, or to express a viral protein that binds to one or more of these proteins. Combinations of such modifications can be used. For example, cells may be modified to express SV40 large T (LT), hTERT, and H-*ras*V12*.* Other means of immortalizing and/or transforming cells are known in the art and are within the scope of the invention.

In certain embodiments of the invention the test cells and control cells are derived from an initial population of substantially identical cells that have not undergone an EMT. Certain of these cells are manipulated so as to render them suitable for use as test cells, e.g., by modifying them so as to be able to induce EMT in a controlled manner and then inducing EMT or by treating them with an agent that induces EMT, e.g., as described above. In certain embodiments such as these the test and control cells are genetically matched but have one or several defined genetic differences such as those described herein that result in the test cells having undergone EMT while the control cells have not undergone EMT. In certain embodiments, two populations of cells derived from the same starting population, wherein one population has been modified by introducing a vector and the other population has not been so modified. In certain embodiments, two populations of cells derived from the same starting population, wherein one population has been modified by introducing an expression construct encoding an inhibitory nucleic acid or protein element and the other population has been modified by introducing a expression construct encoding a control nucleic acid or protein element (e.g., one that would not be expected to inhibit an endogenous cellular gene or protein). Typically the expression constructs are otherwise similar or identical. In certain embodiments of the invention, the test cells and control cells are genetically matched and contain an expression construct (optionally integrated into the genome) comprising a sequence encoding a short interfering RNA capable of inducing EMT (such as a shRNA or miRNA targeted to E-cadherin), wherein the sequence is operably linked to a regulatable (e.g., inducible or repressible) promoter. In certain embodiments of the invention, the test cells and control cells are genetically matched and contain an expression construct (optionally integrated into the genome) comprising a sequence encoding a protein capable of inducing EMT, wherein the sequence is linked to a regulatable (e.g., inducible or repressible) promoter. Regulatable expression systems are known in the art and include, e.g., systems utilizing promoters that are inducible by heavy metals, small molecules, etc. Drug-regulatable promoters that are suited for use in mammalian cells include the tetracycline/doxycycline regulatable promoter systems.

"Genetically matched" includes cells or populations of cells that have largely identical genomes, e.g., their genomes are at least 95%, 98%, 99%, 99.5%, 99.9%, 99.99% identical, or more. Typically, genetically matched cells are derived from the same subject or, in the case of certain species such as mice or rats, from different subjects belonging to a particular inbred strain. In some embodiments genetically matched cells are derived from the same tissue sample. In some embodiments of the invention, test and control cells will have been derived from the same initial population of genetically matched cells and will have undergone no more than 2, 5, 10, 15, 20, 25, 30, 35, 40, 50, 60, 70, 80, 90, or 100 rounds of cell division before being used in an inventive method.

The invention provides genetically matched test cells (or cells that can be induced to undergo an EMT and thereby become suitable for use as test cells) and control cells and kits containing such cells. Without wishing to be bound by any theory and without limiting the invention in any way, by using test and control cells that are genetically matched and differ primarily or essentially in that the test cells have undergone an EMT and the control cells have not, the invention allows identification of compounds that differentially affect the test cells versus the control cells (e.g., compounds that inhibit growth of the test cells to a significantly greater extent than the extent to which they inhibit growth of the control cells) as a result of differences in the test cells and control cells that arise as a consequence of the differentiation state of the cells e.g., as a consequence of the test cells having undergone an EMT (associated with acquiring cancer stem cell-like properties) rather than because of other, possibly unknown, genetic or epigenetic differences in the test and control cells-

The practice of the present invention employs, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook et al., 1989) Cold Spring Harbor Press; Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney, ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Methods in Enzymology (Academic Press, Inc.); Handbook of Experimental Immunology (D.M. Weir and C.C. Blackwell, eds.); Gene Transfer Vectors for Mammalian Cells (J.M. Miller and M.P. Calos, eds., 1987); Current Protocols in Molecular Biology (F.M. Ausubel et al., eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: a practical approach (D. Catty., ed., IRL Press, 1988-1989); Monoclonal antibodies : a practical approach (P. Shepherd and C. Dean, eds., Oxford University Press, 2000); Using antibodies: a laboratory manual (E. Harlow and D. Lane (Cold Spring Harbor Laboratory Press, 1999); The Antibodies (M. Zanetti and J.D. Capra, eds., Harwood Academic Publishers, 1995); and Cancer: Principles and Practice of Oncology (V.T. DeVita et al., eds., J.B. Lippincott Company, 7th ed., 2004 or 8^{th} ed., forthcoming in 2008). Further information on cancer may be found in The Biology of Cancer, Weinberg, RA, et al., Garland Science, 2006.

### Screening For CSC Targeting Compounds

In one embodiment, a method for identifying compounds or compositions that inhibit CSC-mediated tumor formation or growth comprises contacting a test cell with a compound or composition and assaying for alterations in the growth and/or survival of the test cell. Typically a test cell is a cell that has undergone an epithelial to mesenchymal transition. The screening may be carried out *in vitro* or *in vivo* using any of the assays disclosed herein, or any assay known to one of ordinary skill in the art to be suitable for contacting a test cell with a compound or composition and assaying for alterations in the growth and/or survival of the test cell.

In one aspect compounds are contacted with test cells (and optionally control cells) at a predetermined dose. In one embodiment the dose may be about up to 1nM. In another embodiment the dose may be between about 1 nM and about 100nM. In another embodiment the dose may be between about 100nM and about 10uM. In another embodiment the dose may be at or above 10uM. Following incubation for an appropriate time, optionally a predetermined time, the effect of compounds or composition on the growth and/or survival of the test cell is determined by an appropriate method known to one of ordinary skill in the art. Cells can be contacted with compounds for various periods of time. In certain embodiments cells are contacted for between 12 hours and 20 days, e.g., for between 1 and 10 days, for between 2 and 5 days, or any intervening range or particular value. Cells can be contacted transiently or continuously. If desired, compound can be removed prior to assessing growth and/or survival. As used herein, "suppress", "inhibit", or "reduce" may, or may not, be complete. For example, cell proliferation, also referred to as growth, may, or may not, be decreased to a state of complete arrest for an effect to be considered one of suppression, inhibition or reduction of cell proliferation. Similarly, gene expression may, or may not, be decreased to a state of complete cessation for an effect to be considered one of suppression, inhibition or reduction of gene expression. Moreover, "suppress", "inhibit", or "reduce" may comprise the maintenance of an existing state and the process of affecting a state change. For example, inhibition of cell proliferation may refer to the prevention of proliferation of a non-proliferating cell (maintenance of a non-proliferating state) and the process of inhibiting the proliferation of a proliferating cell (process of affecting a proliferation state change). Similarly, inhibition of cell survival may refer to killing of a cell, or cells, such as by necrosis or apoptosis, and the process of rendering a cell susceptible to death, such as by inhibiting the expression or activity of an anti-apoptotic regulatory factor. The suppression, inhibition, or reduction may be at least about 5%, 10%, 15%, 20%, 25%, 30%, 35%, 40%, 45%, 50%, 55%, 60%, 65%, 70%, 75%, 80%, 85%, 90%, 95%, 99% of a reference level (e.g., a control level).

In some cases the level of modulation (e.g., suppression, inhibition, or reduction) compared with a control level is statistically significant. As used herein, "statistically significant" refers to a p-value of less than 0.05, e.g., a p-value of less than 0.025 or a p-value of less than 0.01, using an appropriate statistical test (e.g, ANOVA, t-test, etc.).

In certain embodiments, the growth and/or survival of the test cell and/or control cell is determined by an assay selected from: a cell counting assay, a replication labeling assay, a cell membrane integrity assay, a cellular ATP-based viability assay, a mitochondrial reductase activity assay, a caspase activity assay, an Annexin V staining assay, a DNA content assay, a DNA degradation assay, and a nuclear fragmentation assay. Other exemplary assays include BrdU, EdU, or H3-Thymidine incorporation assays; DNA content assays using a nucleic acid dye, such as Hoechst Dye, DAPI, Actinomycin D, 7-aminoactinomycin D or Propidium Iodide; Cellular metabolism assays such as AlamarBlue, MTT, XTT, and CellTitre Glo; Nuclear Fragmentation Assays; Cytoplasmic Histone Associated DNA Fragmentation Assay; PARP Cleavage Assay; TUNEL staining; and Annexin staining.

In one embodiment, gene expression analysis (e.g., microarray, cDNA array, quantitative RT-PCR, RNAse protection assay) is employed to examine the expression of genes whose products mediate cell cycle/growth and/or survival. Exemplary cell cycle/growth related genes include those affecting G1 Phase and G1/S Transition: ANAPC2, CCND1 (cyclin D1), CCNE1 (cyclin E1), CDC7, CDC34, CDK4, CDK6, CDKN1B (p27), CDKN1C (p57), CDKN3, CUL1, CUL2, CUL3, CUL4A, CUL5, E2F1, SKP2; S Phase and DNA Replication: ABL1 (C-ABL), MCM2, MCM3, MCM4 (CDC21), MCM5 (CDC46), MCM6 (Mis5), MCM7 (CDC47), PCNA, RPA3, SUMO1, UBE1; G2 Phase and G2/M Transition: ANAPC2, ANAPC4, ANAPC5, ARHI, BCCIP, BIRC5, CCNA1 (cyclin A1), CCNB1 (cyclin B1), CCNG1 (cyclin G1), CCNH, CCNT1, CCNT2, CDC25A, CDC25C, CDC37, CDK5R1, CDK5R2, CDK5RAP1, CDKSRAP3, CDK2, CDK7, CDKN3, CKS1B, CKS2, DDX11, DNM2, GTF2Hl, GTSE1, HERC5, KPNA2, MNAT1, PKMYT1, RGC32, SERTAD1; M Phase: CCNB2 (cyclin B2), CCNF, CDC2 (CDK1), GDC16, CDC20 (p55cdc), CDC25A, CDC25C, MRE11A, RAD50, RAD51; Cell Cycle Checkpoint and Cell Cycle Arrest: ATM, ATR, BRCA1, BRCA2, CCNA2 (cyclic A2), CCNE2 (cyclin E2), CCNG2 (cyclin G2), CDC2 (CDK1), CDC25A, CDC34, CDC45L, CDC6, CDK2, CDKN1A (p21), CDKN1B (p27), CDKN1C (p57), CDKN2A (p16), CDKN2B (p15), CDKN2C (p18), CDKN2D (p19), CDKN3, CHEK1 (CHK1), CHEK2 (cho2 RAD53), CUL1, CUL2, CUL3, CUL4A, CUL5, GADD45A, HUS1, KNTC1, MAD2L1, MAD2L2, NBS1 (NIBRIN), RAD1, RAD17, RAD9A, RB1, RBBP8, TP53 (p53); Regulation of the Cell Cycle: ABL1 (C-ABL), ANAPC2, ANAPC4, ANAPC5, ARHI, ATM, ATR, BCCIP, BCL2, BRCA2, CCNA1 (cyclin A1), CCNA2 (cyclin A2), CCNB1 (cyclin B1), CCNB2 (cyclin B2), CCNC (cyclin C), CCND1 (cyclic D1), CCND2 (cyclin D2), CCND3 (cyclin D3), CCNE1 (cyclin E1), CCNE2 (cyclin E2), CCNF (cyclin F), CCNH (cyclin H), CCNT1, CCNT2, CDC16, CDC2 (CDK1), CDC20 (p55cdc), CDC25A, CDC25C, CDC37, CDC45L, CDC6, CDK2, CDK4, CDK5R1, CDK5R2, CDK6, CDK7, CDK8, CDKN1A (p21), CDKN1B (p27), CKS1B, CUL5, DDX11, E2F1, E2F2, E2F3, E2F4, E2F5, E2F6, GADD45A, KNTC1, MK167 (Ki67), PCNA, PKMYT1, RAD9A, RB1, SKP2, TFDP1 (DP1), TFDP2 (DP2); Negative Regulation of the Cell Cycle: ATM, BAX, BRCA1, CDC7, CDKN2B (p15), CDKN2D (p19), RBL1 (p107 RB), RBL2 (p134 RB2), TP53 (p53). Exemplary Cell Survival/Apoptotic Genes include those of the TNF Ligand Family: LTA (TNF-a), TNF (TNF-a), TNFSFS (CD40 Ligand), TNFSF6 (FasL), TNFSF7 (CD27 Ligand), TNFSF8 (CD30 Ligand), TNFSF9 (4-1BB Ligand), TNFSF10 (TRAIL), TNFSF14 (HVEM-L), TNFSF18; the TNF Receptor Family: LTBR, TNFRSF1A (TNFR1), TNFRSF1B (TNFR2), TNFRSF5 (CD40), TNFRSF6 (Fas), TNFRSF6B, TNFRSF7 (CD27), TNFRSF9 (4-1BB), TNFRSF10A (DR4), TNFRSF10B (DR5), TNFRSF10C (DcR1) TNFRSF10D (DcR2), TNFRSF11B, TNFRSF12A, TNFRSF14 (HVEM), TNFRSF19, TNFRSF21, TNFRSF25; the Bcl-2 Family: BAD, BAG1, BAG3, BAG4, BAK1, BAX, BCL2, BCL2A1 (bfl-1), BCL2L1 (bcl-x), BCL2L2 (bcl-w), BCL2L10, BCL2L11 (bim-like protein), BCL2L12, BCL2L13, BCLAF1, BID, BIK, BNIP1, BNIP2, BNIP3 (nip3), BNIP3L, BOK (Mtd), HRK, MCL1; the Caspase Family: CASP1, CASP2, CASP3, CASP4, CASP5, CASP6, CASP7, CASP8, CASP9, CASP10, CASP14; the IAP Family: BIRC1 (NIAP), BIRC2 (IAP2), BIRC3 (IAP1), BIRC4 (XIAP), BIRC5 (Survivin), BIRC6 (Bruce), BIRC7, BIRC8; the TRAF Family: TRAF1, TRAF2, TRAF3 (CRAF1), TRAF4, TRAF5; the CARD Family: APAF1, BCL10 (HuE10), BIRC2, BIRC3, CARD4 (NOD1), CARD6, CARD8, CARD9, CARD10, CARD11, CARD12, CARD14, CARD15, CASP1, CASP2, CASP4, CASP5, CASP9, CRADD, NOL3 (Nop30), PYCARD, RIPK2 (CARDIAC); the Death Domain Family: CRADD, DAPK1, DAPK2, FADD, RIPK1, TNFRSF10A, TNFRSF10B, TNFRSF11B, TNFRSF1A, TNFRSF21, TNFRSF25, TNFRSF6, TRADD; the CIDE Domain Family: CIDEA, CIDEB, DFFA, DFFB; the p53 and DNA Damage Response: ABL1, AKT1, APAF1, BAD, BAX, BCL2, BCL2L1, BID, CASP3, CASP6, CASP7, CASP9, GADD45A, TP53 (p53), TP53BP2, TP73, TP73L; and AKT1, BAG1, BAG3, BAG4, BCL2, BCL2A1, BCL2L1, BCL2L10, BCL2L2, BFAR, BIRC1, BIRC2, BIRC3, BIRC4, BIRC5, BIRC6, BIRC7, BIRC8, BNIP1, BNIP2, BNIP3, BRAF, CASP2, CFLAR, GDNF, IGF1R, MCL1, TNF (TNF-a), TNFRSF6, TNFRSF6B, TNFRSF7, TNFSF18, TNFSF5. Compounds or compositions that substantially alter the expression of one or more cell cycle, growth, and/or survival genes may be candidates for cancer stem cell treatment and/or can be examined/tested further using any of the methods disclosed herein.

In other embodiments, alterations in the growth and/or survival of the test cell and/or control cell is/are assessed by examining protein levels, for example the level of protein encoded by a foregoing cell cycle/growth and/or survival gene. Protein levels can be assessed by an appropriate method known to one of ordinary skill in the art, such as western analysis. Other methods known to one of ordinary skill in the art could be employed to analyze proteins levels, for example immunohistochemistry, immunocytochemistry, ELISA, radioimmunoassays, proteomics methods, such as mass spectroscopy or antibody arrays.

Still other parameters disclosed herein that are relevant assessing cell growth and/or survival can provide assays for screening for compounds. For example, high-content imaging or Fluorescence-activated cell sorting (FACS) of cells may be used. In one embodiment, the effect of a compound or composition on a test cell and/or control cell can be assessed by evaluating the apoptotic state of the test cell using automated microscopic imaging or FACS (See for example United States Patent Publication 20070172818). In some cases, fluorescence-based TUNEL staining (e.g., using a FITC-dUTP with standard TUNEL methods known in the art) can reveal apoptosis in a test cell and/or control cell. Other methods include immunocytochemistry using an antibody (e.g., cleaved PARP, cleaved Larnin A, etc.) to detect caspase activity. In other embodiments, an image-based cell cycle/growth marker can be used, such as one or more of those exemplified in Young DW, et al., Nat Chem Biol. 2008 Jan;4(1):59-68. These examples of imaging are not intended to be limiting, and other similar methods will be readily apparent to one of ordinary skill in the art.

In other embodiments, the activity of a cell growth and/or survival gene, such as those disclosed herein, can be assayed in a compound screen. In one embodiment, the assay comprises an expression vector that includes a regulatory region of a cell growth and/or survival gene operably linked to a sequence that encodes a reporter gene product (e.g., a luciferase enzyme), wherein expression of the reporter gene is correlated with the activation of the cell growth and/or survival gene. In this embodiment assessment of reporter gene expression (e.g., luciferase activity) provides an indirect method for assessing cell growth and/or survival. This and other similar assays will be well known to one of ordinary skill in the art. The reporter gene product could be, without limitation, a fluorescent or luminescent protein, enzyme, or other protein amenable to convenient detection and, optionally, quantitation. Examples include GFP, RFP, BFP, YFP, CYP, SFP, reef coral fluorescent protein, mFruits such as mCherry, luciferase, aequorin and derivatives of any of the foregoing. Enzyme reporter proteins such as beta-galactosidase, alkaline phosphatase, chloramphenicol acetyltransferase, etc., are also of use. In other embodiments, chromatin immunoprecipitation assays could be used to assess the binding of transcription factors at a regulatory DNA region of cell growth and/or survival gene(s).

The foregoing assay methods of the invention are amenable to high-throughput screening (HTS) implementations. In some embodiments, the screening assays of the invention are high throughput or ultra high throughput (e.g., Fernandes, P. B., Curr Opin Chem Biol. 1998 2:597; Sundberg, S A, Curr Opin Biotechnol. 2000, 11:47). HTS refers to testing of up to, and including, 100,000 compounds per day. Whereas ultra high throughput (uHTS) refers to screening in excess of 100,000 compounds per day. The screening assays of the invention may be carried out in a multi-well format, for example, a 96-well, 384-well format, or 1,536-well format, and are suitable for automation. In the high throughput assays of the invention, it is possible to screen several thousand different compounds or compositions in a single day. In particular, each well of a microtiter plate can be used to run a separate assay against a selected test compound, or, if concentration or incubation time effects are to be observed, a plurality of wells can contain test samples of a single compound. It is possible to assay many plates per day; assay screens for up to about 6,000, 20,000, 50,000, or more than 100,000 different compounds are possible using the assays of the invention. Typically, HTS implementations of the assays disclosed herein involve the use of automation. In some embodiments, an integrated robot system consisting of one or more robots transports assay microplates between multiple assay stations for compound, cell and/or reagent addition, mixing, incubation, and finally readout or detection. In some aspects, an HTS system of the invention may prepare, incubate, and analyze many plates simultaneously, further speeding the data-collection process. High throughput screening implementations are well known in the art. Exemplary methods are also disclosed in High Throughput Screening: Methods and Protocols (Methods in Molecular Biology) by William P. Janzen (2002) and High-Throughput Screening in Drug Discovery (Methods and Principles in Medicinal Chemistry) (2006) by Jörg Hüser, the contents of which are both incorporated herein by reference in their entirety.

As described above, compounds or compositions that substantially affect the growth and/or survival of a test cell and/or control cell, and/or that are potential modulators of cancer stem cell dependent tumor growth can be discovered using the disclosed test methods.

Examples of types of compounds or compositions that may be tested include, but are not limited to: anti-metastatic agents, cytotoxic agents, cytostatic agents, cytokine agents, antiproliferative agents, immunotoxin agents, gene therapy agents, angiostatic agents, cell targeting agents, HDAC inhibitory agents, etc. As used herein, compounds or compositions may in some cases be referred to as test agents.

The following provides further examples of test compounds and is not meant to be limiting. Those of ordinary skill in the art will recognize that there are numerous additional types of suitable test compounds that may be tested using the methods, cells, and/or animal models of the invention. Test compounds can be small molecules (*e.g.*, compounds that are members of a small molecule chemical library). The compounds can be small organic or inorganic molecules of molecular weight below about 3,000 Daltons. The small molecules can be, *e.g.*, from at least about 100 Da to about 3,000 Da (*e.g.,* between about 100 to about 3,000 Da, about 100 to about 2,500 Da, about 100 to about 2,000 Da, about 100 to about 1,750 Da, about 100 to about 1,500 Da, about 100 to about 1,250 Da, about 100 to about 1,000 Da, about 100 to about 750 Da, about 100 to about 500 Da, about 200 to about 1500, about 500 to about 1000, about 300 to about 1000 Da, or about 100 to about 250 Da). Test compounds can also be microorganisms, such as bacteria (*e.g.*, *Escherichia coli*, *Salmonella typhimurium, Mycobacterium avium,* or *Bordetella pertussis*), fungi, and protists (*e.g.*, *Leishmania amazonensis*), which may or may not be genetically modified. See, *e.g.*, U.S. Patents No. 6,190,657 and 6,685,935 and U.S. Patent Applications No. 2005/0036987 and 2005/0026866.

The small molecules can be natural products, synthetic products, or members of a combinatorial chemistry library. A set of diverse molecules can be used to cover a variety of functions such as charge, aromaticity, hydrogen bonding, flexibility, size, length of side chain, hydrophobicity, and rigidity. Combinatorial techniques suitable for synthesizing small molecules are known in the art (*e.g.*, as exemplified by Obrecht and Villalgrodo, Solid-Supported Combinatorial and Parallel Synthesis of Small-Molecular-Weight Compound Libraries, Pergamon-Elsevier Science Limited (1998)), and include those such as the "split and pool" or "parallel" synthesis techniques, solid-phase and solution-phase techniques, and encoding techniques (see, for example, Czarnik, A. W., Curr. Opin. Chem. Biol. (1997) 1:60). In addition, a number of small molecule libraries are publicly or commercially available (*e.g.*, through Sigma-Aldrich, TimTec (Newark, DE), Stanford School of Medicine High-Throughput Bioscience Center (HTBC), and ChemBridge Corporation (San Diego, CA).

Compound libraries screened using the new methods can comprise a variety of types of test compounds. A given library can comprise a set of structurally related or unrelated test compounds. In some embodiments, the test compounds are peptide or peptidomimetic molecules. In some embodiments, test compounds include, but are not limited to, peptide analogs including peptides comprising non-naturally occurring amino acids, phosphorous analogs of amino acids, amino acids having non-peptide linkages, or other small organic molecules. In some embodiments, the test compounds are peptidomimetics (*e.g.*, peptoid oligomers, *e.g.*, peptoid amide or ester analogues, D-peptides, L-peptides, oligourea or oligocarbamate); peptides (*e.g.*, tripeptides, tetrapeptides, pentapeptides, hexapeptides, heptapeptides, octapeptides, nonapeptides, decapeptides, or larger, *e.g.*, 20-mers or more); cyclic peptides; other non-natural peptide-like structures; and inorganic molecules (*e.g*., heterocyclic ring molecules). Test compounds can also be nucleic acids.

The test compounds and libraries thereof can be obtained by systematically altering the structure of a first "hit" compound, also referred to as a lead compound, that has a chemotherapeutic (*e.g.*, anti-CSC) effect, and correlating that structure to a resulting biological activity (*e.g.*, a structure-activity relationship study).

Such libraries can be obtained using any of the numerous approaches in combinatorial library methods known in the art, including: peptoid libraries (libraries of molecules having the functionalities of peptides, but with a novel, non-peptide backbone which are resistant to enzymatic degradation but which nevertheless remain bioactive; see, *e.g.*, Zuckermann, et al., J. Med. Chem., 37:2678-85 (1994)); spatially addressable parallel solid phase or solution phase libraries; synthetic library methods requiring deconvolution; the "one-bead on e-Compound" library method; and synthetic library methods using affinity chromatography selection (Lam, Anticancer Drug Des. 12:145 (1997)). Examples of methods for the synthesis of molecular libraries can be found in the art, for example in: DeWitt et al., Proc. Natl. Acad. Sci. USA, 90:6909 (1993); Erb et al., Proc. Natl. Acad. Sci. USA, 91:11422 (1994); Zuckermann et al., J. Med. Chem., 37:2678 (1994); Cho et al., Science, 261:1303 (1993); Carrell et al., Angew. Chem. Int. Ed. Engl., 33:2059 (1994); Carell et al., Angew. Chem. Int. Ed. Engl., 33:2061 (1994); and in Gallop et al., J. Med. Chem., 37:1233 (1994). Libraries of compounds can be presented in solution (*e.g.*, Houghten (1992) Biotechniques, 13:412-421), or on beads (Lam (1991) Nature, 354:82-84), chips (Fodor (1993) Nature, 364:555-556), bacteria (Ladner, USP 5,223,409), spores (Ladner, U.S. Patent No. 5,223,409), plasmids (Cull et al. (1992) Proc. Natl. Acad. Sci. USA, 89:1865-1869) or on phage (Scott and Smith (1990) Science, 249:386-390; Devlin (1990) Science, 249:404-406; Cwirla et al. (1990) Proc. Natl. Acad. Sci. USA, 87:6378-6382; Felici (1991) J. Mol. Biol., 222:301-310; Ladner, *supra.*)*.*

In some embodiments, the methods of the invention are used to screen "approved drugs". An "approved drug" is any compound (which term includes biological molecules such as proteins and nucleic acids) which has been approved for use in humans by the FDA or a similar government agency in another country, for any purpose.

Applicants reserve the right to exclude any particular compound, compounds, or compound class from the scope of "test compound" and/or from the compositions and methods of the invention. In some embodiments the "test compound" is not a compound found in, or known in the art as an ingredient of, tissue culture medium, e.g., a compound provided for purposes of culturing the cells. In some embodiments the test compound may be one found in, or known in the art as an ingredient of, tissue culture medium, but is used as a test compound at concentrations differing from those at which it is typically used as an ingredient of tissue culture medium. In some embodiments the compound is not a compound known in the art as being useful for treating cancer and/or for reducing side effects associated with chemotherapy.

Certain results of the compound identification and characterization methods disclosed herein may be clinically beneficial, such as if the compound is a suppressor of CSC tumor growth such as those disclosed herein. Still other clinically beneficial results include: (a) inhibition or arrest of primary tumor growth, (b) inhibition of metastatic tumor growth and (c) extension of survival of a test subject. Compounds with clinically beneficial results are potential chemotherapeutics, and may be formulated as such.

Compounds identified as having a chemotherapeutic or anti-CSC effect are referred to herein as lead compounds and can be selected and systematically altered, *e.g.*, using rational design, to optimize binding affinity, avidity, specificity, or other parameters. Such optimization can also be screened for using the methods described herein. Thus, one can screen a first library of small molecules using the methods described herein, identify one or more compounds that are "hits" or "leads" (by virtue of, for example, their ability to inhibit the growth and/or survival of a test cell and/or cancer stem cell and/or their ability to reduce the size and/or number of CSC dependent tumors, *e.g.*, at the original site of implantation and at metastasis sites), and subject those hits to systematic structural alteration to create a second library of compounds (e.g., refined lead compounds) structurally related to the hit. The second library can then be screened using the methods described herein. A refined lead compound can be produced by modifying the lead compound to achieve (i) improved potency, (ii) decreased toxicity (improved therapeutic index); (iii) decreased side effects; (iv) modified onset of therapeutic action and/or duration of effect; and/or (v) modified pharmacokinetic parameters (absorption, distribution, metabolism and/or excretion). The lead compound could be, e.g., purified from natural sources or chemically synthesized. Modifications could be made directly to the lead compound, or refined lead compounds (e.g., derivatives) could be synthesized from suitable starting materials.

In certain embodiments of the invention a compound identified using the inventive methods displays selective activity (e.g., inhibition of proliferation, toxicity) against test cells relative to its activity against control cells. For example, the IC50 of a compound may be about 2, 5, 10, 20, 50, 100, 250, 500, or 1000-fold lower for test cells versus control cells. In some embodiments, the IC50 of a compound may be about 2, 5, 10, 20, 50, 100,250, 500, or 1000-fold lower for cells that have undergone EMT than for genetically matched cells that have not undergone EMT. In some embodiments, the IC50 of a compound may be about 2, 5, 10, 20, 50, 100,250, 500, or 1000-fold lower for CSCs than for non-CSC cancer cells. In some embodiments, the IC50 of a compound may be about 2, 5, 10,20, 50, 100, 250, 500, or 1000-fold lower for CSCs than for normal (non-cancerous) cells that have not undergone EMT.

Without limitation, as described in the Examples, salinomycin was identified using the inventive methods as a compound having selective toxicity towards cancer stem cells. Synthesis of salinomycin and various derivatives and structurally similar compounds has been reported (Allen, PR, et al., J. Chem. Soc. Perkin Trans. 1: 2403-2411(1998)). The invention encompasses use of salinomycin, salinomycin derivatives and structurally similar compounds including but not limited to those described in the afore-mentioned publication, in the compositions and methods of the invention.

Without limitation, as described in the Examples, abamectin was identified using the inventive methods as a compound having selective toxicity towards cancer stem cells.

Abamectin is a mixture of avermectins containing avermectin B1a and lesser amounts of avermectin B1b, e.g., at least 80% avermectin B1a and less than about 20% avermectin B1b. The avermectins are macrocyclic lactones commonly derived from the soil bacterium *Streptomyces avermitilis.* Abamectin is a natural fermentation product of this bacterium. Abamectin is also known as Avermectin B1 and MK-936. Avermectin family members include ivermectin (22,23-dihydroavermectin B₁ₐ + 22,23-dihydroavermectin B_{1b)}, selamectin, doramectin, as well as the afore-mentioned avermectins. See, e.g., U.S. Pat. Pub. No. 20040101936, PCT Pub. WO/1993/018778, WO/1994/029328, WO/1999/005156, WO2008037934, WO2008019784 and references in any of the foregoing, all of which are incorporated herein by reference, for discussion of various avermectin, avermectin derivatives, and processes for producing certain avermectins and derivatives. The invention further encompasses use of avermectins, avermectin derivatives and structurally similar compounds including but not limited to those described in the afore-mentioned publications, in the compositions and methods of the invention.

Without limitation, as described in the Examples, etoposide was identified using the inventive methods as a compound having selective toxicity towards cancer stem cells. See, Various etoposide derivatives and structurally related compounds and methods of preparation thereof are described, e.g., in WO/2003/048166, WO/2003/035661, WO2004000859, and WO2005056549, and references in the foregoing, all of which are incorporated herein by reference. The invention encompasses use of etoposide, etoposide derivatives and structurally similar compounds including but not limited to those described in the afore-mentioned publications, in the compositions and methods of the invention.

Without limitation, as described in the Examples, nigericin was identified using the inventive methods as a compound having selective toxicity towards cancer stem cells. Nigericin is an antibiotic whose structure and properties are similar to those of the antibiotic monensin, both of which act as ionophores and form complexes with mono and divalent cations. Various nigericin derivatives and structurally related compounds and methods of preparation thereof are described, e.g., in EP0356944, EP0346760, and EP0336248, and references in the foregoing, all of which are incorporated herein by reference. The invention encompasses use of nigericin, nigericin derivatives and structurally similar compounds including but not limited to those described in the afore-mentioned publications, in the compositions and methods of the invention.

Additional compounds having selective toxicity towards CSCs are mentioned in the Examples. The invention provides combination therapies comprising administering two or more CSC-specific compounds disclosed herein to a subject. Also provided are compositions comprising two or more CSC-specific compounds disclosed herein. In certain embodiments the two or more compounds are selected from: salinomycin, salinomycin derivatives and structurally similar compounds, avermectins, avermectin derivatives and structurally similar compounds etoposide, etoposide derivatives and structurally similar compounds, and nigericin, nigericin derivatives and structurally similar compounds.

In some cases it is desirable to determine the potency of a compound or composition of the invention using a dose response assay. Methods for determining the potency of compounds or compositions are well known in the art. In some embodiments, potency is characterized as a half maximal effective concentration (EC50) of a compound or composition. As used herein, the term half maximal effective concentration (EC50) refers to the concentration of a compound or composition that induces a response in a biological system (e.g., one or more cells) halfway between the baseline response (e.g., no compound) and the maximal response. EC50 is commonly used in the art as a measure of compound or composition potency (e.g., drug potency). The EC50 of a dose response curve represents the concentration of a compound or composition where 50% of its maximal effect (also referred to as maximal response) is observed. EC50 is related to the half maximal inhibitory concentration (IC50), which is often used as a measure of inhibition by a compound or composition (50% inhibition) in a biochemical assays, for example competitive binding assays and functional agonist/antagonist assays. Methods for determining EC50/IC50 values are well known in the art.

A variety of techniques useful for determining the structures of compounds are known and can be used in the methods described herein (*e.g.*, NMR, mass spectrometry, gas chromatography equipped with electron capture detectors, fluorescence, and absorption spectroscopy).

Assays of chemotherapeutic activity of test compounds may be conducted *in vitro* or *ex vivo* and/or *in vivo* using cells (e.g., test cells that have undergone an epithelial to mesenchymal transition, cancer stem cells identified or generated using any suitable method, cancer cells, cancer cell lines, etc.) and methods of the invention or any suitable system for testing efficacy. For example, a test compound may be administered to a nonhuman subject to which has been administered (*e.g.*, implanted or injected with) a plurality of the test cells described herein, *e.g.*, a number of cancer stem cells sufficient to induce the formation of one or more tumors (*e.g.*, CSC-dependent tumors), a tumor xenograft, etc. The nonhuman subject can be, *e.g.*, a rodent *(e.g.,* a mouse). Optionally the nonhuman subject is immunocompromised, e.g., a Nude, SCID, NOD-SCID, Rag1-/-, and Rag2-/- mouse. In some embodiments the test subject is a cancer-prone animal, e.g., an animal model harboring an activated oncogene and/or lacking a tumor suppressor gene, or an animal that has been exposed to a condition, compound, or stimulus that renders the animal prone to develop cancer. As used herein, a non-human test subject may also be referred to as an animal host.

The test compound can be administered to the subject by any regimen known in the art. For example, the test compound can be administered prior to, concomitant with, and/or following the administration of cancer stem cells of the invention. A test compound can also be administered regularly throughout the course of the method, for example, one, two, three, four, or more times a day, weekly, bi-weekly, or monthly, beginning before or after cells of the invention have been administered. In other embodiments, the test compound is administered continuously to the subject (*e.g.*, intravenously or by release from an implant, pump, sustained release formulation, etc.). The dose of the test compound to be administered can depend on multiple factors, including the type of compound, weight of the subject, frequency of administration, etc. Determination of dosages is routine for one of ordinary skill in the art. Typical dosages are 0.01-200 mg/kg (*e.g.*, 0.1-20 or 1-10 mg/kg).

The size and/or number of tumors (e.g., CSC-dependent tumors) in the subject can be determined following administration of the tumor cells and the test compound. The size and/or number of tumors can be determined non-invasively by any means known in the art. For example, tumor cells that are fluorescently labeled (*e.g.*, by expressing a fluorescent protein such as GFP) can be monitored by various tumor-imaging techniques or instruments, *e.g.*, non-invasive fluorescence methods such as two-photon microscopy. The size of a tumor implanted subcutaneously can be monitored and measured underneath the skin.

To determine whether a compound affects CSC-dependent tumor formation or the growth of cancer stem cells, the size and/or number of tumors in the subject can be compared to a reference standard (*e.g.*, a control value). A reference standard can be a control subject which has been given the same regimen of administration of cancer stem cells and test compound, except that the test compound is omitted or administered in an inactive form.

Alternately, a compound believed to be inert in the system can be administered. A reference standard can also be a control subject which has been administered cancer cells that are not cancer stem cells and test compound, cancer cells that are not cancer stem cells and no test compound, or cancer cells that are not cancer stem cells and an inactive test compound. The reference standard can also be a numerical figure(s) or range of figures representing the size and/or number of CSC-dependent tumors expected in an untreated subject. This numerical figure(s) or range of figures can be determined by observation of a representative sample of untreated subjects. A reference standard may also be the test animal before administration of the compound.

In some cases the activity of a compound (e.g., a lead compound) can be tested by contacting control cells and test cells that are grown in a co-culture. Co-cultures enable evaluation of the selective growth and/or survival properties of two or more populations of cells (e.g., control and test cells) in contact with a compound in a common growth chamber. Typically, each population of cells grown a co-culture will have an identifying characteristic that is detectable and distinct from an identifying characteristic of the other population(s) of cells in the co-culture. In some embodiments, the identifying characteristic comprises a level of expression of GFP protein or other reporter protein such as those mentioned above and/or a cancer stem cell biomarker. However, the invention is not so limited and other identifying characteristics known in the art may be suitable, provided that the identifying characteristic enables measurement (e.g., by FACS or other suitable assay method disclosed herein) of the level growth and/or survival of each of the two or more populations of cells in the co-culture. Compositions, e.g., co-cultures, comprising at least some test cells (e.g., between 1 and 99% test cells) and at least some control cells (e.g., between 1 and 99% control cells), are an aspect of the invention. In some embodiments the percentage of test cells is between 10% and 90%. In other embodiments the percentage of test cells is between 20% and 80%. In some embodiments the percentage of test cells is between 30% and 70%. In some embodiments the percentage of test cells is between 40% and 60%, e.g., about 50%. In some embodiments the composition further comprises a test agent.

In other embodiments, test cells and control cells are maintained in separate vessels (e.g., separate wells of a microwell plate) under substantially identical conditions.

Assay systems comprising test cells, control cells, and one or more test compounds, e.g., 10, 100, 1000, 10,000, or more test compounds, wherein the cells and test agents are arranged in one or more vessels in a manner suitable for assessing effect of the test compound(s) on the cells, are aspects of the invention. Typically the vessels contain a suitable tissue culture medium, and the test compounds are present in the tissue culture medium. One of skill in the art can select a medium appropriate for culturing a particular cell type. In some embodiments, a medium is free or essentially free of serum or tissue extracts while in other embodiments such a component is present. In some embodiments, cells are cultured on a plastic or glass surface. In some embodiments cells are cultured on or in a material comprising collagen, laminin, Matrigel®, or a synthetic material, e.g., a synthetic hydrogel, intended to provide an environment that resembles in at least some respects the extracellular environment found in many tissues. In some embodiments test and/or control cells are cultured with non-cancerous stromal cells. In some embodiments test and/or control cells are cultured with fibroblasts. In some embodiments test and/or control cells are cultured in three-dimensional culture matrix.

### Genetic Screening for Cancer Stem Cell Genes

In some embodiments, the assay methods of the invention are useful to identify novel cancer stem cell genes. As used herein, cancer stem cell genes are genes that affect the growth and/or survival of cancer stem cells. In one embodiment, a cancer stem cell genetic screen combines RNAi mediated gene suppression in test cell(s) and/or control cell(s) with an assay for cell growth and/or survival. For example, test cell(s) that have undergone an epithelial to mesenchymal transition and/or a control cell(s) that have not undergone an epithelial to mesenchymal transition can be contacted with an siRNA (or similar RNAi based gene suppression modality) to a test gene, thereby inhibiting the expression of the test gene in the test cell(s) and/or control cell(s). After inhibiting expression of the test gene, the growth and/or survival of the test and/or control cell(s) can be evaluated, for example by using any of the methods disclosed herein. In one embodiment, a test gene, whose inhibition affects (e.g., inhibits, activates) the growth and/or survival of the test cell(s), but not the control cell(s) is a cancer stem cell gene.

In one embodiment a genome-wide RNAi based genetic screen is used to identify cancer stem cell genes on a genome-wide scale. In some embodiments, test cells for the RNAi based genetic screen are cells that have undergone a epithelial to mesenchymal transition. The foregoing genetic screening methods use libraries comprising RNAi gene suppression modalities (e.g., shRNA, siRNA, esiRNA, etc.) targeting from a single gene to all, or substantially all, known genes in an organism under investigation. In one embodiment, the library utilizes a mir-30-based shRNA (shRNAmir) expression vector in which shRNA sequence is flanked by approximately 125 bases 5' and 3' of the pre-miR-30 sequence (Chang K, Elledge SJ, Hannon GJ. Nat. Methods. 2006 Sep;3(9):707-14.). Expression vectors can employ either polymerase I, polymerase II or polymerase III promoters to drive expression of these shRNAs and result in functional siRNAs in cells. The former polymerase permits the use of classic protein expression strategies, including inducible and tissue-specific expression systems. Other library compilations, such as Lentiviral-based systems, esiRNA libraries, siRNA and libraries directed against human sequences, are readily available and well known to one of ordinary skill in the art. Genetic screening for cancer stem cell genes is not limited to RNAi methods and may comprise cDNA-based exogenous gene expression or use of genetic suppressor elements (GSEs). In one embodiment, a genome-wide cDNA based genetic screen is used. Thus, the screening methods are applicable to the use of libraries comprising cDNA or GSE-based modalities consisting of from a single gene to all, or substantially all, known genes in an organism under investigation.

### Target Identification

The invention encompasses identifying targets of compounds that are identified using the inventive methods, e.g., the compounds disclosed in the Examples. The term "target" is used herein consistent with its meaning in the art to refer to a biomolecule (e.g., a biomolecule present in the body of a subject) on which a biologically active agent exerts an effect, e.g., an effect leading to a therapeutic benefit such as inhibiting growth and/or proliferation of CSCs. For example, a compound may act directly (by physical interaction) or indirectly on one or more cellular gene products. Targets may be, e.g., cell surface or intracellular receptors, enzymes, channels, secreted proteins, etc. Targets of compounds that are identified as CSC-selective compounds using the methods of the invention are candidates for drug discovery efforts (such candidates are sometimes referred to as "drug discovery targets"). The invention thus provides methods for identifying candidate biomolecules towards which drug discovery efforts may be usefully directed. Compounds that are already known in the art to act on such biomolecules may be useful as CSC-selective agents. Such agents may be tested using the methods described herein. Furthermore, standard screening methods known in the art can be used to identify additional compounds such as small molecules, proteins, aptamers, antibodies or antibody fragments, nucleic acids (e.g., short interfering RNAs), etc., that act on such biomolecules.

Once a compound is identified as a CSC-selective compound using the methods of the invention, a variety of approaches can be used to identify one or more biological target(s) of the compound. In one embodiment, an RNAi or genetic suppressor element (GSE) library is used to inhibit individual genes in cells, e.g., CSCs or cells that have undergone an EMT and are susceptible to growth inhibition by the compound. In one embodiment, a cDNA library is used to overexpress each gene in cells, e.g., CSCs or cells that have undergone an EMT and are susceptible to growth inhibition by the compound. If inhibition or overexpression of a particular gene modulates (e.g., abrogates, reduces, or increases, in various embodiments of the invention) sensitivity, the gene or product of such gene is a candidate for being a target of the compound and/or a target for drug discovery. One of skill in the art will select an appropriate assay or combination of assay.

In another embodiment, the compound is used to identify its molecular target. Any suitable method can be used for target identification. The compound may be labeled or modified to include a reactive group or tag. The reactive group or tag may be incorporated, e.g., at a site where such incorporation does not substantially affect activity of the compound. Cells are contacted with the compound and maintained under conditions wherein the compound interacts with its target(s). The cells may, but need not be, test cells, e.g., cells that have undergone EMT. The cells may, but need not be, of the same type as those cells used to initially identify the compound. Optionally the compound is crosslinked to the biomolecules with which it physically interacts. The compound is then isolated (e.g., removed from some or all other cellular constituents; partially purified) together with the biomolecule(s) to which it is bound. If the compound is tagged, the tag may be used to isolate the compound.

In another embodiment, affinity chromatography is used to identify the target(s) of a compound. The compound is attached to a support, e.g., chromatography resin or other support, thereby forming an affinity matrix. For example, a linker, e.g., tetraethylene glycol linker, may be attached at a suitable position of the compound and then coupled to Affi-Gel 10 resin under appropriate conditions to afford an affinity matrix. Affinity matrices are incubated with cell lysates (e.g., test cell lysate, control cell lysate) and then washed, and bound biomolecules, e.g., proteins, eluted from resin, e.g., by boiling in SDS-containing buffer. The proteins retained by the affinity matrices may be separated, e.g., by SDS/PAGE and visualized, e.g., by silver staining or any appropriate method. They may then be isolated from the gel. The biomolecules are then identified, e.g., using peptide sequencing, mass spectrometry, etc. If desired, an inactive but structurally similar compound may be used as a negative control. Competition with soluble compound may be carried out by adding the compound to cell lysates during binding to the affinity matrix to confirm specificity. In some embodiments, biomolecules that are present selectively in test cells and/or that selectively bind to the compound in test cells versus control cells are of interest.

In another embodiment, microarray analysis, serial analysis of gene expression (SAGE), or similar techniques is/are used to assess the effect of a compound identified using the inventive methods on gene expression in cells of a selected type, e.g., test cells, control cells, cancer cells, CSCs, etc. Genes whose expression is modulated as a result of exposure to the compound are candidates for being targets of the compound and/or targets for drug discovery. In other embodiments of the invention, methods known in the art are used to determine the effect of a compound on protein levels, localization, and/or modification state (e.g., phosphorylation state), etc. Proteins whose levels, localization, and/or modification state are modulated as a result of exposure to the compound are candidates for being targets of the compound and/or targets for drug discovery.

Thus compounds identified using the inventive methods are useful as probes to identify genes and gene products that play significant roles in CSC biology, e.g., genes and gene products whose activity contributes to CSC properties and/or distinguishes CSCs from non-CSC cancer cells, normal cells, etc. Such genes and gene products are useful, without limitation, as targets for drug discovery for purposes of identifying additional agents that selectively inhibit growth and/or proliferation of CSCs.

### Cancer Stem Cell Biomarkers and Treatment of a Subject

The methods described herein have broad application to treating disorders, such as cancer, that are associated with cancer stem cells. Cancer is a disease characterized by uncontrolled or aberrantly controlled cell proliferation and other malignant cellular properties. As used herein, the term cancer includes, but is not limited to, the following types of cancer: breast cancer; biliary tract cancer; bladder cancer; brain cancer including glioblastomas and medulloblastomas; cervical cancer; choriocarcinoma; colon cancer; endometrial cancer; esophageal cancer; gastric cancer; hematological neoplasms including acute lymphocytic and myelogenous leukemia; T-cell acute lymphoblastic leukemia/lymphoma; hairy cell leukemia; chronic myelogenous leukemia, multiple myeloma; AIDS-associated leukemias and adult T-cell leukemia/tyrnphoma; intraepithelial neoplasms including Bowen's disease and Paget's disease; liver cancer; lung cancer; lymphomas including Hodgkin's disease and lymphocytic lymphomas; neuroblastomas; oral cancer including squamous cell carcinoma; ovarian cancer including those arising from epithelial cells, stromal cells, germ cells and mesenchymal cells; pancreatic cancer; prostate cancer; rectal cancer; sarcomas including leiomyosarcoma, rhabdomyosarcoma, liposarcoma, fibrosarcorna, and osteosarcoma; skin cancer including melanoma, Merkel cell carcinoma, Kaposi's sarcoma, basal cell carcinoma, and squamous cell cancer; testicular cancer including germinal tumors such as sezn.inoma, non-seminoma (teratomas, choriocarcinomas), stromal tumors, and germ cell tumors; thyroid cancer including thyroid adenocarcinoma and medullary carcinoma; and renal cancer including adenocarcinoma and Wilms tumor. In preferred embodiments, cancer is a colon carcinoma, a pancreatic cancer, a breast cancer, an ovarian cancer, a prostate cancer, a squamous cell carcinoma, a cervical cancer, a lung carcinoma, a small cell lung carcinoma, a bladder carcinoma, a squamous cell carcinoma, a basal cell carcinoma, an adenocarcinoma, a sweat gland carcinoma, a sebaceous gland carcinoma, a papillary carcinoma, a papillary adenocarcinoma, a cystadenocarcinoma, a medullary carcinoma, a bronchogenic carcinoma, a renal cell carcinoma, a hepatocellular carcinoma, a bile duct carcinoma, a choriocarcinoma, a seminoma, a embryonal carcinoma, a Wilms' tumor, or a testicular tumor. In one embodiment, cancer is a lung carcinoma. In one embodiment, cancer is a breast carcinoma. Other cancers will be known to one of ordinary skill in the art.

Some aspects of the invention are methods for treating a subject having, or suspected of having, cancer comprising administering to the subject an effective amount of a compound that selectively targets cancer stem cells. Other aspects of the invention are methods for treating a subject having, or suspected of having, cancer comprising administering to the subject an effective amount of a cancer chemotherapeutic (e.g., doxorubicin, paclitaxel, actinomycin D, camptothecin, and staurosporine) in combination with a compound that selectively targets cancer stem cells. As used herein, cancer chemotherapeutics embrace CSC selective compounds as well as compounds that are not CSC selective. Compounds that are not CSC selective include compounds that are known to target both CSCs and cancer cells that are not CSCs, and compounds that only target cancer cells that are not CSCs. In some embodiments, a subject is treated with paclitaxel in combination with an effective amount of a pharmaceutical composition comprising a selective cancer stem cell specific compound selected from: etoposide, salinomycin, abamectin, and nigericin and derivatives of any of the foregoing.

Non-limiting examples of cancer chemotherapeutics that are useful with the methods disclosed herein include Alkylating and alkylating-like agents such as Nitrogen mustards (e.g., Chlorambucil, Chlormethine, Cyclophosphamide, Ifosfamide, and Melphalan), Nitrosoureas (e.g., Carmustine, Fotemustine, Lomustine, and Streptozocin), Platinum agents (i.e., alkylating-like agents) (e.g., Carboplatin, Cisplatin, Oxaliplatin, BBR3464, and Satraplatin), Busulfan, Dacarbazine, Procarbazine, Temozolomide, ThioTEPA, Treosulfan, and Uramustine; Antimetabolites such as Folic acids (e.g., Aminopterin, Methotrexate, Pemetrexed, and Raltitrexed); Purines such as Cladribine, Clofarabine, Fludarabine, Mercaptopurine, Pentostatin, and Thioguanine; Pyrimidines such as Capecitabine, Cytarabine, Fluorouracil, Floxuridine, and Gemcitabine; Spindle poisons/mitotic inhibitors such as Taxanes (e.g., Docetaxel, Paclitaxel) and Vincas (e.g., Vinblastine, Vincristine, Vindesine, and Vinorelbine); Cytotoxic/antitumor antibiotics such anthracyclines (e.g., Daunorubicin, Doxorubicin, Epirubicin, Idarubicin, Mitoxantrone, Pixantrone, and Valrubicin), compounds naturally produced by various species of *Streptomyces* (e.g., Actinomycin, Bleomycin, Mitomycin, Plicamycin) and Hydroxyurea; Topoisomerase inhibitors such as Camptotheca (e.g., Camptothecin, Topotecan and Irinotecan) and Podophyllums (e.g., Etoposide, Teniposide); Monoclonal antibodies for cancer immunotherapy such as anti-receptor tyrosine kinases (e.g., Cetuximab, Panitumumab, Trastuzumab), anti-CD20 (e.g., Rituximab and Tositumomab), and others for example Alemtuzumab, Bevacizumab, and Gemtuzumab; Photosensitizers such as Aminolevulinic acid, Methyl aminolevulinate, Porfimer sodium, and Verteporfin, Tyrosine kinase inhibitors such as Cediranib, Dasatinib, Erlotinib, Gefitinib, Imatinib, Lapatinib, Nilotinib, Sorafenib, Sunitinib, and Vandetanib; serine/threonine kinase inhibitors, (e.g., inhibitors of Abl, c-Kit, insulin receptor family member(s), EGF receptor family member(s), Akt, mTOR (e.g., rapamycin or analogs thereof, direct inhibitors of mTORC1 and/or mTORC2), Raf kinase family, phosphatidyl inositol (PI) kinases such as PI3 kinase, PI kinase-like kinase family members, cyclin dependent kinase family members, Aurora kinase family), growth factor receptor antagonists, and others such as retinoids (e.g., Alitretinoin and Tretinoin), Altretamine, Amsacrine, Anagrelide, Arsenic trioxide, Asparaginase (e.g., Pegaspargase), Bexarotene, Bortezomib, Denileukin diftitox, Estramustine, Ixabepilone, Masoprocol, Mitotane, and Testolactone, Hsp90 inhibitors, proteasome inhibitors, HDAC inhibitors, angiogenesis inhibitors, e.g., anti-vascular endotlnelial growth factor agents such as Bevacizumab or VEGF-Trap, matrix metalloproteinase inhibitors, pro-apoptotic agents (e.g., apoptosis inducers), anti-inflammatory agents, etc. in some cases it may be desirable to evaluate a cancer stem cell biomarker in a subject having, or suspect of having, cancer, and to select a treatment for the subject based on the results of the biomarker evaluation. For example, if the cancer stem cell biomarker is detected, the subject may be treated with an effective amount of a pharmaceutical composition comprising a cancer stem cell specific compound such as one identified using the methods disclosed herein. In some embodiments, if the cancer stem cell biomarker is detected, the subject may be treated with an effective amount of a pharmaceutical composition comprising salinomycin, abamectin, etoposide or nigericin, or a derivative of any of the foregoing, optionally in combination with paclitaxel or a derivative thereof (e.g., water-soluble or targeted derivatives or structurally related compounds, e.g., analogs such as docetaxel (see, e.g., WO/2003/045932 and US2008033189). The cancer stem cell biomarker of the foregoing methods may be evaluated using methods disclosed herein or any suitable methods known in the art. Exemplary cancer stem cell biomarkers include E-Cadherin Expression, TWIST expression, and a CD44⁺ CD24⁻marker profile. Other exemplary cancer stem cell biomarkers are disclosed herein and will be apparent to one of ordinary skill in the art.

In order to evaluate the cancer stem cell biomarker it may be necessary to obtain a clinical sample from the subject (e.g., a sample of the cancer). Typically, a clinical sample is a tumor biopsy or cells isolated therefrom. However, the invention is not so limited and any suitable clinical sample may be used, provided that the sample has a detectable cancer stem cell biomarker in a subject having a cancer stem cell. Exemplary clinical samples include saliva, gingival secretions, cerebrospinal fluid, gastrointestinal fluid, mucus, urogenital secretions, synovial fluid, blood, serum, plasma, urine, cystic fluid, lymph fluid, ascites, pleural effusion, interstitial fluid, intracellular fluid, ocular fluids, seminal fluid, mammary secretions, vitreal fluid, and nasal secretions.

In some embodiments, the treatment methods of the invention involve treatment of a subject having (e.g., harboring) or at risk of having a cancer stem cell (CSC) and/or a CSC-dependent tumor. As used herein, a subject is a mammal, including but not limited to a dog, cat, horse, cow, pig, sheep, goat, chicken, rodent, or primate. Subjects can be house pets (e.g., dogs, cats), agricultural stock animals (e.g., cows, horses, pigs, chickens, etc.), laboratory animals (e.g., mice, rats, rabbits, etc.), zoo animals (e.g., lions, giraffes, etc.), but are not so limited. Preferred subjects are human subjects. The human subject may be a pediatric or adult subject. In some embodiments the adult subject is a geriatric subject. Whether a subject is deemed "at risk" of having a CSC or CSC-dependent tumor is a determination that may be within the discretion of the skilled practitioner caring for the subject. Any suitable diagnostic test and/or criteria can be used. For example, a subject may be considered "at risk" of having a CSC or CSC-dependent tumor if (i) the subject has a mutation, genetic polymorphism, gene or protein expression profile, and/or presence of particular substances in the blood, associated with increased risk of developing or having cancer relative to other members of the general population not having mutation or genetic polymorphism; (ii) the subject has one or more risk factors such as having a family history of cancer, having been exposed to a carcinogen or tumor-promoting agent or condition, e.g., asbestos, tobacco smoke, aflatoxin, radiation, chronic infection/inflammation, etc., advanced age; (iii) the subject has one or more symptoms of cancer, etc. In some embodiments, if the compound is one that has been previously (i.e., prior to the instant invention) administered to subjects for purposes other than treating cancer, e.g., for treatment of a condition other than cancer, the subject is not one to whom the compound would normally be administered for such other purpose and/or the compound is administered in a formulation or at a dose distinct from that known in the art to be useful for such other purpose.

Moreover, as used herein treatment or treating includes amelioration, cure, and/or maintenance of a cure (i.e., the prevention or delay of relapse) of a disorder (e.g, a CSC-dependent tumor). Treatment after a disorder has started aims to reduce, ameliorate or altogether eliminate the disorder, and/or its associated symptoms, to prevent it from becoming worse, to slow the rate of progression, or to prevent the disorder from re-occurring once it has been initially eliminated (i.e., to prevent a relapse). A suitable dose and therapeutic regimen may vary depending upon the specific compound used, the mode of delivery of the compound, and whether it is used alone or in combination. As used herein, a therapeutically effective amount is an amount of a compound or composition that inhibits CSC-dependent tumor formation, progression, and/or spread (e.g., metastasis). A therapeutically effective amount can refer to any one or more of the compounds or compositions described herein, or discovered using the methods described herein, that have CSC-dependent tumor inhibitory properties (e.g, inhibit the growth and/or survival of CSCs). Methods for establishing a therapeutically effective amount for any compounds or compositions described herein will be known to one of ordinary skill in the art. As used herein, pharmacological compositions comprise compounds or compositions that have therapeutic utility, and a pharmaceutically acceptable carrier, i.e., that facilitate delivery of compounds or compositions, in a therapeutically effective amount. The effective amount for any particular application can also vary depending on such factors as the cancer being treated, the particular compound being administered, the size of the subject, or the severity of the disease or condition. One of ordinary skill in the art can empirically determine the effective amount of a particular molecule of the invention without necessitating undue experimentation. Combined with the teachings provided herein, by choosing among the various active compounds and weighing factors such as potency, relative bioavailability, patient body weight, severity of adverse side-effects and preferred mode of administration, an effective prophylactic or therapeutic treatment regimen can be planned with the goal of avoiding substantial toxicity and yet effective to treat the particular subject. In some embodiments a useful compound increases the average length of survival, increases the average length of progression-free survival, and/or reduces the rate of recurrence, of subjects treated with the compound in a statistically significant manner.

Subject doses of the compounds described herein typically range from about 0.1 µg to 10,000 mg, more typically from about 1 µg to 8000 mg, e.g., from about 10 µg to 100 mg once or more per day, week, month, or other time interval. Stated in terms of subject body weight, typical dosages in certain embodiments of the invention range from about 0.1 µg to 20 mg/kg/day, e.g., from about 1 to 10 mg/kg/day, e.g., from about 1 to 5 mg/kg/day. The absolute amount will depend upon a variety of factors including the concurrent treatment, the number of doses and the individual patient parameters including age, physical condition, size and weight. These are factors well known to those of ordinary skill in the art and can be addressed with no more than routine experimentation. It is often the case that a maximum dose be used, that is, the highest safe dose according to sound medical judgment.

The dose used may be the maximal tolerated dose or a sub-therapeutic dose or any dose there between. Multiple doses of the molecules of the invention are also contemplated. When the molecules of the invention are administered in combination a sub-therapeutic dosage of either of the molecules, or a sub-therapeutic dosage of both, may be used in the treatment of a subject having, or at risk of developing, cancer. When the two classes of drugs are used together, the cancer medicament may be administered in a sub-therapeutic dose to produce a desirable therapeutic result. A "sub-therapeutic dose" as used herein refers to a dosage which is less than that dosage which would produce a therapeutic result in the subject if administered in the absence of the other agent. Thus, the sub-therapeutic dose of a cancer medicament is one which would not produce the desired therapeutic result in the subject in the absence of the administration of the molecules of the invention. Therapeutic doses of cancer medicaments are well known in the field of medicine for the treatment of cancer. These dosages have been extensively described in references such as Remington's Pharmaceutical Sciences, 18th ed., 1990; as well as many other medical references relied upon by the medical profession as guidance for the treatment of cancer.

The compositions disclosed herein may be administered by any suitable means such as orally, intranasally, subcutaneously, intramuscularly, intravenously, intra-arterially, parenterally, intraperitoneally, intrathecally, intratracheally, ocularly, sublingually, vaginally, rectally, dermally, or as an aerosol. Depending upon the type of condition (e.g., cancer) to be treated, compounds of the invention may, for example, be inhaled, ingested or administered by systemic routes. Thus, a variety of administration modes, or routes, are available. The particular mode selected will depend, of course, upon the particular compound selected, the particular condition being treated and the dosage required for therapeutic efficacy. The methods of this invention, generally speaking, may be practiced using any mode of administration that is medically acceptable, meaning any mode that produces acceptable levels of efficacy without causing clinically unacceptable adverse effects. Preferred modes of administration are parenteral and oral routes. The term "parenteral" includes subcutaneous, intravenous, intramuscular, intraperitoneal, and intrasternal injection, or infusion techniques. In some embodiments, inhaled medications are of particular use because of the direct delivery to the lung, for example in lung cancer patients- Several types of metered dose inhalers are regularly used for administration by inhalation. These types of devices include metered dose inhalers (MDI), breath-actuated MDI, dry powder inhaler

(DPI), spacer/holding chambers in combination with MDI, and nebulizers. Other appropriate routes will be apparent to one of ordinary skill in the art.

According to the methods of the invention, the compounds may be administered in a pharmaceutical composition. Administering the pharmaceutical composition of the present invention may be accomplished by any means known to the skilled artisan. In addition to the active agent, the pharmaceutical compositions of the present invention typically comprise a pharmaceutically-acceptable carrier. The term "pharmaceutically-acceptable carrier", as used herein, means one or more compatible solid or liquid filler diluents or encapsulating substances which are suitable for administration to a human or lower animal. In preferred embodiments, a pharmaceutically-acceptable carrier is a non-toxic material that does not interfere with the effectiveness of the biological activity of the active ingredients. The term "compatible", as used herein, means that the components of the pharmaceutical compositions are capable of being comingled with the compound of the present invention, and with each other, in a manner such that there is no interaction which would substantially reduce the pharmaceutical efficacy of the pharmaceutical composition under ordinary use situations. Pharmaceutically-acceptable carriers must, of course, be of sufficiently high purity and sufficiently low toxicity to render them suitable for administration to the human or lower animal being treated.

Some examples of substances which can serve as pharmaceutically-acceptable carriers are sugars such as lactose, glucose and sucrose; starches such as corn starch and potato starch; cellulose and its derivatives, such as sodium carboxymethylcellulose, ethylcellulose, cellulose acetate; powdered tragacanth; malt; gelatin; talc; stearic acid; magnesium stearate; calcium sulfate; vegetable oils such as peanut oil, cottonseed oil, sesame oil, olive oil, corn oil and oil of theobrama; polyols such as propylene glycol, glycerin, sorbitol, mannitol, and polyethylene glycol; sugar; alginic acid; pyrogen-free water; isotonic saline; phosphate buffer solutions; cocoa butter (suppository base); emulsifiers, such as the Tweens.RTM.; as well as other non-toxic compatible substances used in pharmaceutical formulation. Wetting agents and lubricants such as sodium lauryl sulfate, as well as coloring agents, flavoring agents, excipients, tableting agents, stabilizers, antioxidants, and preservatives, can also be present.

The pharmaceutically-acceptable carrier employed in conjunction with the compounds of the present invention is used at a concentration sufficient to provide a practical size to dosage relationship. The pharmaceutically-acceptable carriers, in total, may comprise from about 60% to about 99.99999% by weight of the pharmaceutical compositions of the present invention, e.g., from about 80% to about 99.99%, e.g., from about 90% to about 99.95%, from about 95% to about 99.9%, or from about 98% to about 99%.

Pharmaceutically-acceptable carriers suitable for the preparation of unit dosage forms for oral administration and topical application are well-known in the art. Their selection will depend on secondary considerations like taste, cost, and/or shelf stability, which are not critical for the purposes of the subject invention, and can be made without difficulty by a person skilled in the art.

Pharmaceutically acceptable compositions can include diluents, fillers, salts, buffers, stabilizers, solubilizers and other materials which are well-known in the art. The choice of pharmaceutically-acceptable carrier to be used in conjunction with the compounds of the present invention is basically determined by the way the compound is to be administered. Exemplary pharmaceutically acceptable carriers for peptides in particular are described in U.S. Patent No. 5,211,657. Such preparations may routinely contain salt, buffering agents, preservatives, compatible carriers, and optionally other therapeutic agents. When used in medicine, the salts should be pharmaceutically acceptable, but non-pharmaceutically acceptable salts may conveniently be used to prepare pharmaceutically-acceptable salts thereof and are not excluded from the scope of the invention. Such pharmacologically and pharmaceutically-acceptable salts include, but are not limited to, those prepared from the following acids: hydrochloric, hydrobromic, sulfuric, nitric, phosphoric, maleic, acetic, salicylic, citric, formic, malonic, succinic, and the like. Also, pharmaceutically-acceptable salts can be prepared as alkaline metal or alkaline earth salts, such as sodium, potassium or calcium salts. It will also be understood that the compound can be provided as a pharmaceutically acceptable pro-drug, or an active metabolite can be used. Furthermore it will be appreciated that agents may be modified, e.g., with targeting moieties, moieties that increase their uptake, biological half-life (e.g., pegylation), etc.

The formulations of the invention are administered in pharmaceutically acceptable solutions, which may routinely contain pharmaceutically acceptable concentrations of salt, buffering agents, preservatives, compatible carriers, adjuvants, and optionally other therapeutic ingredients.

The compounds of the invention may be formulated into preparations in solid, semisolid, liquid or gaseous forms such as tablets, capsules, powders, granules, ointments, solutions, depositories, inhalants and injections, and usual ways for oral, parenteral or surgical administration. The invention also embraces pharmaceutical compositions which are formulated for local administration, such as by implants.

Compositions suitable for oral administration may be presented as discrete units, such as capsules, tablets, lozenges, each containing a predetermined amount of the active agent. Other compositions include suspensions in aqueous liquids or non-aqueous liquids such as a syrup, elixir or an emulsion.

When the compounds described herein are used therapeutically, in certain embodiments a desirable route of administration may be by pulmonary aerosol. Techniques for preparing aerosol delivery systems containing compounds are well known to those of skill in the art. Generally, such systems should utilize components which will not significantly impair the biological properties of the peptides (see, for example, Sciarra and Cutie, "Aerosols," in Remington's Pharmaceutical Sciences, 18th edition, 1990, pp 1694-1712; incorporated by reference). Those of skill in the art can readily determine the various parameters and conditions for producing aerosols without resort to undue experimentation.

The compounds of the invention may be administered directly to a tissue. Preferably, the tissue is one in which the cancer cells are found. Alternatively, the tissue is one in which the cancer is likely to arise. Direct tissue administration may be achieved by direct injection. The peptides may be administered once, or alternatively they may be administered in a plurality of administrations. If administered multiple times, the peptides may be administered via different routes. For example, the first (or the first few) administrations may be made directly into the affected tissue while later administrations may be systemic.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl cellulose, sodium carboxymethylcellulose, and/or polyvinylpyrrolidone (PVP). If desired, disintegrating agents may be added, such as the cross linked polyvinyl pyrrolidone, agar, or alginic acid or a salt thereof such as sodium alginate. Optionally the oral formulations may also be formulated in saline or buffers for neutralizing internal acid conditions or may be administered without any carriers.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations which can be used orally include push fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

For buccal administration, the compositions may take the form of tablets or lozenges formulated in conventional manner. For administration by inhalation, the compounds for use according to the present invention may be conveniently delivered in the form of an aerosol spray presentation from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch. Techniques for preparing aerosol delivery systems are well known to those of skill in the art. Generally, such systems should utilize components which will not significantly impair the biological properties of the active agent (see, for example, Sciarra and Cutie, "Aerosols," in Remington's Pharmaceutical Sciences, 18th edition, 1990, pp 1694-1712; incorporated by reference). Those of skill in the art can readily determine the various parameters and conditions for producing aerosols without resort to undue experimentation.

The compounds, when it is desirable to deliver them systemically, may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like. Lower doses will result from other forms of administration, such as intravenous administration. In the event that a response in a subject is insufficient at the initial doses applied, higher doses (or effectively higher doses by a different, more localized delivery route) may be employed to the extent that patient tolerance permits. Multiple doses per day are contemplated to achieve appropriate systemic levels of compounds.

In yet other embodiments, the preferred vehicle is a biocompatible microparticle or implant that is suitable for implantation into the mammalian recipient. Exemplary bioerodible implants that are useful in accordance with this method are described in PCT International Application No. PCT/US/03307 (Publication No. WO 95/24929, entitled "Polymeric Gene Delivery System", claiming priority to U.S. patent application serial no. 213,668, filed March 15, 1994). PCT/US/0307 describes a biocompatible, preferably biodegradable polymeric matrix for containing a biological macromolecule. The polymeric matrix may be used to achieve sustained release of the agent in a subject. In accordance with one aspect of the instant invention, the agent described herein may be encapsulated or dispersed within the biocompatible, preferably biodegradable polymeric matrix disclosed in PCT/US/03307. The polymeric matrix preferably is in the form of a microparticle such as a microsphere (wherein the agent is dispersed throughout a solid polymeric matrix) or a microcapsule (wherein the agent is stored in the core of a polymeric shell). Other forms of the polymeric matrix for containing the agent include films, coatings, gels, implants, and stents. The size and composition of the polymeric matrix device is selected to result in favorable release kinetics in the tissue into which the matrix device is implanted. The size of the polymeric matrix device further is selected according to the method of delivery which is to be used, typically injection into a tissue or administration of a suspension by aerosol into the nasal and/or pulmonary areas. The polymeric matrix composition can be selected to have both favorable degradation rates and also to be formed of a material which is bioadhesive, to further increase the effectiveness of transfer when the device is administered to a vascular, pulmonary, or other surface. The matrix composition also can be selected not to degrade, but rather, to release by diffusion over an extended period of time.

Both non-biodegradable and biodegradable polymeric matrices can be used to deliver the agents of the invention to the subject Biodegradable matrices are preferred. Such polymers may be natural or synthetic polymers. Synthetic polymers are preferred. The polymer is selected based on the period of time over which release is desired, generally in the order of a few hours to a year or longer. Typically, release over a period ranging from between a few hours and three to twelve months is most desirable. The polymer optionally is in the form of a hydrogel that can absorb up to about 90% of its weight in water and further, optionally is cross-linked with multivalent ions or other polymers.

In general, the agents of the invention may be delivered using the bioerodible implant by way of diffusion, or more preferably, by degradation of the polymeric matrix. Exemplary synthetic polymers which can be used to form the biodegradable delivery system include: polyamides, polycarbonates, polyalkylenes, polyalkylene glycols, polyalkylene oxides, polyalkylene terepthalates, polyvinyl alcohols, polyvinyl ethers, polyvinyl esters, poly-vinyl halides, polyvinylpyrrolidone, polyglycolides, polysiloxanes, polyurethanes and co-polymers thereof, alkyl cellulose, hydroxyalkyl celluloses, cellulose ethers, cellulose esters, nitro celluloses, polymers of acrylic and methacrylic esters, methyl cellulose, ethyl cellulose, hydroxypropyl cellulose, hydroxy-propyl methyl cellulose, hydroxybutyl methyl cellulose, cellulose acetate, cellulose propionate, cellulose acetate butyrate, cellulose acetate phthalate, carboxylethyl cellulose, cellulose triacetate, cellulose sulphate sodium salt, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), poly(octadecyl acrylate), polyethylene, polypropylene, poly(ethylene glycol), poly(ethylene oxide), poly(ethylene terephthalate), poly(vinyl alcohols), polyvinyl acetate, poly vinyl chloride, polystyrene and polyvinylpyrrolidone.

Examples of non-biodegradable polymers include ethylene vinyl acetate, poly(meth)acrylic acid, polyamides, copolymers and mixtures thereof.

Examples of biodegradable polymers include synthetic polymers such as polymers of lactic acid and glycolic acid, polyanhydrides, poly(ortho)esters, polyurethanes, poly(butic acid), poly(valeric acid), and poly(lactide-cocaprolactone), and natural polymers such as alginate and other polysaccharides including dextran and cellulose, collagen, chemical derivatives thereof (substitutions, additions of chemical groups, for example, alkyl, alkylene, hydroxylations, oxidations, and other modifications routinely made by those skilled in the art), albumin and other hydrophilic proteins, zein and other prolamines and hydrophobic proteins, copolymers and mixtures thereof. In general, these materials degrade either by enzymatic hydrolysis or exposure to water in vivo, by surface or bulk erosion.

Bioadhesive polymers of particular interest include bioerodible hydrogels described by H.S. Sawhney, C.P. Pathak and J.A. Hubell in Macromolecules, 1993, 26, 581-587, the teachings of which are incorporated herein, polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

Other delivery systems can include time-release, delayed release or sustained release delivery systems. Such systems can avoid repeated administrations of the peptide, increasing convenience to the subject and the physician. Many types of release delivery systems are available and known to those of ordinary skill in the art. They include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Patent 5,075,109. Delivery systems also include non-polymer systems that are: lipids including sterols such as cholesterol, cholesterol esters and fatty acids or neutral fats such as mono- di- and triglycerides; hydrogel release systems; silastic systems; peptide based systems; wax coatings; compressed tablets using conventional binders and excipients; partially fused implants; and the like. Specific examples include, but are not limited to: (a) erosional systems in which the platelet reducing agent is contained in a form within a matrix such as those described in U.S. Patent Nos. 4,452,775, 4,675,189, and 5,736,152 and (b) diffusional systems in which an active component permeates at a controlled rate from a polymer such as described in U.S. Patent Nos. 3,854,480, 5,133,974 and 5,407,686. In addition, pump-based hardware delivery systems can be used, some of which are adapted for implantation.

Use of a long-term sustained release implant may be particularly suitable for prophylactic treatment of subjects at risk of developing a recurrent cancer. Long-term release, as used herein, means that the implant is constructed and arranged to delivery therapeutic levels of the active ingredient for at least 30 days, and preferably 60 days. Long-term sustained release implants are well-known to those of ordinary skill in the art and include some of the release systems described above.

### EXAMPLE 1:

### Identification of selective inhibitors of cancer stem cells by high-througbput screening

Recent studies have suggested that only a subpopulation of cells within solid tumors, termed cancer stem cells (CSCs), have the ability to seed the formation of new tumors upon transplantation; these CSCs have been proposed to be responsible for metastasis, which is the primary cause of cancer mortality. This concept suggests that many cancer therapies, while killing the bulk of tumors cells, may ultimately fail because they do not eliminate CSCs. Ideally, one would like to identify agents that selectively target CSCs - both to study CSC biology and to use as leads for undertaking drug development. Unfortunately, such screens have not been possible previously due to the rarity of CSCs in tumor cell populations and their relative instability in culture. Here, we report that it is possible to achieve a stable 100-fold increase in the proportion of CSCs by inducing mesenchymal transdifferentiation of cultured epithelial cells. Based on this observation, we designed and conducted a chemical screen to discover agents with selective toxicity for breast CSCs; this resulted in the identification of several such compounds. In functional assays of tumor seeding-ability in vivo (i.e., in vivo tumor seeding assays), one compound (salinomycin) reduced CSCs by 100-fold relative to control treatment and 1000-fold relative to a common breast cancer drug (paclitaxel), which induced an increase in CSCs. This study demonstrates a practical approach to the identification of agents exhibiting specific toxicity for epithelial CSCs.

CSCs have been operationally defined by their ability to seed tumors at limiting dilutions in animal models. In addition, CSCs often have certain important cellular properties: (1) CSCs can be enriched in some cases by sorting cells for specific cell-surface markers (1-5); for example, CSCs in breast cancer are enriched in the CD44+/CD24- sub-fraction of cells. (2) CSCs can form spherical colonies in specialized suspension cultures; these colonies are termed mammospheres in the case of breast cancer. (3) CSCs often exhibit increased resistance to many chemotherapy agents (3-7); this latter finding underscores the importance of developing CSC-specific therapies.

Screening for agents that selectively kill CSCs depends on the ability to obtain and propagate stable, highly purified populations of CSCs in vitro. This is possible for hematopoietic stem cells, which can be enriched to >90% purity by sorting with cell-surface markers (8,9) and can be maintained in culture, but this has not yet been achieved for solid tumors. Sorting with the available markers (CD44+/CD24-) for breast cancer achieves substantial enrichment (100-fold), but CSCs remain a tiny minority (∼10⁻³) of the whole population. Moreover, CSC-enrichment is rapidly lost during cell culture, presumably because they differentiate and give rise to phenotypically diverse progeny cells.

We were therefore interested in developing an in vitro system with properties resembling those of CSCs in breast cancer. Visual observation of cells sorted for CSC-markers suggested that CSC frequency might be correlated to and modulated by differentiation state. Consequently, we set out to examine whether the frequency of CSCs in breast cancer is modulated by differentiation state. Published reports have shown that altered expression of certain genes can cause differentiated epithelial cells to lose characteristic epithelial proteins and acquire proteins expressed in basal and mesenchymal cell lineages. Accordingly, we induced HMLER breast cancer cells to undergo mesenchymal transdifferentiation (MT) through short hairpin RNA interference (shRNA)-mediated inhibition of the E-cadherin gene (shEcad) (Figure 1A).

We found that mesenchymal transdifferentiation caused a substantial increase in the proportion of breast cancer cells carrying the markers associated with CSCs. Specifically, we observed that the percentage of CD44+/CD24- cells was -10-fold higher in HMLERshEcad cells than in control cells (HMLERshCntrl) (∼90% vs. 5%; Figure 1F). A similar increase was observed in cells induced into MT by expression of Twist (data not shown).

These findings suggested that MT breast cancer cells may have a higher proportion of CSCs. We therefore tested the ability of HMLERshEcad cells to form mammospheres when grown in suspension; HMLERshEcad cells showed a ∼100-fold increase in mammosphere formation relative to control cells (15 spheres vs. ∼0.15 spheres per 100 cells; Figure 1E) (10). We also directly assayed the ability of HMLERshEcad cells to seed tumors in vivo in mice. Tumors were reliably generated with 1000 HMLERshEcad cells, which is 100-fold lower than for control cells (Figure 1D). Notably, the ∼100-fold enrichment for CSCs is somewhat higher than the ∼15-20 fold increase in CD44+/CD24- cells. These results show that MT breast cancer cells have significantly greater numbers of CSCs relative to control breast cancer cells.

We also tested whether MT breast cancer cells exhibit increased drug resistance, which is a reported property of CSCs. Indeed, HMLERshEcad cells were significantly more resistant than control cells to two commonly used chemotherapy drugs, paclitaxel and doxorubicin (Figure 2A).

Increased drug resistance could reflect an increase in the proportion of CSCs or could be a more general property of the distinctive state of mesenchymal transdifferentiation. We therefore studied HMLE cells, which are immortalized but non-tumorigenic breast epithelial cells that differ from HMLER cells in that they lack an oncogenic HrasV12 transgene. These cells are incapable of forming tumors and thus by definition lack CSCs. Similar to HMLERshEcad cells, HMLEshEcad cells were found to contain increased numbers of CD44+/CD24- cells relative to HMLEshCntrl controls. Notably, HMLEshEcad cells also exhibited increased resistance to paclitaxel and doxorubicin relative to control cells (Figure 2B). HMLEshEcad cells were also resistant to other established chemotherapy drugs, actinomycin D, camptothecin and the broad kinase inhibitor, staurosporine. Increased drug resistance thus appears to be a consequence of mesenchymal transdifferentiation rather than a unique property of the CSC subset.

To further characterize the observed drug resistance, we paclitaxel-treated co-cultures of GFP-labeled HMLEshEcad cells and unlabeled controls cells (1:20 ratio). Treatment for 4 days with 10nM paclitaxel treatment resulted in a 4-fold increase in the proportion of HMLEshEcad cells compared to DMSO-treated co-cultures (Figure 2C). These results indicate that paclitaxel treatment of heterogeneous epithelial cell populations leads to the selective outgrowth of MT cells.

Having discovered that mesenchymally transdifferentiated HMLEshEcad cells exhibit increased resistance to standard chemotherapy drugs, we designed a proof-of-concept screen to identify agents that selectively target these cells with the hope that they might also target CSCs. We screened compounds for cytotoxicity against both HMLEshEcad and control HMLEshCntrl cells. Cells were seeded in 384-well plates, allowed to grow for one day, treated with compound and assayed for cell viability after three days using a luminescence assay; compounds were screened in duplicate for each cell line (Figure 3A; *See* Example 3). We screened a collection of ∼16,000 compounds, including many with known bioactivity, natural extracts, modulators of histone deacetylases, and several diverse commercial libraries.

About 10% of the compounds inhibited the viability of HMLEshEcad cells, but the vast majority of these compounds (98%) also inhibited the control cells. Only 32 compounds (∼0.2% of total library) exhibited specific toxicity towards the HMLEshEcad (Figure 3B). Among the ~100 chemotherapy drugs contained in the compound library, the proportion of hits was not significantly higher, with only three showing any evidence of selective toxicity.

We pursued 8 of the 32 compounds for further study and evaluated their effects across a range of doses. Upon retesting, four of these eight compounds showed consistent evidence of selective toxicity (Figure 4A). Three compounds (etoposide, salinomycin, abamectin) showed moderate to strong selectivity (IC50 is ∼10-fold lower for HMLEshEcad cells vs HMLEshCntrl cells); one compound showed weaker selectivity (nigericin; ∼7-fold).

The compounds that selectively inhibited MT immortalized breast epithelial cells (HMLEshEcad) also inhibited MT cells arising from expression of Twist (HMLETwist). For all four compounds, the dose-response curves for HMLETwist cells were virtually identical to those observed for HMLEshEcad cells (Figure 4B). We also found that treatment of co-cultures of HMLETwist and control cells (10:1 ratio) with salinomycin or abamectin resulted in a dose-dependent decrease in the proportion of HMLETwist cells (Figure 3C). These results suggest that the action of the compounds is independent of the inducer of mesenchymal transdifferentiation.

Because the compounds were identified as selective inhibitors of MT breast epithelial cells (HMLEshEcad), which are non-tumorigenic and thus lack CSCs, it was not clear whether they would have any selective effect on MT tumorigenic cells (HMLERshEcad) and, in particular, on CSCs.

We found that the compounds showed selective toxicity for MT tumorigenic cells (Figure 4D). Across a range of concentrations, salinomycin was selectively toxic for the HMLERshEcad cells, while abamectin displayed modest but selective toxicity for HMLERshEcad cells.

We then turned to the critical question of whether the compounds had the general ability to inhibit CSCs in breast cancer cell lines. A preliminary experiment with three breast cancer lines (SUM 159, T47D, MDA-MB-231) indicated that salinomycin sensitivity was positively correlated with CSC marker expression (Figure 5). We next compared the effects of salinomycin, control vehicle DMSO and paclitaxel on HMLER cells. Salinomycin treatment decreased the proportion of CD44+/CD24- cells, the CSC-enriched fraction, by 20-fold relative to vehicle-treated controls; in contrast, paclitaxel treatment increased the CSC-fraction by 18-fold. The CD44+/CD24- fraction was thus 360-fold lower following treatment with salinomycin than with paclitaxel (Figure 6A). Proliferation of the bulk population was not inhibited following treatment with salinomycin relative to either vehicle- or paclitaxeltreatment (Figure 6B). Similar results were observed with the SUM 159 breast cancer line (Figure 5).

We examined the ability of breast cancer cells to form mammospheres following treatment with salinomycin. Salinomycin treatment resulted in ∼10-fold decrease in the number of mammospheres relative to controls (Figure 6A). In contrast, neither paclitaxel nor vehicle treatment affected the number of mammospheres, with paclitaxel actually causing a significant increase in mammosphere size.

Finally, we also directly assessed the functional presence of CSCs by assaying in vivo tumor-seeding ability following drug treatment. Cells were treated in vitro for 5 days and then injected subcutaneously into NOD/SCID mice. Serial dilution experiments showed that salinomycin treatment resulted in a ∼100-fold decrease in tumor-seeding ability relative to vehicle-treatment, whereas paclitaxel treatment resulted in ∼10-fold increase relative to controls (data not shown). The proportion of functional CSCs was thus reduced by 1000-fold following salinomycin treatment compared to paclitaxel treatment. Thus, every assay currently used to operationally define CSCs indicates that salinomycin successfully targets the viability of CSCs in breast cancer cell populations. By contrast, treatment with the common breast cancer chemotherapy drug, paclitaxel, resulted in an increase in the numbers of CSCs in breast cancer cell populations.

These results have implications for the treatment of solid tumors. The observed heterogeneity of cancer cells within malignant neoplasms suggests that the cancer cells of a single tumor exist in various states, each of which may exhibit distinct sensitivities to any given therapy. To be optimally effective, combination therapies should ideally incorporate agents that target each of the relevant subtypes with a tumor, and in particular CSCs. We describe an approach that makes it possible to screen for agents with specific toxicity for epithelial CSCs. A critical issue to be resolved is whether targeting cancer stem cells in vivo will be of therapeutic benefit to patients.

### Cell Lines:

HMLE is a human breast epithelial cell line immortalized with SV40 large T and hTERT and HMLER is a human breast epithelial cell line immortalized and transformed with SV40 large T, hTERT, and H-*ras*V12. See, e.g., Elenbaas, et. al, Human breast cancer cells generated by oncogenic transformation of primary mammary epithelial cells, Genes and Development, Vol. 15, No. 1, pp. 50-65, (2001); and Yang, J, Mani, SA, Donaher, JL, Ramaswamy, S, Itzykson, RA, Come, C, Savagner, P, Gitelman, I, Richardson, A & Weinberg, RA. Cell 117, 927-939 (2004).

### Example 1 References

1. Qin Y, Capaldo C, Gumbiner BM, Macara IG. The mammalian Scribble polarity protein regulates epithelial cell adhesion and migration through E-cadherin. J Cell Biol 2005;171:1061-71.
2. Pagliarini RA, Xu T. A genetic screen in Drosophila for metastatic behavior. Science 2003;302:1227-31.
3. Szotek PP, Pieretti-Vanmarcke R, Masiakos PT, et al. Ovarian cancer side population defines cells with stem cell-like characteristics and Mullerian Inhibiting Substance responsiveness. Proc Natl Acad Sci U S A 2006;103:11154-9.
4. Bao S, Wu Q, McLendon RE, et al. Glioma stem cells promote radioresistance by preferential activation of the DNA damage response. Nature 2006;444:756-60.
5. Phillips TM, McBride WH, Pajonk F. The response of CD24(-/low)/CD44+ breast cancer-initiating cells to radiation. J Natl Cancer Inst 2006;98:1777-85.
6. Liu G, Yuan X, Zeng Z, et al. Analysis of gene expression and chemoresistance of CD133+ cancer stem cells in glioblastoma. Mol Cancer 2006;5:67.
7. Dean M, Fojo T, Bates S. Tumour stem cells and drug resistance. Nat Rev Cancer 2005;5:275-84.
8. Wagers AJ, Weissman IL. Plasticity of adult stem cells. Cell 2004; 116:639-48.
9. Moore KA, Ema H, Lemischka IR. In vitro maintenance of highly purified, transplantable hematopoietic stem cells. Blood 1997;89:4337-47.
10. Dontu G, Abdallah WM, Foley JM, et al. In vitro propagation and transcriptional profiling of human mammary stem/progenitor cells. Genes Dev 2003;17:1253-70.

### EXAMPLE 2:

### General purpose:

Recent findings have demonstrated that tumor formation and growth are driven by a minor subpopulation of cancer cells within tumors (Al-Hajj M, Wicha MS, Benito-Hernandez A, Morrison SJ, Clarke MF., Proc Natl Acad Sci U S A 2003;100(7):3983-8) (Li C, Heidt DG, Dalerba P, et al., Cancer Res 2007;67(3):1030-7) (O'Brien CA, Pollett A, Gallinger S, Dick JE., Nature 2007;445(7123):106-10) (Ricci-Vitiani L, Lombardi DG, Pilozzi E, et al., Nature 2007;445(7123):111-5) (Singh SK, Hawkins C, Clarke ID, et al., Nature 2004;432(7015):396-401). Cancer stem cells (CSCs) are defined functionally as those cells within a tumor mass that have the capacity to seed and generate secondary tumors. Implicit in this functional categorization is the notion that cancer stem cells are the cells within tumors responsible for the primary cause of cancer mortality--metastatic dissemination. This concept has significant implications for the development and preclinical assessment of potential cancer therapies. The present invention describes a new method that enables the discovery of novel therapeutics that target cancer stem cells. The discovery of cancer stem cell-targeted therapies was not possible in a high-throughput screening setting prior to the invention of the method described herein. We establish a proof-of-principle for our invention by discovering novel compounds that specifically target cancer stem cells through reducing the described method to practice. Thus, the present invention makes possible for the first time the identification of therapies that target cancer stem cells using high-throughput screening methods.

### Technical Description

Normal and tumor cells exist in various states of differentiation *in vitro* and *in vivo.* These differentiation states are regulated through the integration of complex signals arising from the tissue microenvironment in which a cell resides. Cancer cells can be induced to undergo an epithelial-to-mesenchymal transition (EMT) through any number of genetic perturbations, e.g. via the overexpression of particular factors (such as Twist, Snail, TGF-beta, or MMPs), or by the inhibition of adherens junction proteins such as E-Cadherin. Cells that have been induced into EMT, irrespective of the inducing method used, express similar phenotypic traits and protein markers, indicating that the EMT is a core differentiation program. We have observed that cells that have been induced into EMT share many of the properties that functionally define cancer stem cells, including the expression of cell surface markers associated with cancer stem cells, growth in suspension culture, and tumor formation at low cell numbers *in vivo.* In addition, as is the case for CSCs (Dean M, Fojo T, Bates S., Nat Rev Cancer 2005;5(4):275-84) (Szotek PP, Pieretti-Vanmarcke R, Masiakos PT, et al., Proc Natl Acad Sci U S A 2006; 103(30):11154-9), we have observed that, relative to sibling cell lines not induced into EMT, cells induced into EMT are significantly more resistant to a wide panel of chemical compounds, including standard chemotherapy drugs. Importantly, the resistance to death in response to death-inducing agents is manifest irrespectively of whether the cells in EMT are cancerous or non-cancerous. As a central component of the present invention, we reasoned that the state of differentiation exhibited by cells that have undergone an epithelial-to-mesenchymal transition can be exploited to identify therapies that specifically target cancer stem cells. In the method of this invention, sibling cell lines, derived from a common source, are used to identify compounds that are selectively toxic to cells that have undergone EMT. Below, we describe the detailed technical aspects of one reduction to practice of the present invention. This reduction to practice resulted in the identification of two compounds that selectively target CSC populations, including Salinomycin and Abamectin.

The efficacy of potential anti-cancer compounds is currently evaluated using tumor xenograft assays in immunocompromised mice. Since cancer stem cells frequently constitute a minor subpopulation, it is not to be expected that treatments that are specifically toxic to cancer stem cells would perceptibly impact tumor size measurements in these short-term assays. On the contrary, it is quite possible that assays of this type would fail to identify potential therapies targeting cancer stem cells. Thus, with few exceptions, current therapies are developed to kill rapidly proliferating cancer cells within tumors and do not target CSCs. Importantly, recent reports have indicated that cancer stem cells are more resistant to a wide spectrum of cancer treatments in current use, including chemotherapy, targeted enzyme inhibition, and radiation therapy (Dean M, Fojo T, Bates S., Nat Rev Cancer 2005;5(4):275-84) (Szotek PP, Pieretti-Vanmarcke R, Masiakos PT, et al., Proc Natl Acad Sci U S A 2006;103(30):11154-9) (Bao S, Wu Q, McLendon RE, et al., Nature 2006;444(7120):756-60) (Phillips TM, McBride WH, Pajonk F., J Natl Cancer Inst 2006;98(24):177-85) (Diehn M, Clarke MF., J Natl Cancer Inst 2006;98(24):1755-7). However, at this stage, prior to the instant invention, it is unclear whether it is even possible to identify treatments that target cancer stem cells, or whether it might not be the case that cancer stem cells are inevitably resistant to all forms of therapeutic intervention as a result of an intrinsic resistance to cell death.

These considerations indicate the importance and necessity of developing strategies to assay for the specific effect of candidate treatments on cancer stem cells. Cell-surface markers that allow for the enrichment of cancer stem cell populations have been recently reported for a variety of cancer types (Al-Hajj M, Wicha MS, Benito-Hernandez A, Morrison SJ, Clarke MF., Proc Natl Acad Sci U S A 2003;100(7):3983-8) (Li C, Heidt DG, Dalerba P, et al., Cancer Res 2007;67(3):103 0-7) (O'Brien CA, Pollett A, Gallinger S, Dick JE., Nature 2007;445(7123):106-10) (Ricci-Vitiani L, Lombardi DG, Pilozzi E, et al., Nature 2007;445(7123):111-5) (Singh SK, Hawkins C, Clarke ID, et al., Nature 2004;432(7015):396-401). These markers enable researchers to prospectively isolate cancer stem cell-enriched fractions from either cultured cancer cells or tumors in vivo. In principle, one might envision that the separation of cell populations using such markers would prove useful for the identification of compounds specifically toxic to cancer stem cells. In actuality, the accomplishment of this objective has been fundamentally hampered by the rapidity with which enrichment for cancer stem cells is lost in isolated fractions of cells, often within a few population doublings in culture. These factors have served as a major stumbling block in the identification and development of novel therapies that target CSCs in tumors. Indeed, these difficulties preclude the application of screening technologies that might otherwise, in principle, facilitate the discovery of novel CSC-directed anti-tumor compounds. In the present invention, we describe a method that resolves the difficulties inherent in the application of high-throughput screening technologies for the identification of compounds that target cancer stem cells.

### Screening Alternatives

- Methods of inducing EMT
- Types of libraries to screen (e.g., chemical libraries, RNAi libraries, etc.)
- Cell type (e.g., test cell) could be derived from any type of epithelial tissue. In addition to cells immortalized using SV40ER and hTERT, the invention is equally applicable to primary human cells, cells immortalized using any suitable method of immortalization, or carcinomatous cells. In each case, cells in an EMT and non-EMT state can be screened to identify entities (e.g., compounds) that exhibit selective toxicity for cells in the EMT state.

### E-Cadherin ablation in mammary epithelial cells induces EMT

Epithelial cells manifest distinct states of differentiation in vivo. We wished to determine whether these epigenetically specified states impacted on tumor phenotypes following neoplastic transformation. As a model to study this question, we focused on human mammary epithelial cells, since there are known cellular markers that correspond to their states of differentiation. In order to address our question of interest, it was necessary to utilize a system in which cellular differentiation state could be stably altered.

Extensive previous work has demonstrated that it is possible to stably induce a morphologic shift in cultured epithelial cells that is accompanied by the loss of expression of markers of differentiation (Thiery JP., Nat Rev Cancer 2002;2(6):442-54). We have previously described one such model, in which the inhibition of E-Cadherin in immortalized HMECs results in stable EMT (Onder et al 2008Cancer Research, in press; Figure 1A). Compared to control cells (HMLEsiGFP), these cells (HMLEsiEcad) cease to express cytokeratin proteins associated with epithelial cell differentiation, and gain expression of proteins associated with mesenchymal lineages, including N-cadherin and vimentin (Figure 1A).

These results indicated that E-Cadherin ablation is sufficient to induce EMT in immortalized epithelial cells. Remarkably, the neoplastic conversion of essentially isogenic epithelial cells resulted in markedly distinct tumor phenotypes depending solely on the state of cellular differentiation at the time of transformation. While the neoplastic conversion of epithelial cells that have undergone EMT produces highly malignant cancer cells (HMLERsiEcad) that metastasize rapidly *in vivo*, the analogous transformation of essentially isogenic epithelial cells not having undergone EMT generates cancer cells (HMLERsiGFP) that are incapable of metastasizing (Figure 1 B). Moreover, the HMLERsiEcad tumors exhibited a reduced latency compared to their differentiation HMLERsiGFP counterparts (Figure 1C). Accordingly, we examined whether this distinction was attributable to differences in the numbers of cancer stem cells in the two sibling cell lines.

Limiting dilution assays indicated that the HMLERsiEcad line seeded tumors with as few as 1000 cells *in vivo,* which was two orders of magnitude lower than the minimum number of cells required by the HMLERsiGFP line to form tumors (Figure 1D). Additionally, we observed that the HMLERsiEcad line contained increased numbers of mammosphere-forming cells relative to the HMLERsiGFP line (Figure 1E) (Dontu G, Abdallah WM, Foley JM, et al., Genes Dev 2003;17(10):1253-70). These functional assays, which define cancer stem cells (CSCs), indicated that the HMLERsiEcad line harbored significantly greater numbers of CSCs relative to the HMLERsiGFP cells. This notion was further supported using FACS with antibodies against markers that are reported to enrich for breast cancer stem cell populations (Figure 1F) (Al-Hajj M, Wicha MS, Benito-Hernandez A, Morrison SJ, Clarke MF., Proc Natl Acad Sci U S A 2003;100(7):3983-8). Thus, the HMLERsiEcad line exhibited a ∼10-fold increase in the percentage of CD44+/CD24- cells compared to the differentiated HMLERsiGFP line (Figure 1F). Taken together, these findings indicated that epithelial cells having undergone EMT gave rise to transformed cell populations with significantly higher numbers of cancer stem cells than transformed populations arising from epithelial cells not having undergone EMT.

### Epithelial cells that have undergone EMT are resistant to a variety of chemotherapy drugs

Previous work has indicated that cancer stem cells are resistant to a wide spectrum of cytotoxic agents (Dean M, Fojo T, Bates S., Nat Rev Cancer 2005;5(4):275-84) (Szotek PP, Pieretti-Vanmarcke R, Masiakos PT, et al., Proc Natl Acad Sci U S A 2006;103(30):11154-9) (Bao S, Wu Q, McLendon RE, et al., Nature 2006;444(7120):756-60) (Phillips TM, McBride WH, Pajonk F., J Natl Cancer Inst 2006;98(24):1777-85) (Liu G, Yuan X, Zeng Z, et al., Mol Cancer 2006;5:67). Since the HMLERsiEcad cells have a 100-fold increase in the frequency of cells capable of seeding tumors, we examined their response to two commonly used chemotherapy drugs, relative to HMLERsiGFP controls. We treated cultured HMLERsiGFP and HMLERsiEcad cells for a period of 3 days with either doxorubicin or paclitaxel, at various concentrations. Examination of the dose-response curves indicated that, in the case of both drugs, the IC50 for the HMLERsiEcad cells was significantly higher than the IC50 for the HMLERsiGFP cells (Figure 2A). These data were consistent with previously published reports that cancer stem cells are resistant to common chemotherapy drugs.

In principle, the increased resistance of the HMLERsiEcad line relative to the HMLERsiGFP cell line could be a consequence of the increased numbers of cancer stem cells present in the former cell line. However, we postulated that the differential drug sensitivity observed was, rather, a consequence of the respective differentiation states of the immortalized cells from which the cancer cell lines were derived. To directly examine this hypothesis, we treated HMLEsiGFP and HMLEsiEcad cells for a period of 4 days with a series of chemical compounds at 3 different concentrations each. Included in these compounds were the established chemotherapy drugs Doxorubicin, Paclitaxel, Actinomycin D, Camptothecin as well as a broad kinase inhibitor, Staurosporine. The HMLEsiEcad cells displayed an increased resistance to each of the examined drugs, relative to the HMLEsiGFP cells (Figure 2B). While the extent of resistance naturally depended on the particular drug examined (cp. Actinomycin D vs. Camptothecin; Figure 2B), the increased resistance was observed for all tested compounds. This observation indicated that the HMLEsiEcad cells having undergone EMT are intrinsically resistant to cell death compared to their HMLEsiGFP counterparts, which have not having undergone EMT , irrespective of the death-inducing agent used. Moreover, these findings indicated that the differential sensitivity to cell death exhibited by the HMLERsiEcad line relative to the HMLERsiGFP line was a direct consequence of divergent differentiation states.

The above results indicated that EMT and non-EMT populations of cells exhibited differential sensitivities to a variety of chemical compounds. This was observed independently of whether or not the cell lines had undergone neoplastic transformation. To determine the effect of conventional chemotherapy drugs on a mixture of epithelial cells that have and epithelial cells that have not undergone EMT, we co-mixed GFP-labeled HMLEsiEcad cells with unlabeled HMLEsiGFP cells at a ratio of 1:20, and treated the resulting co-culture with Paclitaxel. This non-stoichiometric mixture provided an internally controlled method to evaluate whether differential drug sensitivity was also manifest under conditions in which epithelial cells having undergone EMT were in the minority. After 3 days of paclitaxel (10nM) treatment, FACS analysis revealed a 4-fold increase in HMLEsiEcad cells having undergone EMT relative to DMSO-treated co-cultures (Figure 2C). A more modest increase in HMLEsiEcad cells having undergone EMT was also observed with a lower dose of paclitaxel (2.5nM). Together, these results indicated that paclitaxel treatment of heterogeneous epithelial cell populations can result in the selective outgrowth of cells having undergone EMT.

### Chemical compound screen

Collectively, the above observations indicated that EMT of epithelial cells is associated with an increase in tumor-seeding potential and metastatic ability, also accompanied by an increased resistance to apoptosis. We therefore reasoned that compounds that inhibit the viability of epithelial cells having undergone EMT but not epithelial cells that have not undergone EMT would preferentially target cancer cell subpopulations capable of seeding tumors and metastasizing. We postulated that the HMLEsiGFP and HMLEsiEcad cell lines could be exploited for the purpose of uncovering compounds exhibiting synthetic lethality towards epithelial cells having undergone EMT.

We developed conditions to use an ATP-based luminescence viability assay with 384-well plates in high throughput screening (see Example 3 for Methods). We screened a total of 16,000 compounds as part of our screen. These compounds included collections with known bioactivity, natural extracts, HDAC-biased collections, and several commercial libraries. To enable rigorous plate-based normalization, each compound plate carried ∼10% control wells with DMSO carrier only. Each compound plate was pin-transferred into 2 replicates for each of the HMLEsiEcad and HMLEsiGFP cell lines. Drug treatment was initiated one day following seeding and the terminal assay was performed 3 days following drug treatment (Figure 3A-schematic). We conducted our screen in duplicate for each of the HMLEsiGFP and HMLEsiEcad cell lines.

Using the measurements from the plate-normalized DMSO control wells, we constructed the distribution of luminescence intensities for each cell line in the absence of compound (Figure 3A). Each measurement for both control and test wells constituted an average of the two replicates for a cell line. Relative to these null distributions, we assigned a Z score for the effect of every compound, one for each of the HMLEsiGFP and HMLEsiECad cell lines. The computed Z-scores did not display a positional bias across drug plate, column, or row number, suggesting that there were no systematic biases in the analysis of the normalized data (Figure 3B).

Identification of inhibitory compounds using this method indicated that the vast majority of such compounds inhibited the viability of both the HMLEsiGFP and HMLEsiEcad cells. Since we were interested in compounds exhibiting lethality synthetic with induced EMT, we identified those compounds that inhibited HMLEsiEcad cell viability but did not surpass the threshold of significance of inhibiting HMLEsiGFP viability. Of the total number of compounds toxic to siEcad cells, ∼2% exhibited specific toxicity towards siEcad line. These compounds are listed in Table 1.

Various established drugs used for cancer chemotherapy were included in the compound plates screened. Less than 1% of these chemotherapeutic agents scored as positive in our assay. This suggests that compounds targeting rapidly proliferating cells were not selectively enriched in our assay. In fact, the HMLEsiGFP cells have a slightly higher rate of proliferation than the HLMEsiEcad cells (data not shown). Therefore, the rare chemotherapeutics that did score positively in our screen (e.g, Etoposide, methotrexate) are likely to be targeting cellular processes distinct from proliferation. With respect to this latter point, it is worth noting that neither etoposide nor methotrexate directly induce DNA damage by intercalation or adduct formation. Thus, there was no enrichment of positive hits in the subset of established chemotherapy drugs relative to the total compounds screened.

We decided to pursue 8 of the compounds identified as being selectively toxic for HMLEsiEcad cells for further study: Salinomycin, Etoposide, Acridine, Cetylpyridinium Chloride, Resorcinol, Clotrimazole, Abamectin, and Nigericin. For each of these compounds, we evaluated the effect on cell viability across a series of doses for sibling cell lines either induced into or not induced into EMT. Three of the aforementioned compounds exhibited strong specificity for viability reduction of the HMLEsiEcad cells relative to the HMLEsiGFP cells (etoposide, salinomycin, abamectin; Figure 4A). One of the identified compounds, nigericin, exhibited modest selective toxicity for the HMLEsiEcad cells relative the HMLEsiGFP cells. The remaining four compounds did not exhibit specific effects on viability on follow-up.

**Table 1: List of 40 hit compounds identified in the screen:**

| | |
|---|---|
| CompoundName | Source |
| avermectin b1 | SpectrumJPN |
| salinomycin, sodium | SpectrumJPN |
| zearalenone | SpectrumJH |
| acrisorcin | SpectrumJPN2 |
| etoposide | SpectrumNAT |
| nigericin sodium | SpectrumJPN |
| larixol acetate | SpectrumNAT |
| avocadyne | SpectrumJH |
| heudelottin c | Spectrumx |
| Clotrimazole;clotrimazole | PrestwickMED |
| Thiostrepton | PrestwickMED |
| aminopterin | SpectrumJH |
| stictic acid | SpectrumNAT |
| melphalan | SpectrumJH |
| orlistat | SpectrumJH |
| triamterene | SpectrumJPN |
| pyrimethamine | SpectrumC |
| diffratic acid | SpectrumNAT |
| hieracin | SpectrumNAT |
| 4-methyldaphnetin | SpectrumNAT |
| NP-000422 | Analyticon |
| NP-005365 | Analyticon |
| 354169;VII_A12 | Tang-W/Harvard |
| WT_BH9 | Tang-W/Harvard |
| phytosphingosine 4-hydroxysphinganine | Avanti |
| PACOCF3 | Calbiochem |
| 354473;V_A12 | Tang-W/Harvard |
| BiomolKI2_000006 | Biomol-KI |
| 326369 | Franz |
| AG 494 | Calbiochem |
| 356135;WT-III-101-II_XG09 | Tang-W/Harvard |
| 354331;VI_B12 | Tang-W/Harvard |
| 354635;II_B12 | Tang-W/Harvard |
| 356110;WT-III-101-II_XD05 | Tang-W/Harvard |
| Bis-Tyrphostin | Calbiochem |
| NP-010664 | Analyticon |
| NP-001642 | Analyticon |
| ascomycin | Sigma |
| 354386;VI_H08 | Tang-W/Harvard |
| 354325;VI_B04 | Tang-W/Harvard |

To determine if the specificity of these compounds for the HMLEsiEcad cells was a consequence of EMT state of the line rather than the particular genetic perturbation used, we also tested the effects of these compounds on cells induced to EMT due to Twist overexpression (HMLE-Twist). Remarkably, the dose response curves for the HMLE-Twist cells were virtually identical to those observed for the HMLEsiEcad cells across each of the tested compounds (Figure 4B). This observation strongly suggested that compounds exhibiting specific activity in this assay were targeting distinct states of differentiation rather than the particular inducing agents themselves.

To examine the effects of salinomycin and abamectin treatment on heterogeneous populations of epithelial cells that have and epithelial cells that have not undergone EMT, we co-mixed GFP-labeled HMLE cells with unlabeled HMLE-Twist cells, treating the resulting co-cultures with compounds. As a positive control for this experiment we treated co-cultures with blasticidin, which selects for the differentiated HMLE-GFP cells, which harbor a blasticidin-resistance gene. We observed that treatment with salinomycin, abamectin or blasticidin resulted in an increase in the GFP-positive fraction relative to DMSO-treated controls, indicating that epithelial cells not having undergone EMT were selectively surviving treatment (Figure 4C). This stood in contrast to our earlier findings demonstrating that paclitaxel treatment resulted in a selection for epithelial cells having undergone (Figure 2C). Thus, the chemical compounds identified through our screen, in contrast to a commonly used chemotherapeutic, resulted in the selective killing of epithelial cells having undergone EMT. In concordance with our previous observations (Figure 2A,B), a subset of these compounds, which were identified by screening for selective toxicity using non-tumorigenic cell lines, also displayed selective toxicity for transformed cell lines having undergone EMT (Figure 4D).

### Salinomycin specifically targets CSC-enriched cancer cell populations

Cancer stem cells are resistant to a wide spectrum of treatments and chemotherapeutics. Since the compounds identified using the described screening approach preferentially target epithelial cells having undergone EMT, we wished to determine whether such compounds might also serve to target cancer stem cells. To this end, we analyzed using FACS several breast cancer cell lines for the expression of markers (CD44hi/CD241o) reported to enrich for cancer stem cells: the MDA-MB-231 and SUM159 cells contained greater than 90% CD44hi/CD241o cells; in contrast, the T47D cell line displayed <1% CD44hi/CD241o cells (Figure 5A). We observed, upon salinomycin treatment, that the CSC-enriched cell lines SUM159 and MDA-MB-231 were more sensitive across a range of doses relative to the CSC-poor T47D cells. Similar results, albeit less pronounced, were also observed with abamectin treatment (Figure 5B).

To further characterize the effects of compound treatment on cancer cells, we conducted FACS analyses on SUM159 cells treated with salinomycin, abamectin, taxol, or DMSO. We observed that, while taxol treatment did not significantly affect the CD44hi/CD241o fraction, treatment with salinomycin significantly reduced the percent of cells in the CD44hi/CD241o fraction relative to DMSO controls, with a concomitant increase (∼3-fold) in the CSC-depleted fraction (Figure 5C). In contrast, abamectin treatment did not significantly alter the percent of cells in the CD44hi/CD241o fraction.

### Functional inhibition of cancer stem cells with Salinomycin treatment

The results above indicated that salinomycin treatment preferentially targets cell lines and cell subpopulations that are reported to enrich for CSCs. To directly evaluate the effect of salinomycin on cancer stem cell populations, we functionally assayed for cancer stem cell activity in salinomycin taxol, or DMSO-treated cultures. Mammosphere formation assays with SUM159 breast cancer cells indicated that, while taxol resulted in an increase in the number of cell capable of forming colonies in suspension culture, salinomycin treatment significantly reduced this ability relative to control DMSO treatment. In vivo tumorformation dilution series assays also indicated that salinomycin treatment significantly reduced CSC functional activity relative to DMSO-treated controls, whereas taxol treatment resulted in the opposite effect. These findings, which utilized the two assays used to functionally defined CSCs, indicated that salinomycin significantly reduced the number of cancer stem cells in treated cancer cell populations.

### Discussion

The present findings suggest that the fraction of cancer stem cells in a population of cancer cells is a correlate of the differentiation state of the target cell prior to transformation. It is this principle that enabled us to exploit untransformed cells that diverge only in their state of differentiation as screening tools to identify agents with cancer stem cell-specific toxicity. Considering that the cell lines we used for screening are isogenic but for a defined perturbation, we reasoned that there was a high likelihood that compounds exhibiting synthetic lethality for the HMECsiEcad cells would also target epithelial cells in a state of mesenchymal differentiation, whether transformed or untransformed.

### Example 2 References

1. (Al-Hajj M, Wicha MS, Benita-Hernandez A, Morrison SJ, Clarke MF., Proc Natl Acad Sci U S A 2003;100(7):3983-8)
2. (Li C, Heidt DG, Dalerba P, et al., Cancer Res 2007;67(3):1030-7)
3. (O'Brien CA, Pollett A, Gallinger S, Dick JE., Nature 2007;445(7123)-106-10)
4. (Ricci-Vitiani L, Lombardi DG, Pilozzi E, et al., Nature 2007;445(7123):111-5)
5. (Singh SK, Hawkins C, Clarke ID, et al., Nature 2004;432(7015):396-401)
6. (Dean M, Fojo T, Bates S., Nat Rev Cancer 2005;5(4):275-84)
7. (Szotek PP, Pieretti-Vanmarcke R, Masiakos PT, et al., Proc Natl Acad Sci U S A 2006;103(30):11154-9)
8. (Bao S, Wu Q, McLendon RE, et al., Nature 2006;444(7120):756-60)
9. (Phillips TM, McBride WH, Pajonk F., J Natl Cancer Inst 2006;98(24):1777-85)
10. (Diehn M, Clarke MF., J Natl Cancer Inst 2006;98(24):1755-7)
11. (Thiery JP., Nat Rev Cancer 2002;2(6):442-54)
12. (Dontu G, Abdallah WM, Foley JM, et al., Genes Dev 2003;17(10):1253-70)
13. (Liu G, Yuan X, Zeng Z, et al., Mol Cancer 2006;5:67)

### EXAMPLE 3: Materials and Methods

### Cell culture.

Immortalized (HMLE) or transformed (HMLER) breast epithelial cells that express either control shRNA (shCntrl) or shRNA targeting E-cadherin (shEcad) were generated and maintained as described (Onder et al. 2008 Cancer Research, in press;). HMLE-Twist cells were also described previously (Yang et al 2004). To create GFP expressing strains, we infected the HMLE and HMLE-shEcad cells with a pWZL-GFP retrovirus carrying the blasticidin resistance gene using standard procedures (Stewart et al 2003).

Mammosphere culture was performed as described (Dontu et al., 2003), except that the culture medium contained 0.9 % methyl cellulose (Stem cell technologies) to prevent cell aggregation. 102 or 103 cells were plated per well in 96-well plates. The mammospheres were cultured for 7-10 days and then photographed and counted.

### Antibodies, Immunoblotting.

Antibodies utilized for immunoblotting were E-cadherin, N-cadherin (BD Transduction), Vimentin V9 (NeoMarkers), Actin (Abcam), H-Ras (Santa Cruz), Cytokeratin 8 (Troma-1, Developmental Studies Hybridoma Bank, University of Iowa). All procedures were carried out as described (Onder et al 2008 Cancer Research, in press;).

### FACS

The anti-CD44 (clone G44-26) antibody conjugated to APC and the anti-CD24 antibody (clone ML5) conjugated to PE used for FACS analysis were obtained from BD Bioscience and used according to the manufacturer's instructions. Propidium Iodide (5ug/ml) was included in the staining protocol to distinguish live cells.

### Characterization of resistance to cytotoxic agents.

Doxorubicin, paclitaxel, actinomycin D, campthotecin and staurosporine were purchased from Sigma and dissolved in dimethyl sulfoxide (dmso). 5000 cells in 100 ul of medium were plated per well in 96-well plates. 24 hrs after seeding, compounds at the indicated concentration were added to wells (5 wells per each concentration). Dmso treatment was used as negative control. Cell viability was measured after 72 hrs using the CellTiter96 AQueous Assay (Promega) according to manufacturer's instructions. Dose response curves were generated with GraphPad Prism software (GraphPad Software, Inc.).

For hypoxia and 2-deoxyglucose treatments cell viability was also measured after 72 hrs using the CellTiter96 AQueous Assay (MTT).

For the mix experiments, unlabelled and GFP-labeled cells were mixed at the indicated ratios and seeded onto 6-well plates. Triplicate wells were then treated with dmso or the compounds (paclitaxel, salinomycin, abamectin and blasticidin) for 48 hrs. The cells were washed with PBS, trypsinized and analyzed by FACS for GFP positivity.

### Chemical Screen

Information about the general automated HTS protocol is available online at broad.harvard.edu/chembio/index.html. The assay was initiated by plating 40 µL of medium containing 1000 cells/well into white 384-well opaque-bottom plates (Nunc, Rochester, NY) using an automated plate filler (Bio-Tek µFiller; Winsooki, VT) and allowing the cells to adhere for 24 hours. 100 nL of compound stock solutions in dmso was transferred from stock plates into the 384-well assay plates using an automated pin-based compound transfer robot (CyBio CyBi-Well vario; Woburn, MA). For most compounds the final concentration in each well was calculated to be 10uM.

The screen was performed in duplicate. For negative controls, entire dmso-treated control plates were employed, in addition to dmso-only control wells that were incorporated into each compound assay plate. The cells were assayed for luciferase activity with the addition 20 ul of CellTiter-Glo Luminescent Cell Viability Assay solution (Promega). The luminescent signal from each plate was detected using an automated plate reader (Perkin-Elmer Envision 1; Wellesley, MA).

The compound plate numbers for screened plates were 2158-2167, 2099-2105, 2290-2297, 2403-2407, Biokinl-2. The primary data were analyzed using the commercial software package SpotFire (SpotFire, Inc., Somerville, MA).

### Validation of hits

Individual compounds were purchased from Sigma and dissolved in DMSO with the exception of nigericin which was dissolved in 100% ethanol. Activity of the compounds were quantified by generating dose-response curves for HMLE-shCntrl, HMLE-shEcad and HMLE-Twist under the same cell density and culture conditions described for the initial screen, using the 384-well plate based system.

### Tumorigenesis Assays

106, 105 104, and 103 of HMLER-shCntrl or HMLER-shEcad cells in 100 µl of Matrigel diluted 1:2 in DME were injected subcutaneously into NOD-SCID mice. The tumor incidence was monitored for 60 days following injection. All mouse procedures were preapproved by the Animal Care and Use Committee of the Massachusetts Institute of Technology and performed according to institutional policies.

### EXAMPLE 4: Exemplary Methods for Inducing EMT

EMT is brought about in a cell by inducing the activity of certain transcription factors (See Table 1). Epithelial cells (e.g., immortalized cells such as HMLE, or transformed cells, such as HMLER) are generated that have one or more transgenes capable of expressing an EMT inducing transcription factor selected from: Snail1, Snail2, Goosecoid, FoxC2, TWIST, E2A, SIP-1/Zeb-2, dEF1/ZEb1, LEF1, Myc, HMGA2, TAZ, Klf8, HIF-1, HOXB7, SIM2s, and Fos. Expression of the transgene is constitutive or inducible, and induces an EMT in the cell.

**Table 1: Transcription factors that mediate EMT**

| Snail1 | Snail2 | Goosecoid |
|---|---|---|
| FoxC2 | Twist | E2A (coding for E12/E47) |
| SIP-1/Zeb-2 | dEF1/ZEb1 | LEF1 |
| Myc | HMG-box transcription factors-HMGA2 | TAZ |
| Klf8 | HIF-1 | HOXB7 |
| Loss of SIM2s | Fos | |

EMT is brought about by modulating the activity of a signaling pathway in a cell (See Table 2 and 3). An epithelial cell (e.g., immortalized cells such as HMLE, or transformed cells, such as HMLER) is contacted with a growth factor selected from: a TGF-β/BMP superfamily member, a Wnt-family member, an FGF family member, a Notch Ligand, an

EGF family member, an IGF family member, PDGF, and HGF, thereby inducing an EMT in the cell.

**Table 2: Major pathways that mediate EMT**

| TGF-beta | Wnt | BMP |
|---|---|---|
| Notch | HGF- Met | EGF, IGF, PDGF, FGF-Receptor tyrosine kinase signaling |
| P38-mapk | Ras | PI3Kinase- Akt |
| Src | Nf-kB | |

**Table 3: Exemplary pathways/molecules reported to mediate EMT**

| Hippo pathway | interleukin-like EMT inducer (ILEI) | Integrin-Integrin linked kinase |
|---|---|---|
| Endothelin-1 | Protein kinase A (PKA) and the signal transducer and activator of transcription 3 (STAT3) | Matrix metalloproteases (MMP3, MT-MMP1) |
| Reactive oxygen species (ROS) | NBS1 | uPAR |
| P68 RNA helicase | KCC3 (potassium chloride cotransporter) | Phosphatases: Pez, PRL-3 |

Other approaches that bring about EMT and/or E-Cadherin downregulation are outlined in Table 4. EMT is brought about by subjecting an epithelial cell (e.g., immortalized cells such as HMLE, or transformed cells, such as HMLER) to hypoxic conditions, irradiation, and chronic chemotherapy treatment; by treating an epithelial cell with blocking antibody to E-cadherin; by inducing the expression of dysadherin in an epithelial cell; and/or by inhibiting the expression of Scribble in an epithelial cell.

**Table 4: Other exemplary approaches**

| Hypoxia | Irradiation |
|---|---|
| Chronic chemotherapy treatment | Expression of dysadherin |
| Function blocking antibodies to E-Cadherin | Inhibition of adherens junction proteins (eg. catenins) |
| Interference with cell-polarity genes (e.g., Scribble) | Mechanical Stretching |

### Description of downstream activities (e.g., hit characterization and development)

We have established several downstream assays for the follow-up evaluation of screen hits with respect to their effects on cancer stem cells: (1) Treat cells in culture for a period of time with screen hits (e.g., compounds, compositions, antibodies, shRNAs, etc.), including the necessary controls, and then allow the cells to recover for a period (e.g., up to 12 hours, up to 24 hours, up to 48 hours, up to 72 hours etc.), and then perform FACS with markers that identify populations enriched for cancer stem cells (for example, but not limited to, CD44+/CD24- in the case of breast cancer). (2) Treat cells in culture for a period of time with screen hits, allow them to recover for a prescribed period of time, and then perform colony formation assays in suspension in the absence of chemical compound (for breast cancer cells, these assays are referred to as mammosphere formation assays). (3) Treat cells in culture for a period of time with screen hits, allow them to recover for a prescribed period of time, and then inject cells at various cell numbers subcutaneously into immunocompromised animals and evaluate the frequency of tumor formation. The number of injected cells could include 10^6, 10^5, 10^4, 10^3, and 10^2 cells. Using follow-up assays, such as those disclosed herein, one can evaluate whether or not a screen hit (or any drug with potential CSC-directed activity) is a bonafide inhibitor of cancer stem cells.

### EXAMPLE 5: Normal and neoplastic human breast stem-like cells express markers associated with an EMT

We analyzed stem-like markers in human mammary epithelial cells isolated directly either from reduction mammoplasties or breast carcinomas. We initially isolated CD44^{high}/CD24^{low} and CD44^{low}/CD24^{high} mammary epithelial cells from normal reduction mammoplasty tissues (Figure 7A) and gauged their mRNA expression pattern using real-time RT-PCR. The CD44^{high}/CD24^{low} cells expressed low levels of E-cadherin mRNA, high levels of N-cadherin mRNA, and elevated levels of the mRNAs specifying two key EMT-inducing transcription factors - SIP1 and FOXC2 - relative to the expression levels of the respective mRNAs in the CD44^{low}/CD24^{high} population (Figure 7B).

We also isolated CD44^{high}/CD24^{low} cells and CD44^{low}/CD24^{high} cells from three normal reduction mammoplasty tissues and from five neoplastic human breast tissues and carried out serial analysis of gene expression (SAGE) - an alternative method of gauging the spectrum of mRNAs expressed within tissues (Shipitsin et al., 2007). Relative to the CD44^{low}/CD24^{high} cells, the CD44^{high}/CD24^{low} cells expressed high levels of the mRNAs encoding mesenchymal markers, specifically *CDH2* (N-cadherin), *VIM* (Vimentin), *FN1* (Fibronectin), *ZEB2* (SIP-1), *FOXC2, SNAIL1* (Snail), *SNAIL2* (Slug), *TWIST1* and *TWIST2,* and a low level of the *CDH1I* (E-cadherin) mRNA (Figure 7C and Table 5). These data indicate that the CD44^{high}/CD24^{low} cells isolated from cultured immortalized MECs as well as from normal and neoplastic human tissue samples express multiple genes associated with cells that have undergone an EMT.

### EXAMPLE 6: EMT promotes the generation of cancer stem cells

The observations disclosed herein suggested mammary epithelial stem-like cells could be generated from more differentiated populations of normal mammary epithelial cells by inducing an EMT. By extension, we speculated that EMT could promote the generation of cancer stem cells from more differentiated neoplastic cells. To address this possibility, we induced an EMT in experimentally immortalized human mammary epithelial cells (Elenbaas et al., 2001; Hahn et al., 1999) that had been transformed by introduction of an activated form of the *HER2*/*neu* oncogene (HMLEN cells). These cells were also infected with a vector expressing the tamoxifen-activatable form of either the Snail (Snail-ER) or the Twist (Twist-ER) transcription factors as described earlier.

These cells underwent an EMT when treated with tamoxifen for ten days in monolayer culture (Figure 8A and 8B), similar to the behavior of the immortalized, untransformed precursors of these *HER2*/*neu*-transformed cells (Figure 9A and 9B). Following withdrawal of tamoxifen, we subjected these HMLEN cells to both soft agar and tumor sphere assays in the absence of tamoxifen; the first of these assays serves as an *in vitro* surrogate measure of tumorigenicity (Cifone and Fidler, 1980; Singh et al., 2004), while the second gauges stemness. Interestingly, we observed that the cells that had undergone an EMT following tamoxifen treatment formed at least 10-fold more tumor spheres than did control cells that had not been exposed to tamoxifen (Figure 8C). Equally important, the transformed cells that underwent an EMT formed ~10-fold more colonies in soft agar suspension culture than did the control, untreated cells (Figure 8D).

In order to gauge the tumorigenicity of the transformed cells that have undergone an EMT, we injected HMLEN cells that carried either Snail-ER or Twist-ER and had been treated with 4-OHT for 12 days prior to implantation in limiting dilutions into subcutaneous sites of mouse hosts. These cells failed to form tumors *in vivo,* which suggested that long-term maintenance of the EMT/stem-cell state depends on continuous EMT-inducing signals, at least in this experimental model. To test this notion further, we cultured the HMLEN cells that had undergone an EMT *in vitro* for an additional 15 days in the absence of 4-OHT. These cells reverted completely back to an epithelial phenotype (Figure 10), suggesting that maintenance of the stem-cell state by these cells depends, at least *in vitro,* on continuous EMT-inducing signals.

In order to test whether the constitutive expression of an EMT-inducing transcription factor succeeded in altering the tumor-initiating frequency of transformed cells, we injected HMLER cells [i.e., HMLE cells transformed with a V12H-Ras oncogene to render them tumorigenic; (Elenbaas et al., 2001)] and constitutively expressing either Snail or Twist into immunodeficient hosts. Similar to the previously observed behavior of HMLE cells, both Snail and Twist induced EMTs in these HMLER cells and increased the number of CD44^{high}/CD24^{low} cells and the ability to form mammospheres (Figure 11). The majority of the mice that were injected with 10³ Twist- or Snail-expressing HMLER cells formed tumors (6 of 9 - Snail; 7 of 9 - Twist), while no tumors arose when an equal number of cells expressing a control vector were injected into mice (Table 6). In fact, 10⁵ of the control cells (lacking either Twist or Snail expression) were required to initiate tumor formation, and even then, tumor formation was inefficient (3 of 9 injected hosts). Hence, expression of either the Twist or Snail EMT-inducing transcription factor significantly increased (by ~2 orders of magnitude) the number of tumor-initiating cells. The histology of the tumors developed from both the control cells and the cells expressing Snail or Twist appeared as squamous metaplasias, similar to that of the tumors formed by the parental cells (Figure 12), as reported earlier (Elenbaas et al., 2001).

**TABLE 5**

| | **CD44_{high}/CD24_{low}** | | | | | | **CD44_{low}/CD24_{high}** | | | | | | |
|---|---|---|---|---|---|---|---|---|---|---|---|---|---|
| **SAGE Tag** | **Normal** | | **Tumor** | | | | **Normal** | | | **Tumor** | | | **Gene Name** |
| **SEO ID NO 1: TGTGGGTGCTGATAATT** | 18 | 0 | 0 | 8 | 8 | 22 | 115 | 18 | 34 | 12 | 81 | 78 | ***CDH1* (E-cadherin)** |
| **SEQ ID NO 2: ATCTTGTTACTGTGATA** | 7 | 30 | 46 | 30 | 91 | 26 | 0 | 0 | 0 | 0 | 0 | 0 | ***FN1* (Fibronectin 1)** |
| **SEQ ID NO 3: TCCAAATCGATGTGGAT** | 1038 | 642 | 332 | 504 | 472 | 300 | 101 | 71 | 5 | 0 | 0 | 0 | ***VIM* (Vimentin)** |
| **SEQ ID NO 4: TGCGTCCCGCCCGCCCT** | 4 | 2 | 0 | 0 | 0 | 0 | 0 | 0 | 0 | 0 | 0 | 0 | ***FOXC2*** |
| **SEQ ID NO 5: GAATTCCCTCCTGAGTG** | 11 | 0 | 0 | 4 | 4 | 0 | 0 | 0 | 0 | 0 | 0 | 0 | ***SNAI1* (snail)** |
| **SEQ ID NO 6: CTCCATTGTCTTACTAT** | 0 | 5 | 4 | 0 | 4 | 4 | 0 | 0 | 0 | 0 | 0 | 0 | ***SNAI2* (Slug)** |
| **SEQ ID NO 7: GTAGATGCAAGGGAAAC .** | 0 | 0 | 0 | 0 | 4 | 0 | 0 | 5 | 0 | 0 | 0 | 0 | ***TCF-3* (Transcription factor 3)** |
| **SEQ ID NO 8: GTAAAATGCAAATAGAT** | 18 | 15 | 0 | 0 | 4 | 22 | 0 | 3 | 0 | 0 | 0 | 0 | ***Twist1* (Twist homolog 1)** |
| **SEQ ID NO 9: ATTGTTTCAAGTCAGCC** | 0 | 10 | 4 | 15 | 15 | 7 | 0 | 0 | 0 | 0 | 0 | 0 | ***Twist2* (Twist homolog 2)** |
| **SEQ ID NO 10: CTCGAATAAAAATGTAG** | 39 | 42 | 57 | 0 | 15 | 30 | 0 | 0 | 0 | 0 | 0 | 0 | ***ZFH1B* (Zinc finger homeobox 1b)** |

Table 5 discloses the sequence of SAGE tags specific for the EMT genes disclosed high low low high herein and the numbers of each tags present in CD44^{high} /CD24^{low} and CD44 /CD24 populations isolated from normal and neoplastic human mammary glands. The tumors samples were prepared from two invasive ductal carcinomas, one pleural effusion, and one ascites; the normal samples were prepared from two reduction mammoplasties.

**TABLE 6**

| **Cells injected** | **Tumors incidence/number of injections** | | | |
|---|---|---|---|---|
| | **1X10⁶** | **1X10⁵** | **1X10⁴** | **1X10³** |
| **HMLE-Vector-Ras** | 2/6 | 3/9 | 0/9 | 0/9 |
| **HMLE-Snail-Ras** | 6/6 | 9/9 | 9/9 | 6/9 |
| **HMLE-Twist-Ras** | 6/6 | 9/9 | 9/9 | 7/9 |

Table 6 discloses tumor incidence of transformed HMLEs induced to undergo EMT by ectopic expression of Snail or Twist and then injected into host mice in limiting dilutions.

### EXAMPLE 7:

**Cell culture** The immortalized human mammary epithelial cells (HMLE) were maintained as previously described (Elenbaas et al., 2001). The HMLE-Snail-ER, HMLE-Twist-ER, HMLEN-Snail-ER, and HMLEN-Twist-ER cells were generated by infecting the HMLE cells or HMLEN cells with pWZL-Snail-ER or pWZL-Twist-ER vectors followed by selection with 5 ng/ml of blasticidin. HMLE-Snail-Ras, HMLE-Twist-Ras, and HMLE-Vector-Ras cells were generated by infecting immortalized human mammary epithelial cells (Elenbaas et al., 2001) with retroviral vectors expressing Snail or Twist or the control vector and selecting with 2µg/ml puromycin. These cells were then transformed by introduction of a pWZL retroviral vector expressing the V12H-RAS oncogene followed by selection with 4µg/ml of blasticidin. For 4-hydroxy tamoxifen (4-OHT) treatment, The HMLE-Snail-ER, HMLE-Twist-ER, HMLEN-Snail-ER, and HMLEN-Twist-ER cells were exposed to 4-OHT at final concentration of 20 nM for the indicated number of days. The plasmids used in this study, procedures used to produce virus and the infection of target cells using these viruses are described herein.

### Antibodies, Western Blotting and Immunofluorescence

Cells were lysed in the presence of 50mM Tris PH7.5, 150nM NaCl and 0.5% NP-40 on ice. 50 µg of total protein from each sample was resolved on a 4-12% Bis-Tris Gel with MOPs Running Buffer and transferred to PVDF membranes. The blots were then probed with various antibodies, such as anti-β-actin (Abcam), anti-E-cadherin (BD Transduction), anti-Fibronectin (BD Transduction), anti-vimentin V9 (NeoMarkers), or anti-N-cadherin (BD Transduction).

**Soft agar and Tumorigenesis assays** Soft agar assays were performed as described previously (Cifone and Fidler, 1980). Cultures were photographed, and the colonies with diameters larger than 500 µm were counted using ImageJ software (previously NIH image). 105,104, and 103 of HMLE-Snail-Ras or HMLE-Twist-Ras or HMLE-Vector-Ras cells were injected subcutaneously into athymic nude mice. The tumor incidence was monitored for 25 days following injection. All mouse procedures were preapproved by the Animal Care and Use Committee of the Massachusetts Institute of Technology and performed according to institutional policies.

**Reverse Transcriptase PCR analysis** SYBR-Green Real-time RT-PCR and the corresponding data analysis were described earlier (Yang et al., 2004). For all RT-PCR analysis GAPDH mRNA was used to normalize RNA inputs. Primers sequence used to amplify genes are listed in Table 7.

**FACS analysis** The anti-CD44 (clone G44-26) and anti-CD24 (clone ML5) antibodies used for FACS analysis were obtained from BD Bioscience. To sort primary mouse mammary epithelial cells, mouse mammary glands were digested with collagenase (1 µg/ml), and the organoids were collected by brief centrifugation (800 rpm for 45 seconds). The organoids were digested with 0.025% trypsin for 5 min at 37°C in order to dissociate into single cells. The dissociated cells were stained with antibodies against CD49f (Pharmingen) and CD24 (Pharmingen) and several lineage markers (CD45, CD31, Ter-119 and CD140a (ebioscience), as described in Stingl et al 2006. The Lin--cells were used for sorting.

**Primary human samples and SAGE libraries** CD44^{high}/CD24^{low} and CD44^{low}/CD24^{high} cells were isolated from normal reduction mammoplasty tissues and breast tumors by sequential capturing of cell types with antibody-coupled beads specific for various cell markers (Shipitsin et al., 2007). Fresh tissue samples were collected from Harvardaffiliated hospitals and cell fractions were immediately purified. Three normal tissues, 1 pleural effusion, 2 ascites, and 3 invasive ductal carcinomas were collected in triplicate from different patients. Specimens were collected without patient identifiers following protocols approved by the Dana-Farber Cancer Institute Institutional Review Board.

**Heat map** The heat map in Figure 7C was produced using the SAGE tag counts in Table 5 which were visualized using MapleTree (developed by L. Simirenko). Before visualization, tag counts were log2-transformed (treating a count of 0 as if it were 1), median-centered by tag, and subjected to hierarchical complete linkage clustering by tags and SAGE libraries with uncentered correlation similarity metrics using Cluster (Eisen et al., 1998)

**Statistical analysis** All data are presented as mean ± SEM except other stated. When two groups were compared, the Student t-test was used (P < 0.05 was considered significant).

**Plasmids, virus production and infection of target cells** The development of pBp-Snail and pBp-Twist was reported earlier (Yang et al., 2004). The pWZL-Blast-Snail-ER and PWZL-Blast-Twist-ER constructs were generated by replacing the MYC cDNA of pWZL-Blast-DN-MycER (Littlewood et al., 1995; Watnick et al., 2003) with the hSnail cDNA PCR amplified from pBp-hSnail or the mTwist cDNA PCR-amplified from pBpmTwist. The production of lentiviruses and amphotropic retroviruses as well as the infection of target cells was described previously (Stewart et al., 2003).

**TABLE 7: Primers used to amplify genes:**

| | | |
|---|---|---|
| hFOXC2-5 | GCCTAAGGACCTGGTGAAGC | SEQ ID NO 11 |
| hFOXC2-3 | TTGACGAAGCACTCGTTGAG | SEQ ID NO 12 |
| hGAPDH-5 | ACCCAGAAGACTGTGGATGG | SEQ ID NO 13 |
| hGAPDH-3 | TCTAGACGGCAGGTCAGGTC | SEQ ID NO 14 |
| hEcad-5 | TGCCCAGAAAATGAAAAAGG | SEQ ID NO 15 |
| hEcad-3 | GTGTATGTGGCAATGCGTTC | SEQ ID NO 16 |
| hNcad-5 | ACAGTGGCCACCTACAAAGG | SEQ ID NO 17 |
| hNcad-3 | CCGAGATGGGGTTGATAATG | SEQ ID NO 18 |
| hFN1-5 | CAGTGGGAGACCTCGAGAAG | SEQ ID NO 19 |
| hFN1-3 | TCCCTCGGAACATCAGAAAC | SEQ ID NO 20 |
| hVim-5 | GAGAACTTTGCCGTTGAAGC | SEQ ID NO 21 |
| hVim-3 | GCTTCCTGTAGGTGGCAATC. | SEQ ID NO 22 |
| hSnail-5 | CCTCCCTGTCAGATGAGGAC | SEQ ID NO 23 |
| hSnail-3 | CCAGGCTGAGGTATTCCTTG | SEQ ID NO 24 |
| hTwist-5 | GGAGTCCGCACrTCTTACGAG | SEQ ID NO 25 |
| hTwist-3 | TCTGGAGGACCTGGTAGAGG | SEQ ID NO 26 |
| hSIP1-5 | TTCCTGGGCTACGACCATAC | SEQ ID NO 27 |
| hSIP1-3 | TGTGCTCCATCAAGCAATTC | SEQ ID NO 28 |
| hSlug-5 | GGGGAGAAGCCTTTTTCTTG | SEQ ID NO 29 |
| hSlug-3 | TCCTCATGTTTGTGCAGGAG | SEQ ID NO 30 |

**Immunofluorescence** About 2.5x10⁴ cells were seeded on a 4-well Lab-Tekll Chamber Slide. After 24 hrs, the cells were washed with phosphate-buffered saline (PBS) twice and permeabilized and fixed in 2% paraformaldehyde and 0.1% Triton X100 in PBS buffer at 40C for 30 minutes. The cells were then washed 3 times with PBS and incubated with the blocking solution (10% goat serum in PBS). The cells were then incubated with the primary antibodies for (2 hr to overnight), washed 3 times with PBS plus 0.1% Tween-20 for 15 minutes, and finally incubated with secondary antibodies (Invitrogen) and DAPI for 2 hours. The slides were washed extensively with PBS and mounted with slow fade Light Anti fade Kit (Invitrogen). All matched samples were photographed (control and test) using immunofluorescence microscope and identical exposure times.

### REFERENCES FOR EXAMPLE 5,6, AND 7

Cifone, M.A., and Fidler, I.J. (1980). Correlation of patterns of anchorage-independent growth with in vivo behavior of cells from a murine fibrosarcoma. Proc Natl Acad Sci U S A 77, 1039-1043.
Eisen, M.B., Spellman, P.T., Brown, P.O., and Botstein, D. (1998). Cluster analysis and display of genome-wide expression patterns. Proc Natl Acad Sci U S A 95, 14863-14868.
Elenbaas, B., Spirio, L., Koerner, F., Fleming, M.D., Zimonjic, D.B., Donaher, J.L., Popescu, N.C., Hahn, W.C., and Weinberg, R.A. (2001). Human breast cancer cells generated by oncogenic transformation of primary mammary epithelial cells. Genes Dev 15, 50-65.
Hahn, W.C., Counter, C.M., Lundberg, A.S., Beijersbergen, R.L., Brooks, M.W., and Weinberg, R.A. (1999). Creation of human tumour cells with defined genetic elements. Nature 400,464468.
Littlewood, T.D., Hancock, D.C., Danielian, P.S., Parker, M.G. & Evan, G.I. (1995). Nucleic Acids Res, 23, 1686-90.
Shipitsin, M., Campbell, L.L., Argani, P., Weremowicz, S., Bloushtain-Qimron, N., Yao, J., Nikolskaya, T., Serebryiskaya, T., Beroukhim, R., Hu, M., et al. (2007). Molecular definition of breast tumor heterogeneity. Cancer Cell 11, 259-273.
Singh, S.K., Hawkins, C., Clarke, I.D., Squire, J.A., Bayani, J., Hide, T., Henkelman, R.M., Cusimano, M.D., and Dirks, P.B. (2004). Identification of human brain tumour initiating cells. Nature 432, 396-401.
Stewart SA, Dykxhoom DM, Palliser D, Mizuno H, Yu EY, An DS, Sabatini DM, Chen IS, Hahn WC, Sharp PA, Weinberg RA, Novina CD. Lentivirus-delivered stable gene silencing by RNAi in primary cells. RNA. 2003 Apr;9(4):493-501.
Stingl, J., Eirew, P., Ricketson, I., Shackleton, M., Vaillant, F., Choi, D., Li, H.I., and Eaves, C.J. (2006). Purification and unique properties of mammary epithelial stem cells. Nature 439, 993-997.
Watnick R.S., Cheng Y.N., Rangarajan A., Ince T.A., Weinberg R.A. 2003. Ras modulates Myc activity to repress thrombospondin- 1 expression and increase tumor angiogenesis. Cancer Cell 3: 219-231
Yang, J., Mani, S.A., Donaher, J.L., Ramaswamy, S., Itzykson, R.A., Come, C., Savagner, P., Gitelman, I., Richardson, A., and Weinberg, R.A. (2004). Twist, a master regulator of morphogenesis, plays an essential role in tumor metastasis. Cell 117, 927939.

### EXAMPLE 8

The effects of salinomycin on cancer stem cell growth were examined. HMLER cells were treated with salinomycin, or paclitaxel or DMSO, as controls, for 4 days, at various doses. (Figure 13A.) The percentage of CD44^{high}/CD24^{low} cells following compound treatment was quantified by fluorescence-activated cell sorting. Two independent experiments with two different HMLER cell populations (HMLER_1, HMLER_2) were conducted and bar charts were generated to depict the results. (Figure 13A.) Scatter plots of fluorescence-activated cell sorting profiles of HMLER_2 cancer cell populations treated with salinomycin or paclitaxel were also examined. (Figure 13B.)

The mammosphere forming potential of parental HMLER cells treated with either DMSO, paclitaxel (taxol) or salinomycin was also examined at various doses. Salinomycin, but not paclitaxel inhibited mammosphere formation in MCF7Ras or 4T1 cells. (Figure 14.) The effects of salinomycin, paclitaxel or vehicle control treatments on *in vivo* tumor formation by SUM159 breast cancer cells injected in mice was determined. Mice treated with Salinomycin or Paclitaxel had reduced tumor volume compared with mice treated with the vehicle control. (Figure 15A.) The mammosphere-forming potential of cancer cells obtained from SUM 159 tumors from salinomycin, paclitaxel or DMSO-treated mice was also determined. (Figure 15B.)

The extent to which Salinomycin treatment reduces the expression of clinically relevant breast CSC and progenitor genes was also determined. Gene set enrichment analysis was used to determine whether three previously reported sets of genes associated with stem-like cells were repressed in response to salinomycin in comparison with paclitaxel treatment. Graphed are the Kolmogorov-Smimov enrichment scores versus Gene ranks based on differential expression. P-values reflecting statistical significance for each analysis are shown. The three previously reported gene signatures are: CD44+CD24- IGS (Liu et al., 2007), CD44+CD24- (Shipitsin et al., 2007) and Mammosphere (Dontu et al., 2003). (Figure 16.)

### References:

Liu, R., Wang, X., Chen, G.Y., Dalerba, P., Gurney, A., Hoey, T., Sherlock, G., Lewicki, J., Shedden, K., and Clarke, M.F. (2007). The prognostic role of a gene signature from tumorigenic breast-cancer cells. The New England journal of medicine 356, 217-226.
Shipitsin, M., Campbell, L.L., Argani, P., Weremowicz, S., Bloushtam-Qimron, N., Yao, J., Nikolskaya, T., Serebryiskaya, T., Beroukhim, R., Hu, M., et al. (2007). Molecular definition of breast tumor heterogeneity. Cancer cell 11, 259-273.
Dontu, G., Abdallah, W.M., Foley, J.M., Jackson, K.W., Clarke, M.F., Kawamura, M.J., and Wicha, M.S. (2003). In vitro propagation and transcriptional profiling of human mammary stem/progenitor cells. Genes & development 17, 1253-1270.
Having thus described several aspects of at least one embodiment of this invention, it is to be appreciated various alterations, modifications, and improvements will readily occur to those skilled in the art. Such alterations, modifications, and improvements are intended to be part of this disclosure, and are intended to be within the spirit and scope of the invention. Accordingly, the foregoing description and drawings are by way of example only. All references described herein are incorporated by reference for the purposes described herein. Moreover, this invention is not limited in its application to the details of construction and the arrangement of components set forth in the disclosed description or illustrated in the drawings. The invention is capable of other embodiments and of being practiced or of being carried out in various ways. Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

### Preferred embodiments

1. A method for testing the ability of a compound to inhibit the growth and/or survival of a cancer stem cell, comprising
   (a) contacting one or more test cells with a sample of the compound, wherein the one or more test cells has undergone an epithelial to mesenchymal transition, and
   (b) detecting the level of inhibition of the growth and/or survival of the one or more test cells by the compound.
2. The method of item 1, wherein the epithelial to mesenchymal transition results from inhibiting the activity of E-Cadherin in the one or more test cells.
3. The method of item 2, wherein the inhibiting the activity of E-Cadherin in the one or more test cells comprises: contacting the one or more test cells with a blocking antibody to E-Cadherin, inducing the expression of dysadherin in the one or more test cells, or interfering with cell-polarity genes in the one or more test cells.
4. The method of item 2, wherein the inhibiting the activity of E-Cadherin in the one or more test cells comprises contacting the one or more test cells with a small-interfering nucleic acid complementary to E-Cadherin mRNA.
5. The method of item 1, wherein the epithelial to mesenchymal transition results from inducing the activity of a transcription factor in the one or more test cells, wherein the transcription factor is selected from: Snail1 Snail2, Goosecoid, FoxC2, TWIST, E2A, SIP-1/Zeb-2, dEF1/ZEb1, LEF1, Myc, HMGA2, TAZ, K1f8, HIF-1, HOXB7, SIM2s, and Fos.
6. The method of item 1, wherein the epithelial to mesenchymal transition results from inducing the activity of TWIST.
7. The method of item 6, wherein the inducing the activity of TWIST in the one or more test cells comprises contacting the one or more test cells with an expression vector encoding TWIST.
8. The method of item 1, wherein the epithelial to mesenchymal transition results from contacting the one or more test cells with a growth factor selected from: a TGF-β/BMP superfamily member, a Wnt-family member, an FGF family member, a Notch Ligand, an EGF family member, an IGF family member, PDGF, and HGF.
9. The method of item 1, wherein the epithelial to mesenchymal transition results from modulating the activity of a signaling pathway in the one or more test cells, wherein the signaling pathway is selected from TGF-β, Wnt, BMP, Notch, HGF-Met, EGF, IGF, PDGF, FGF, P38-mapk, Ras, PI3Kinase-Akt, Src, and NF-kB.
10. The method of item 1, wherein the epithelial to mesenchymal transition results from subjecting the one or more test cells to a stress selected from: hypoxia, irradiation, and chronic chemotherapy treatment.
11. The method of item 1, wherein the epithelial to mesenchymal transition results from subjecting the one or more test cells to treatment with nicotine, nAChR agonists, hydrogen peroxide, C3a, or MFG-E8.
12. The method of item 1, wherein the one or more test cells comprise non-tumorigenic cells.
13. The method of item 1, wherein the one or more test cells comprise tumorigenic cells.
14. The method of item 1, wherein the method comprises
   (a) contacting one or more test cells and one or more control cells with a sample of the compound, wherein the one or more test cells has undergone an epithelial to mesenchymal transition and the one or more control cells has not undergone an EMT, and
   (b) detecting the level of inhibition of the growth and/or survival of the one or more test cells and control cells by the compound; and
   (c) identifying the compound as a candidate CSC-selective chemotherapeutic agent if the compound has a greater inhibitory effect on the growth and/or survival of the test cells than the control cells.
15. The method of item 14, wherein the test cells and the control cells are genetically matched.
16. The method of item 14, wherein the test cells express or contain a small interfering RNA that inhibits expression of E-cadherin and the control cells do not express such an RNA.
17. The method of any of items 1 to 16, further comprising contacting one or more control cells with a sample of the compound and detecting the level of inhibition of the growth and/or survival of the one or more control cells by the compound.
18. The method of item 17, wherein the one or more control cells is an epithelial cell that has not undergone an epithelial to mesenchymal transition.
19. The method of item 17 or item 18, wherein the one or more control cells is contacted with a control expression construct or a control small-interfering nucleic acid.
20. The method of item 19, wherein the control small-interfering nucleic acid does not target an endogenous gene of the one or more control cells, optionally wherein the small-interfering nucleic acid targets GFP mRNA.
21. The method of item 20, wherein the control expression construct encodes a GFP.
22. The method of item 21, wherein the control expression construct encodes a reporter protein.
23. The method of item 18, wherein the one or more control cells comprise non-tumorigenic cells.
24. The method of item 18, wherein the one or more control cells comprise tumorigenic cells.
25. The method of any of items I to 24, wherein each of the one or more test cells is contacted with a different dose of, and/or for a different duration with, the compound than at least one other test cell; and/or wherein each of the one or more control cells is contacted with a different dose of, and/or for a different duration with, the compound than at least one other control cell.
26. The method of item 25, further comprising analyzing a test and/or control dose response curve, wherein the test dose response curve indicates the level of inhibition of the one or more test cells by the compound at a plurality of doses; and wherein the control dose response curve indicates the level of inhibition on the one or more control cells by the compound at a plurality of doses.
27. The method of item 26, wherein the analyzing comprises determining an EC50 value for compound on the one or more test cells and/or the one or more control cells.
28. The method of item 27, wherein the EC50 value for the compound on the one or more control cells is statistically significantly less than the EC50 value for the compound on the one or more test cells.
29. The method of item 27, wherein the EC50 value for the compound on the one or more control cells is statistically significantly greater than the EC50 value for the compound on the one or more test cells.
30. The method of any of items 25 to 28, wherein the compound is a control compound, optionally which is selected from doxorubicin, paclitaxel, actinomycin D, camptothecin, and staurosporine.
31. The method of any of items 17 to 24, wherein the one or more control cells and the one or more test cells are in a co-culture.
32. The method of item 31, wherein the one or more test cells have an identifying characteristic that is detectable and distinct from an identifying characteristic of the one or more control cells, optionally wherein the identifying characteristic comprises a level of expression of GFP protein and/or a cancer stem cell biomarker.
33. The method of item 32, wherein the level of expression of GFP protein in the one or more test cells is compared with the level of expression of GFP protein in the one or more control cells.
34. The method of item 32 or item 33, further comprising monitoring the ratio of the one or more test cells to one or more control cells by detecting the identifying characteristic of each cell in a sample of the co-culture, optionally wherein the detecting comprises fluorescence-activated cell sorting.
35. The method of any of items 1 to 24, further comprising testing a plurality of test samples, wherein each of the test samples comprises at least one of the one or more test cells; and/or further comprising testing a plurality of control samples, wherein each of the control samples comprises at least one of the one or more control cells.
36. The method of item 35, wherein each test sample and/or each control sample is in a separate culture chamber, optionally wherein each culture chamber is a well of a multi-well plate.
37. The method of item 36, wherein the multi-well plate has a number of wells selected from 6, 12, 24, 96, 384, or 1536.
38. The method of any of items 1 to 24 and 35 to 37, wherein the compound is obtained from a library consisting of a plurality of compounds, optionally wherein the library is selected from a natural products library, a diversity library, a kinase-inhibitor library, a HDAC-inhibitor library, a library of known bioactive compounds, a peptide library, an antibody library, or an RNAi library.
39. The method of item 38, wherein the contacting the one or more test cells comprises transferring a sample of the compound from the library to a culture chamber containing the test sample; and/or wherein the contacting the one or more control cells comprises transferring a sample of the compound from the library to a culture chamber containing the control sample.
40. The method of item 39, further comprising identifying a lead compound that is substantially more cytotoxic to the one or more test cells than the one or more control cells, by comparing the level of inhibition of the growth and/or survival of the one or more test cells by the compound to the level of inhibition of the growth and/or survival of the one or more control cells by the compound.
41. The method of item 40, further comprising contacting one or more cancer cells with a sample of the lead compound, and detecting the level of inhibition of the growth and/or survival of the one or more cancer cells by the lead compound.
42. The method of item 41, wherein the one or more cancer cells are obtained from a colon carcinoma, a pancreatic cancer, a breast cancer, an ovarian cancer, a prostate cancer, a squamous cell carcinoma, a cervical cancer, a lung carcinoma, a small cell lung carcinoma, a bladder carcinoma, a squamous cell carcinoma, a basal cell carcinoma, an adenocarcinoma, a sweat gland carcinoma, a sebaceous gland carcinoma, a papillary carcinoma, a papillary adenocarcinoma, a cystadenocarcinoma, a medullary carcinoma, a bronchogenic carcinoma, a renal cell carcinoma, a hepatocellular carcinoma, a bile duct carcinoma, a choriocarcinoma, a seminoma, a embryonal carcinoma, a Wilms' tumor, or a testicular tumor.
43. The method of item 41 or 42, wherein at least one of the one or more cancer cells is a cancer stem cell, which has a cancer stem cell biomarker.
44. The method of item 35 or 43, wherein the cancer stem cell biomarker is the cell surface marker profile of CD44⁺ and CD24⁻.
45. The method of item 42 or 43, wherein the cancer stem cell is a MDA-MB-231, SUM159, or T47D breast cancer cell.
46. The method of any of items 41 to 45, wherein the detecting the level of inhibition of the growth and/or survival of the one or more cancer cells comprises determining the fraction of the one or more cancer cells that is the at least one cancer stem cell.
47. The method of any of items 47 to 45, wherein the detecting the level of inhibition of the growth and/or survival of the one or more cancer cells comprises determine the capacity of the one or more cancer cells to form colonies in suspension culture.
48. The method of any of items 41 to 45, wherein the detecting the level of inhibition of the growth and/or survival of the one or more cancer cells comprises determine the capacity of the one or more cancer cells to form tumors *in vivo.*
49. The method of any of items 40 to 48, further comprising producing a refined lead compound by modifying the lead compound to achieve (i) improved potency, (ii) decreased toxicity, which may optionally be an improved therapeutic index; (iii) decreased side effects; (iv) modified onset of therapeutic action and/or duration of effect; and/or (v) modified pharmacokinetic parameters, which may optionally be absorption, distribution, metabolism and/or excretion.
50. The method of item 49, further comprising determining the *in vivo* toxicology profile of the lead or refined lead compound by a performing a quantitative structure activity relationship analysis of the lead or refined lead compound.
51. The method of item 49 or 50, further comprising producing a pharmaceutical composition by formulating the lead or refined lead compound with a pharmaceutically acceptable carrier.
52. The method of item 51, further comprising testing the lead or refined lead compound *in vivo,* by administering the pharmaceutical composition to a subject having a cancer cell, and evaluating the toxicity of the compound to the subject and/or the effect of the compound on the growth and/or survival of the cancer cell in the subject.
53. The method of item 52, wherein the subject is selected from a mouse, a rat, a rabbit, a dog, a cat, a sheep, a pig, a non-human primate, and a human.
54. The method of any of items 1 to 24, further comprising determining the expression of one or more cancer stem cell markers in the test cell and/or the control cell.
55. The method of item 54, wherein the one or more cancer stem cell markers are selected from: CD20, CD24, CD34, CD38, CD44, CD45, CD105, CD133, CD166, EpCAM, ESA, SCA1, Pecam, and Stro1.
56. The method of item 55, wherein the one or more cancer stem cell markers are CD24 and CD44.
57. The method of any of items 1 to item 56, wherein the detecting the level of inhibition of the growth and/or survival of the test cell and/or the control cell by the compound comprises performing an assay selected from: a cell counting assay, a replication labeling assay, a cell membrane integrity assay, a cellular ATP-based viability assay, a mitochondrial reductase activity assay, a caspase activity assay, an Annexin V staining assay, a DNA content assay, a DNA degradation assay, and a nuclear fragmentation assay.
58. A method for characterizing one or more cells, comprising
   (a) contacting the one or more cells with a compound selected from doxorubicin, paclitaxel, actinomycin D, camptothecin, and staurosporine,
   (b) detecting a level of inhibition of the growth and/or survival of the one or more cells by the compound, and
   (c) comparing the results of (b) to a control level, wherein if the level of inhibition of the growth and/or survival of the one or more cells by the compound is statistically significantly less than the control level then the one or more cells have a cancer stem cell characteristic.
59. The method of item 58, further comprising evaluating a cancer stem cell biomarker or functional assay in the one or more cells.
60. The method of item 59, wherein the cancer stem cell biomarker is selected from E-cadherin expression, TWIST expression, and a CD44/CD24 cell surface marker profile.
61. The method of item 59, wherein the functional assay is a colony formation assay or an in vivo tumor seeding assay.
62. The method of item 58, wherein the control level is a level of inhibition of the growth and/or survival of one or more cancer cells that are not cancer stem cells by the compound.
63. A method for treating a subject having, or suspected of having, cancer comprising administering to the subject an effective amount of paclitaxel in combination with an effective amount of a pharmaceutical composition comprising etoposide, salinomycin, abamectin, or nigericin.
64. A method for treating a subject having, or suspected of having, cancer comprising administering to the subject an effective amount of a pharmaceutical composition comprising etoposide, salinomycin, abamectin, nigericin, or a derivative of any of the foregoing.
65. A method for selecting a treatment for a subject having cancer comprising evaluating a cancer stem cell biomarker in the cancer and, if the cancer stem cell biomarker is detected, treating the subject by administering to the subject an effective amount of a pharmaceutical composition comprising salinomycin, abamectin, etoposide or nigericin or a derivative thereof, optionally in combination with paclitaxel or a derivative thereof.
66. The method of item 65, wherein the cancer stem cell biomarker is selected from:
   E-cadherin expression; TWIST expression, and a CD44/CD24 cell surface marker profile.
67. The method of any of items 63 to 66, wherein evaluating the cancer stem cell biomarker comprises obtaining a sample of the cancer from the subject.
68. The method of item 66, wherein the E-cadherin and/or TWIST expression in the cancer is determining by measuring a level of E-cadherin and/or TWIST protein and/or RNA expression in the cancer, and optionally comparing the level to a reference standard.
69. The method of item 68, wherein the reference standard is the level of E-cadherin and/or TWIST protein and/or RNA expression in a cancer stem cell.
70. The method of item 68, wherein the reference standard is the level of E-cadherin and/or TWIST protein and/or RNA expression in a cancer cell that is not a cancer stem cell.
71. The method of any of items 63 to 70, wherein the cancer is a colon carcinoma, a pancreatic cancer, a breast cancer, an ovarian cancer, a prostate cancer, a squamous cell carcinoma, a cervical cancer, a lung carcinoma, a small cell lung carcinoma, a bladder carcinoma, a squamous cell carcinoma, a basal cell carcinoma, an adenocarcinoma, a sweat gland carcinoma, a sebaceous gland carcinoma, a papillary carcinoma, a papillary adenocarcinoma, a cystadenocarcinoma, a medullary carcinoma, a bronchogenic carcinoma, a renal cell carcinoma, a hepatocellular carcinoma, a bile duct carcinoma., a choriocarcinoma, a seminoma, a embryonal carcinoma, a Wilms' tumor, or a testicular tumor.
72. The method of any of items 63 to 71, wherein the cancer is a breast carcinoma or a lung carcinoma.
73. The method of any of items 63 to 72, wherein the subject is mammal, which is optionally a human.
74. The method of any of items 63 to 73, wherein the pharmaceutical composition is administered intravenously, intramuscularly, subcutaneously, intraperitoneally, orally or as an aerosol.
75. A method for treating a subject having, or suspected of having, cancer comprising administering to the subject an effective amount of a pharmaceutical composition comprising a compound that selectively inhibits growth and/or proliferation of test cells that have undergone an EMT relative to inhibition of growth and/or proliferation of control cells that have not undergone an EMT.
76. The method of item 75, wherein the test and control cells are genetically matched.
77. The method of item 75, wherein the test cells are non-tumorigenic.
78. A method for treating a subject having, or suspected of having, cancer comprising administering to the subject an effective amount of a compound that selectively inhibits proliferation of, or kills, cancer stem cells relative to its effect on non-CSC cancer cells.
79. A method for treating a subject having, or suspected of having, cancer comprising administering to the subject an effective amount of a compound that selectively inhibits proliferation of, or kills, cancer stem cells relative to its effect on noncancerous cells.
80. A method of identifying a target for drug discovery, comprising:
   (a) providing a compound that selectively inhibits growth and/or proliferation of the test cells, wherein the test cells are cells that have undergone EMT; and
   (b) identifying a biological target of the compound.
81. The method of item 80, wherein the biological target is a protein or RNA expressed by the test cells.
82. The method of item 80, wherein the compound is a compound listed in Table 1 or a derivative therof.
83. The method of item 80, further comprising performing a screen to identify a second compound that interacts with or acts on the biological target.
84. A method of identifying a target for drug discovery comprising steps of:
   (a) contacting test cells or test cell lysate with a compound that selectively inhibits growth and/or proliferation of the test cells, wherein the contacting is performed under conditions in which the compound can physically interact with cellular biomolecules, and wherein the test cells are cells that have undergone EMT;
   (b) isolating a cellular biomolecule that physically interacts with the compound; and
   (c) identifying the biomolecule.
85. The method of item 84, further comprising performing a screen to identify a second compound that interacts with or acts on the biomolecule.
86. The method of item 84, wherein the compound is a compound listed in Table 1 or a derivative thereof.
87. The method of item 84, wherein the compound is attached to a support thereby forming an affinity matrix.
88. A method of generating a cancer stem cell, the method comprising inducing a cancer cell to undergo an EMT.
89. The method of item 88, wherein the cancer cell is an experimentally produced cancer cell.
90. The method of item 88, wherein the cancer cell is derived from a naturally occurring cancer.
91. The method of item 88, wherein the epithelial to mesenchymal transition results from inhibiting the activity of E-Cadherin in the cancer cell.
92. The method of item 91, wherein the inhibiting the activity of E-Cadherin in the cancer cell comprises: contacting the cancer cell with a blocking antibody to E-Cadherin, inducing the expression of dysadherin in the cancer cell, or interfering with cell-polarity genes in the cancer cell.
93. The method of item 92, wherein the inhibiting the activity of E-Cadherin in the cancer cell comprises contacting the cancer cell with a small-interfering nucleic acid complementary to E-Cadherin mRNA.
94. The method of item 88, wherein the cancer cell is induced to undergo EMT by expressing a transcription factor in the one or more test cells, wherein the transcription factor is selected from: Snail1, Snail2, Goosecoid, FoxC2, TWIST, E2A, SIP-1/Zeb-2, dEF1/ZEb1, LEF1, Myc, HMGA2, TAZ, K1f8, HIF-1, HOXB7, SIM2s, and Fos.
95. The method of item 94, wherein the cancer cell is induced to undergo EMT by constitutively inducing the activity of the transcription factor.
96. The method of item 88, wherein the cancer stem cell has an increased likelihood of (i) initiating a tumor; (ii) forming a colony in soft agar suspension culture; and/or (iii) forming a tumor sphere, relative to the likelihood had the cancer cell not been induced to undergo EMT.
97. The method of item 89, wherein the experimentally produced cancer cell comprises an expression vector encoding an oncogene.
98. The method of item 97, wherein the oncogene is V12H-RAS.
99. The method of item 88, further comprising assessing the ability of a test agent to inhibit proliferation, colony formation in soft agar suspension culture, tumor sphere formation, and/or tumor initiating ability of the cancer stem cell.
100. The method of item 99, wherein the test agent is identified as a candidate agent for cancer therapy if the proliferation, colony formation in soft agar, suspension culture, tumor sphere formation, and/or tumor initiating ability of the cancer stem cell is inhibited.
101. The method of item 88, further comprising introducing the cancer cell having properties of a cancer stem cell into an animal host.
102. The method of item 101, further comprising assessing the ability of a test agent to inhibit proliferation or tumor initiating ability of the cancer stem cell in the animal host.
103. The method of item 102, wherein the test agent is identified as a candidate agent for cancer therapy if the proliferation or tumor initiating ability of the cancer stem cell is inhibited.
104. A cancer stem cell generated by the method of any of items 88 to 98.
105. An animal host comprising the cell of item 104.
106. A kit comprising a container having the cancer stem cell of item 104.
107. The kit of item 106, further comprising a second container having a cancer cell that has not been induced to undergo an EMT and that is genetically matched with the cancer cell that has been induced to undergo an EMT to generate the cancer stem cell.
108. The kit of item 106 or 107, wherein the container, or containers, further comprise a cryopreservation agent.

## Claims

1. A method for testing the ability of a compound to inhibit the growth and/or survival of a cancer stem cell, comprising
(a) contacting one or more test cells with a sample of the compound, wherein the one or more test cells has undergone an epithelial to mesenchymal transition, and
(b) detecting the level of inhibition of the growth and/or survival of the one or more test cells by the compound,
optionally wherein the method further comprises:
(c) contacting one or more control cells with a sample of the compound, wherein the one or more control cells has not undergone an EMT,
(d) detecting the level of inhibition of the growth and/or survival of the one or more test cells and the one or more control cells by the compound; and
(e) identifying the compound as a candidate CSC-selective chemotherapeutic agent if the compound has a greater inhibitory effect on the growth and/or survival of the test cells than the control cells.

2. The method of claim 1, wherein the epithelial to mesenchymal transition results from
(i) inhibiting the activity of E-Cadherin in the one or more test cells; or
(ii) inducing the activity of a transcription factor in the one or more test cells, wherein the transcription factor is selected from: Snail1, Snail2, Goosecoid, FoxC2, TWIST, E2A, SIP-1/Zeb-2, dEF1/ZEb1, LEF1, Myc, HMGA2, TAZ, Klf8, HIF-1, HOXB7, SIM2s, and Fos; or
(iii) inducing the activity of TWIST, optionally wherein inducing the activity of TWIST in the one or more test cells comprises contacting the one or more test cells with an expression vector encoding TWIST; or
(iv) contacting the one or more test cells with a growth factor selected from: a TGF-β/BMP superfamily member, a Wnt-family member, an FGF family member, a Notch Ligand, an EGF family member, an IGF family member, PDGF, and HGF; or
(v) modulating the activity of a signaling pathway in the one or more test cells, wherein the signaling pathway is selected from TGF-β, Wnt, BMP, Notch, HGF-Met, EGF, IGF, PDGF, FGF, P38-mapk, Ras, P13Kinase-Akt, Src, and NF-kB; or
(vi) subjecting the one or more test cells to a stress selected from: hypoxia, irradiation, and chronic chemotherapy treatment; or
(vii) subjecting the one or more test cells to treatment with nicotine, nAChR agonists, hydrogen peroxide, C3a, or MFG-E8.

3. The method of claim 2, wherein the inhibiting the activity of E-Cadherin in the one or more test cells comprises:
contacting the one or more test cells with a blocking antibody to E-Cadherin, inducing the expression of dysadherin in the one or more test cells; or
interfering with cell-polarity genes in the one or more test cells; or
inhibiting the activity of E-Cadherin in the one or more test cells comprises contacting the one or more test cells with a small-interfering nucleic acid complementary to E-Cadherin mRNA.

4. The method of claim 1, wherein the test cells and the control cells are genetically matched,
optionally wherein the test cells express or contain a small interfering RNA that inhibits expression of E-cadherin and the control cells do not express such an RNA.

5. The method of any of claims 1 to 4 , wherein each of the one or more test cells is contacted with a different dose of, and/or for a different duration with, the compound than at least one other test cell; and/or
wherein each of the one or more control cells is contacted with a different dose of, and/or for a different duration with, the compound than at least one other control cell,
optionally wherein the method further comprises analyzing a test and/or control dose response curve, wherein the test dose response curve indicates the level of inhibition of the one or more test cells by the compound at a plurality of doses; and wherein the control dose response curve indicates the level of inhibition on the one or more control cells by the compound at a plurality of doses, optionally wherein the step of analyzing comprises determining an EC50 value for compound on the one or more test cells and/or the one or more control cells, and/or
optionally wherein the compound is a control compound, optionally which is selected from doxorubicin, paclitaxel, actinomycin D, camptothecin, and staurosporine.

6. The method of any of claims 1-4, wherein the one or more control cells and the one or more test cells are in a co-culture,
optionally wherein the one or more test cells have an identifying characteristic that is detectable and distinct from an identifying characteristic of the one or more control cells, optionally wherein the identifying characteristic comprises a level of expression of GFP protein and/or a cancer stem cell biomarker,
optionally wherein the level of expression of GFP protein in the one or more test cells being different than the level of expression of GFP protein in the one or more control cells distinguishes test cells from control cells.

7. The method of any of claims 1 to 4, further comprising testing a plurality of test samples, wherein each of the test samples comprises at least one of the one or more test cells; and/or further comprising testing a plurality of control samples, wherein each of the control samples comprises at least one of the one or more control cells,
optionally wherein each test sample and/or each control sample is in a separate culture chamber, optionally wherein each culture chamber is a well of a multi-well plate, and optionally wherein the multi-well plate has a number of wells selected from 6, 12, 24, 96, 384, or 1536.

8. The method of any of claims 1 to 4 and 7, wherein the compound is obtained from a library consisting of a plurality of compounds, optionally wherein the library is selected from a natural products library, a diversity library, a kinase-inhibitor library, a HDAC-inhibitor library, a library of known bioactive compounds, a peptide library, an antibody library, or an RNAi library,
optionally wherein the contacting the one or more test cells comprises transferring a sample of the compound from the library to a culture chamber containing the test sample; and/or wherein the contacting the one or more control cells comprises transferring a sample of the compound from the library to a culture chamber containing the control sample.

9. The method of claim 8, further comprising identifying a lead compound that is substantially more cytotoxic to the one or more test cells than the one or more control cells, by comparing the level of inhibition of the growth and/or survival of the one or more test cells by the compound to the level of inhibition of the growth and/or survival of the one or more control cells by the compound,
optionally wherein the method further comprises contacting one or more cancer cells with a sample of the lead compound, and detecting the level of inhibition of the growth and/or survival of the one or more cancer cells by the lead compound,
optionally wherein the one or more cancer cells are obtained from a colon carcinoma, a pancreatic cancer, a breast cancer, an ovarian cancer, a prostate cancer, a squamous cell carcinoma, a cervical cancer, a lung carcinoma, a small cell lung carcinoma, a bladder carcinoma, a squamous cell carcinoma, a basal cell carcinoma, an adenocarcinoma, a sweat gland carcinoma, a sebaceous gland carcinoma, a papillary carcinoma, a papillary adenocarcinoma, a cystadenocarcinoma, a medullary carcinoma, a bronchogenic carcinoma, a renal cell carcinoma, a hepatocellular carcinoma, a bile duct carcinoma, a choriocarcinoma, a seminoma, a embryonal carcinoma, a Wilms' tumor, or a testicular tumor.

10. The method of claim 9, wherein at least one of the one or more cancer cells is a cancer stem cell that has a cancer stem cell biomarker,
optionally wherein the cancer stem cell biomarker is the cell surface marker profile of CD44⁺ and CD24⁻.

11. The method of claim 10, wherein the cancer stem cell is a MDA-MB-231, SUM159, or T47D breast cancer cell.

12. The method of any of claims 9 to 11, wherein the step of detecting the level of inhibition of the growth and/or survival of the one or more cancer cells comprises:
determining the fraction of the one or more cancer cells that is the at least one cancer stem cell, or
determining the capacity of the one or more cancer cells to form colonies in suspension culture, or
determining the capacity of the one or more cancer cells to form tumors *in vivo.*

13. The method of any of claims 9 to 12, further comprising producing a refined lead compound by modifying the lead compound to achieve (i) improved potency, (ii) decreased toxicity, which may optionally be an improved therapeutic index; (iii) decreased side effects; (iv) modified onset of therapeutic action and/or duration of effect; and/or (v) modified pharmacokinetic parameters, which may optionally be absorption, distribution, metabolism and/or excretion.

14. The method of claim 13, further comprising determining the *in vivo* toxicology profile of the lead or refined lead compound by a performing a quantitative structure activity relationship analysis of the lead or refined lead compound and/or
further comprising producing a pharmaceutical composition by formulating the lead or refined lead compound with a pharmaceutically acceptable carrier and optionally testing the lead or refined lead compound *in vivo,* by administering the pharmaceutical composition to a subject having a cancer cell, and evaluating the toxicity of the compound to the subject and/or the effect of the compound on the growth and/or survival of the cancer cell in the subject.

15. The method of any of claims 1 to 4, further comprising determining the expression of one or more cancer stem cell markers in the test cell and/or the control cell,
optionally wherein the one or more cancer stem cell markers are selected from: CD20, CD24, CD34, CD38, CD44, CD45, CD105, CD133, CD166, EpCAM, ESA, SCA1, Pecam, or Stro1,
optionally wherein the one or more cancer stem cell markers are CD24 and CD44.

16. The method of any of claims 1 to 15, wherein the step of detecting the level of inhibition of the growth and/or survival of the test cell and/or the control cell by the compound comprises performing an assay selected from: a cell counting assay, a replication labeling assay, a cell membrane integrity assay, a cellular ATP-based viability assay, a mitochondrial reductase activity assay, a caspase activity assay, an Annexin V staining assay, a DNA content assay, a DNA degradation assay, and a nuclear fragmentation assay.


<110>  Massachusetts Institute of Technology, et al.
       Whitehead Institute For Biomedical Research

<120>  METHODS FOR IDENTIFICATION AND USE OF AGENTS TARGETING CANCER
       STEM CELLS

<130>  M0656.70157WO00


<150>  61/043,948
<151>  2008-04-10

<150>  61/053,563
<151>  2008-05-15

<160>  30

<170>  PatentIn version 3.5

<210>  1
<211>  17
<212>  DNA
<213>  Artificial Sequence

<220>
<223>  Synthetic Oligonucleotide

<400>  1
tgtgggtgct gataatt                                                      17


<210>  2
<211>  17
<212>  DNA
<213>  Artificial Sequence

<220>
<223>  Synthetic Oligonucleotide

<400>  2
atcttgttac tgtgata                                                      17


<210>  3
<211>  17
<212>  DNA
<213>  Artificial Sequence

<220>
<223>  Synthetic Oligonucleotide

<400>  3
tccaaatcga tgtggat                                                      17


<210>  4
<211>  17
<212>  DNA
<213>  Artificial Sequence

<220>
<223>  Synthetic Oligonucleotide

<400>  4
tgcgtcccgc ccgccct                                                      17

<210> 5
<211> 17
<212> DNA
<213> Artificial Sequence

<220>
<223> Synthetic Oligonucleotide

<400> 5
gaattccctc ctgagtg 17


<210> 6
<211> 17
<212> DNA
<213> Artificial Sequence

<220>
<223> Synthetic Oligonucleotide

<400> 6
ctccattgtc ttactat 17


<210> 7
<211> 17
<212> DNA
<213> Artificial Sequence

<220>
<223> Synthetic Oligonucleotide

<400> 7
gtagatgcaa gggaaac 17


<210> 8
<211> 17
<212> DNA
<213> Artificial Sequence

<220>
<223> Synthetic Oligonucleotide

<400> 8
gtaaaatgca aatagat 17


<210> 9
<211> 17
<212> DNA
<213> Artificial Sequence

<220>
<223> Synthetic Oligonucleotide

<400> 9
attgtttcaa gtcagcc 17


<210> 10
<211> 17
<212> DNA
<213> Artificial Sequence

<220>
<223> Synthetic Oligonucleotide

<400>  10
ctcgaataaa aatgtag                                                    17


<210>  11
<211>  20
<212>  DNA
<213>  Artificial Sequence

<220>
<223>  Synthetic Oligonucleotide

<400>  11
gcctaaggac ctggtgaagc                                                 20


<210>  12
<211>  20
<212>  DNA
<213>  Artificial Sequence

<220>
<223>  Synthetic Oligonucleotide

<400>  12
ttgacgaagc actcgttgag                                                 20


<210>  13
<211>  20
<212>  DNA
<213>  Artificial Sequence

<220>
<223>  Synthetic Oligonucleotide

<400>  13
acccagaaga ctgtggatgg                                                 20


<210>  14
<211>  20
<212>  DNA
<213>  Artificial Sequence

<220>
<223>  Synthetic Oligonucleotide

<400>  14
tctagacggc aggtcaggtc                                                 20


<210>  15
<211>  20
<212>  DNA
<213>  Artificial Sequence

<220>
<223>  Synthetic Oligonucleotide

<400>  15
tgcccagaaa atgaaaaagg                                                 20


<210>  16
<211>  20
<212>  DNA

<213>  Artificial Sequence

<220>
<223>  Synthetic Oligonucleotide

<400>  16
gtgtatgtgg caatgcgttc                                                    20


<210>  17
<211>  20
<212>  DNA
<213>  Artificial Sequence

<220>
<223>  Synthetic Oligonucleotide

<400>  17
acagtggcca cctacaaagg                                                    20


<210>  18
<211>  20
<212>  DNA
<213>  Artificial Sequence

<220>
<223>  Synthetic Oligonucleotide

<400>  18
ccgagatggg gttgataatg                                                    20


<210>  19
<211>  20
<212>  DNA
<213>  Artificial Sequence

<220>
<223>  Synthetic Oligonucleotide

<400>  19
cagtgggaga cctcgagaag                                                    20


<210>  20
<211>  20
<212>  DNA
<213>  Artificial Sequence

<220>
<223>  Synthetic Oligonucleotide

<400>  20
tccctcggaa catcagaaac                                                    20


<210>  21
<211>  20
<212>  DNA
<213>  Artificial Sequence

<220>
<223>  Synthetic Oligonucleotide

<400>  21
gagaactttg ccgttgaagc                                                    20

<210>  22
<211>  20
<212>  DNA
<213>  Artificial Sequence

<220>
<223>  Synthetic Oligonucleotide

<400>  22
gcttcctgta ggtggcaatc                                                    20


<210>  23
<211>  20
<212>  DNA
<213>  Artificial Sequence

<220>
<223>  Synthetic Oligonucleotide

<400>  23
cctccctgtc agatgaggac                                                    20


<210>  24
<211>  20
<212>  DNA
<213>  Artificial Sequence

<220>
<223>  Synthetic Oligonucleotide

<400>  24
ccaggctgag gtattccttg                                                    20


<210>  25
<211>  20
<212>  DNA
<213>  Artificial Sequence

<220>
<223>  Synthetic Oligonucleotide

<400>  25
ggagtccgca gtcttacgag                                                    20


<210>  26
<211>  20
<212>  DNA
<213>  Artificial Sequence

<220>
<223>  Synthetic Oligonucleotide

<400>  26
tctggaggac ctggtagagg                                                    20


<210>  27
<211>  20
<212>  DNA
<213>  Artificial Sequence

<220>
<223>  Synthetic Oligonucleotide

<400>  27
ttcctgggct acgaccatac                                                    20


<210>  28
<211>  20
<212>  DNA
<213>  Artificial Sequence

<220>
<223>  Synthetic Oligonucleotide

<400>  28
tgtgctccat caagcaattc                                                    20


<210>  29
<211>  20
<212>  DNA
<213>  Artificial Sequence

<220>
<223>  Synthetic Oligonucleotide

<400>  29
ggggagaagc ctttttcttg                                                    20


<210>  30
<211>  20
<212>  DNA
<213>  Artificial Sequence

<220>
<223>  Synthetic Oligonucleotide

<400>  30
tcctcatgtt tgtgcaggag                                                    20